# EUROPEAN PATENT SPECIFICATION

(11) **EP 2 582 741 B1**
(45) Date of publication and mention of the grant of the patent: **27.07.2016**
(21) Application number: 11728712.8
(22) Date of filing: 20.06.2011
(51) Int. Cl.: C08G 73/02, C08G 65/337, A61K 47/48

(54) **BORONATED POLYMERS**
BORIERTE POLYMERE
POLYMÈRES BORÉS

(30) Priority: 18.06.2010 US 356229 P; 18.06.2010 EP 10166466
(43) Date of publication of application: 24.04.2013
(73) Proprietor: Universiteit Twente, 7522 NB Enschede (NL)
(72) Inventor: ENGBERSEN, Johannes, Franciscus, Joseph, NL-7552 GS Hengelo (NL); PIEST, Martin, NL-9611 CC Sappemeer (NL)
(74) Representative: Nederlandsch Octrooibureau
(86) International application number: PCT/NL2011/050440
(87) International publication number: WO 2011/159161

(56) References cited:
- WO-A1-01/92584
- WO-A2-2008/039827
- WO-A2-2010/048091
- US-A1- 2002 061 288
- US-A1- 2003 059 399
- US-A1- 2003 129 158
- US-A1- 2006 003 900
- US-A1- 2006 089 265
- JEONG J.H. ET AL: "Reducible poly(amido ethyleimine) directed to enhance RNA interference", BIOMATERIALS, vol. 28, 2007, pages 1912-1917, XP002616779,
- YASUMASA KANEKIYO ET AL I: "Novel Nucleotide-Responsive Hydrogels 1", JOURNAL OF POLYMER SCI. PART A: POLYMER CHEMISTRY, vol. 38, 2000, pages 1302-1310, XP002616778,

## Description

### Field of the invention

The present invention relates to boronated polymers and to processes for preparing such boronated polymers. The boronated polymers are useful in various drug delivery applications. The boronated polymers can be incorporated in nanoparticles and can form strong hydrogels.

### Background of the invention

Polymer based drug delivery systems have been shown to be very useful for the controlled delivery of drugs.

US 2005/0244504, incorporated by reference, discloses poly(amino ester)s prepared from bisacrylamides and functionalized primary amines. These poly(amino ester)s are used as pH triggering agents in polymeric micro-particles having a diameter of 100 nm to 10 µm, said polymeric micro-particles being used for the delivery of a drug, e.g. a DNA molecule or fragment. Upon exposure to an acidic environment, e.g. the endosome or the phagosome of a cell, the micro-particles dissolve or disrupt due to an enhanced solubility of the poly(amino ester) which is caused by hydrolysis of ester bonds in the polymer backbone. In their cationic form, the poly(amino ester)s form complexes with DNA molecules or fragments thereof.

US 2008/0242626, incorporated by reference, discloses poly(amino ester)s based on bisacrylamides and functionalized primary amines, wherein the poly(amino ester)s are subjected to an end-modification. Wherein the poly(amino ester) is amino-terminated, the poly(amino ester) is reacted with an electrophile. Wherein the poly(amino ester) is acrylate-terminated, the poly(amino ester) is reacted with a nucleophile. These end-modified poly(amino ester)s are used in drug delivery systems.

It is further known in the art that boric acid and boronic acids as well as polymers comprising boronic acid groups interact with polyol systems, e.g. polyvinyl alcohol, to form hydrogels which are for example used as drug delivery systems.

US 5.478.575, incorporated by reference, discloses sugar responsive polymer complexes which comprise cross-linked polymers comprising a monomer which comprises boronic acid groups. The monomers that are disclosed are acryloylaminobenzene boronic acid, methacryloylamino boronic acid and 4-vinylbenzene boronic acid. These sugar responsive polymer complexes are used as drug delivery systems, e.g. for insulin. Such systems are also disclosed in JP 9301982 and JP 11322761, incorporated by reference.

US 6.350.527, incorporated by reference, discloses water-soluble polymers comprising boronic acid groups which in cross-linked form are for example used in coatings that prevent tissue adhesion.

US 7.041.280, incorporated by reference, discloses polymers comprising boronate ester, boroamide or boronate thioester groups. The polymers are prepared by polymerizing ethylenically unsaturated monomers having a side chain comprising the boronate ester, the boroamide or the boronate thioester groups. These polymers are used in methods for preventing or treating obesity.

US 7.405.183, incorporated by reference, discloses viscosified treatment fluids comprising cross-linked gelling agents which are formed by cross-linking a treatment fluid with boronic acid containing cross-linking agents.

WO 2006/102762, incorporated by reference, discloses functionalized microgels made form cross-linked acrylic polymers comprising boronic acid groups. The microgels are used in insulin-delivery systems.

US 2008/0099172, incorporated by reference, discloses acrylic polymers comprising boronic acid groups which are used in papermaking processes.

US 2007/0116740, incorporated by reference, discloses polymers which include ethylenically unsaturated monomers comprising boronic acid groups, e.g. 4-phenyl boronic acid and N-methacryloyl-3-aminophenyl boronic acid and their application in the manufacture of contact lenses.

US 2002/0061288 discloses gels and multilayer surface structures from boronic acid-containing polymers.

Drug delivery systems based on polymers comprising boronic acid groups which are known from the prior art can only be employed at basic pH, i.e. at a pH above 7. However, it would be very advantageous when reversible drug delivery systems can be provided that deliver the drug at an acidic pH, i.e. at a pH below 7, since such conditions prevail at sites which are involved in transport processes of substances into a cell, e.g. phagosomes and endosomes.

### Summary of the invention

The present invention relates to a boronated polymer according to Formula (1): 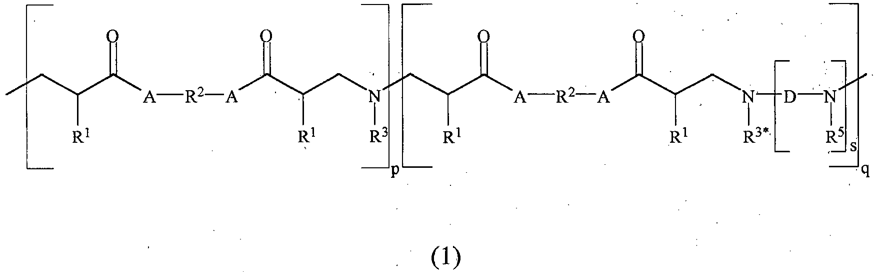 wherein:
A is independently selected from a direct carbon-carbon single bond (i.e. a structure wherein A is absent. i.e. -C(O)-R²-C(O)-), O, N and S;
R¹ is independently selected from H and CH₃;
R² is independently selected from the group consisting of:
   (a) C₁ - C₂₀ alkylene, wherein the alkylene group is optionally substituted and/or is optionally (partly) unsaturated and/or is optionally interrupted by one or more heteroatoms, wherein the heteroatoms are independently selected from O, N and S, and/or wherein the alkylene group is interrupted by one or more -S-S- groups;
   (b) C₃ - C₂₀ cycloalkylene, wherein the cycloalkylene group is optionally substituted and/or is optionally (partly) unsaturated and/or optionally comprises one or more heteroatoms in the ring, wherein the heteroatoms are independently selected from O, N and S, and/or wherein the cycloalkylene group is interrupted by one or more
      - S-S- groups outside the ring;
   (c) C₆ - C₂₀ arylene, wherein the arylene group is optionally substituted;
   (d) C₆ - C₂₀ heteroarylene, wherein the heteroarylene group comprises 1 - 3 heteroatoms independently selected from O, N and S and/or wherein the heteroarylene group is optionally substituted;
   (e) C₇ - C₂₀ alkylarylene wherein the alkylarylene group is optionally substituted and/or wherein an alkyl part of the alkylarylene group is optionally (partly) unsaturated and/or is optionally interrupted by one or more heteroatoms, wherein the heteroatoms are independently selected from O, N and S, and/or wherein an alkyl part of the alkylarylene group is interrupted by one or more -S-S- groups; and
   (f) C₇-C₂₀ alkylheteroarylene, wherein the alkylheteroarylene group comprises 1 - 3 heteroatoms independently selected from O, N and S and/or wherein the alkylheteroarylene group is optionally substituted, and/or wherein an alkyl part of the alkylheteroarylene group is optionally (partly) unsaturated and/or is optionally interrupted by one or more heteroatoms, wherein the heteroatoms are independently selected from O, N and S, and/or wherein an alkyl part of the alkylheteroarylene group is interrupted by one or more -S-S- groups; and
   (g) a group wherein two (hetero)arylene groups and/or alkyl(hetero)arylene groups are connected to each other by a -S-S- group;
D is selected from the group consisting of -(CR⁴₂)ᵣ- and the groups (a), (b), (c), (d), (e), (f) and (g) defined for R²;
R⁴ is independently selected from the group consisting of:
   (a) H;
   (b) C₁ - C₂₀ alkyl, wherein the alkyl group is optionally substituted and/or is optionally (partly) unsaturated and/or is optionally interrupted by one or more heteroatoms, wherein the heteroatoms are independently selected from O, N and S;
   (c) C₃ - C₂₀ cycloalkyl, wherein the cycloalkyl group is optionally substituted and/or, is optionally (partly) unsaturated and/or optionally comprises one or more heteroatoms in the ring, wherein the heteroatoms are independently selected from O, N and S;
   (d) C₆ - C₂₀ aryl, wherein the aryl group is optionally substituted;
   (e) C₆ - C₂₀ heteroaryl, wherein the heteroaryl group comprises 1 - 3 heteroatoms independently selected from O, N and S and/or wherein the heteroaryl group is optionally substituted;
   (f) C₇ - C₂₀ alkylaryl wherein the alkylaryl group is optionally substituted and/or wherein an alkyl part of the alkylaryl group is optionally (partly) unsaturated and/or is optionally interrupted by one or more heteroatoms, wherein the heteroatoms are independently selected from O, N and S; and
   (g) C₇ - C₂₀ alkylheteroaryl, wherein the alkylheteroaryl group comprises 1 - 3 heteroatoms independently selected from O, N and S and/or wherein the alkylheteroaryl group is optionally substituted, and/or wherein an alkyl part of the alkylheteroaryl group is optionally (partly) unsaturated and/or is optionally interrupted by one or more heteroatoms, wherein the heteroatoms are independently selected from O, N and S;
R⁵ is independently selected from the group consisting of H and C₁ - C₂₀ alkyl, wherein the alkyl group is optionally substituted and/or is optionally (partly) unsaturated and/or is optionally interrupted by one or more heteroatoms, wherein the heteroatoms are independently selected from O, N and S;
p = 1 to 100;
q = 0 to 100;
r = 2 - 6;
s = 0 to 5;
R³ has the Formula (2): 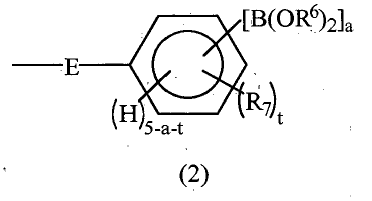
wherein:
a is 1 or 2;
R⁶ is independently selected from
   (a) H;
   (b) C₁ - C₁₂ alkyl, wherein the alkyl group is optionally substituted and/or is optionally (partly) unsaturated and/or is optionally interrupted by one or more heteroatoms, wherein the heteroatoms are independently selected from O, N and S;
   (c) C₃ - C₁₂ cycloalkyl, wherein the cycloalkyl group is optionally substituted and/or is optionally (partly) unsaturated and/or is optionally interrupted by one or more heteroatoms, wherein the heteroatoms are independently selected from O, N and S;
   (d) C₆ - C₂₀ aryl, wherein the aryl group is optionally substituted;
   (e) C₆ - C₂₀ heteroaryl, wherein the heteroaryl group comprises 1 - 3 heteroatoms independently selected from O, N and S and/or wherein the heteroaryl group is optionally substituted;
   (f) C₇ - C₂₀ alkylaryl wherein the alkylaryl group is optionally substituted and/or wherein an alkyl part of the alkylaryl group is optionally (partly) unsaturated and/or is optionally interrupted by one or more heteroatoms, wherein the heteroatoms are independently selected from O, N and S; and
   (g) C₇ - C₂₀ alkylheteroaryl, wherein the alkylheteroaryl group comprises 1 - 3 heteroatoms independently selected from O, N and S and/or wherein the alkylheteroaryl group is optionally substituted, and/or wherein an alkyl part of the alkylheteroaryl group is optionally (partly) unsaturated and/or is optionally interrupted by one or more heteroatoms, wherein the heteroatoms are independently selected from O, N and S;
R⁷ is independently selected from
   (a) halogen;
   (b) C₁ - C₁₂ alkyl, wherein the alkyl group is optionally substituted and/or is optionally (partly) unsaturated and/or is optionally interrupted by one or more heteroatoms, wherein the heteroatoms are independently selected from O, N and S;
   (c) C₃ - C₁₂ cycloalkyl, wherein the cycloalkyl group is optionally substituted and/or is optionally (partly) unsaturated and/or is optionally interrupted by one or more heteroatoms, wherein the heteroatoms are independently selected from O, N and S;
   (d) C₆ - C₂₀ aryl, wherein the aryl group is optionally substituted;
   (e) C₆ - C₂₀ heteroaryl, wherein the heteroaryl group comprises 1 - 3 heteroatoms independently selected from O, N and S and/or wherein the heteroaryl group is optionally substituted;
   (f) C₇ - C₂₀ alkylaryl wherein the alkylaryl group is optionally substituted and/or wherein an alkyl part of the alkylaryl group is optionally (partly) unsaturated and/or is optionally interrupted by one or more heteroatoms, wherein the heteroatoms are independently selected from O, N and S; and
   (g) C₇ C₂₀ alkylheteroaryl, wherein the alkylheteroaryl group comprises 1 - 3 heteroatoms independently selected from O, N and S and/or wherein the alkylheteroaryl group is optionally substituted, and/or wherein an alkyl part of the alkylheteroaryl group is optionally (partly) unsaturated and/or is optionally interrupted by one or more heteroatoms, wherein the heteroatoms are independently selected from O, N and S;
E is independently selected from the group consisting of:
   -(CR⁸₂)ᵤ-N(R⁹)-C(O)-,
   -C(O)-N(R⁹)-(CR⁸₂)ᵤ-,
   -(CR⁸₂)ᵤ-N=CR⁹-,
   -C(R⁹)=N-(CR⁸₂)ᵤ-, and
   -(CR⁸₂)ᵤ-N(R⁹)-(CR⁸₂)ᵥ-;
t = 0 - 4;
u = 1 - 10;
v = 1 - 4;
R⁸ is independently selected from the group consisting of H and C₁ - C₂₀ alkyl;
R⁹ is independently selected from the group consisting of H and C₁ - C₂₀ alkyl; and when s = 0, then R^{3*} is independently selected from the group consisting of
   (a) H,
   (b) C₁ - C₁₂ alkyl, wherein the alkyl group is optionally substituted and/or is optionally (partly) unsaturated and/or is optionally interrupted by one or more heteroatoms, wherein the heteroatoms are independently selected from O, N and S;
   (c) C₃ - C₁₂ cycloalkyl, wherein the cycloalkyl group is optionally substituted and/or is optionally (partly) unsaturated and/or is optionally interrupted by one or more heteroatoms, wherein the heteroatoms are independently selected from O, N and S;
   (d) C₆ - C₂₀ aryl, wherein the aryl group is optionally substituted;
   (e) C₆ - C₂₀ heteroaryl, wherein the heteroaryl group comprises 1 - 3 heteroatoms independently selected from O, N and S and/or wherein the heteroaryl group is optionally substituted;
   (f) C₇ - C₂₀ alkylaryl wherein the alkylaryl group is optionally substituted and/or wherein an alkyl part of the alkylaryl group is optionally (partly) unsaturated and/or is optionally interrupted by one or more heteroatoms, wherein the heteroatoms are independently selected from O, N and S; and
   (g) C₇ - C₂₀ alkylheteroaryl, wherein the alkylheteroaryl group comprises 1 - 3 heteroatoms independently selected from O, N and S and/or wherein the alkylheteroaryl group is optionally substituted, and/or wherein an alkyl part of the alkylheteroaryl group is optionally (partly) unsaturated and/or is optionally interrupted by one or more heteroatoms, wherein the heteroatoms are independently selected from O, N and S;
when s = 1 - 5, then R^{3*} is independently selected from the group consisting of
   (a) H;
   (b) C₁ - C₆ alkyl, wherein the alkyl group is optionally substituted;
   (c) C₃ - C₆ cycloalkyl, wherein the cycloalkyl group is optionally substituted;
   (d) C₆ - C₁₂ aryl, wherein the aryl group is optionally substituted;
   (e) C₆ - C₁₂ heteroaryl, wherein the heteroaryl group comprises 1 - 3 heteroatoms independently selected from O, N and S and/or wherein the heteroaryl group is optionally substituted;
   (f) C₇ - C₁₂ alkylaryl wherein the alkylaryl group is optionally substituted; and
   (g) C₇ C₁₂ alkylheteroaryl, wherein the alkylheteroaryl group comprises 1 - 3 heteroatoms independently selected from O, N and S and/or wherein the alkylheteroaryl group is optionally substituted.

The present invention further relates to processes for preparing the boronated polymer. The present invention further relates to an aggregate and a nanoparticle comprising the boronated polymer and to a hydrogel comprising the boronated polymer.

### Detailed description of the invention

The verb "to comprise" as is used in this description and in the claims and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there is one and only one of the elements. The indefinite article "a" or "an" thus usually means "at least one".

### The boronated polymer

The boronated polymers according to the present invention have several advantageous and beneficial properties. For example, the boronated polymers according to the present invention have a lower toxicity than their counterparts lacking a boronate moiety. Additionally, the boronated polymers according to the present invention have higher transfection efficiency, in particular because the boronated polymers are capable of binding with glycoproteins present on cell membranes. Moreover, the boronated polymers form polyplexes with pDNA that show enhanced response in the endosomal pH range (pH 7.4 - 5.0), which favourably contributes to endosomal escape. Furthermore, the boronated polymers according to the present invention enable to manufacture of drug delivery systems wherein drug delivery is reversibly triggered or induced by e.g. pH, temperature or a carbohydrate. Such drug delivery systems include aggregates, nanoparticles and hydrogels:

According to the present invention, it is preferred that the boronated polymer according to Formula (1) has a number average molecular weight Mₙ in the range of about 1,000 to about 100,000 g/mol, more preferably in the range of about 3,000 to about 20,000.

According to the present invention, the weight average molecular weight M_{w} of the boronated polymer is preferably about 1,000 to about 200,000, more preferably about 2,000 to about 150,000, even more preferably about 3,000 to about 100,000 and in particular about 3,000 to about 75,000.

For certain boronated polymers of the present invention, a M_{w} of about 3,000 corresponds to a value for p of about 10 and a M_{w} of about 75,000 corresponds to a value for p of about 100.

According to the present invention, it is more preferred that R¹ is H.

According to a preferred embodiment of the present invention, R² is preferably selected from the group consisting of groups (a) - (g) as defined above, the alkylene group being preferably a C₁ - C₁₀ alkylene group, the cycloalkylene group being preferably a C₁ - C₁₀ cycloalkylene group, the arylene group being preferably a C₆ - C₁₂ arylene group, the heteroarylene group being preferably a C₆ - C₁₂ heteroarylene group, the alkylarylene group being preferably a C₇ - C₁₃ alkylarylene group and the alkylheteroarylene group being preferably a C₇ - C₁₃ alkylheteroarylene group.

According to another preferred embodiment of the present invention, R² is independently selected from the group consisting of C₁ - C₂₀ alkylene, wherein the alkylene group is optionally substituted and/or is optionally (partly) unsaturated and/or is optionally interrupted by one or more heteroatoms, wherein the heteroatoms are independently selected from O, N and S, and/or wherein the alkylene group is interrupted by one or more -S-S- groups, and C₃ - C₂₀ cycloalkylene, wherein the cycloalkylene group is optionally substituted and/or is optionally (partly) unsaturated and/or optionally comprises one or more heteroatoms in the ring, wherein the heteroatoms are independently selected from O, N and S, and/or wherein the cycloalkylene group is interrupted by one or more -S-S- groups outside the ring. Even more preferably, R² is a C₁ - C₂₀ alkylene as defined above.

According to yet another preferred embodiment of the present invention, R^{3*} is H.

According to a yet another preferred embodiment of the present invention, when E is selected from groups (a) - (g) defined above for R², it is preferred that the alkylene group is a C₁ - C₁₀ alkylene group, the cycloalkylene group is a C₁ - C₁₀ cycloalkylene group, the arylene group is a C₆ - C₁₂ arylene group, the heteroarylene group is a C₆ - C₁₂ heteroarylene group, the alkylarylene group is a C₇ - C₁₃ alkylarylene group and that the alkylheteroarylene group is a C₇ - C₁₃ alkylheteroarylene group. According to another preferred embodiment, it is preferred that E represents a C₁ - C₂₀ alkylene group, wherein the alkylene group is optionally substituted and/or is optionally (partly) unsaturated and/or is optionally interrupted by one or more heteroatoms, wherein the heteroatoms are independently selected from O, N and S, and/or wherein the alkylene group is interrupted by one or more -S-S- groups, and C₃ - C₂₀ cycloalkylene, wherein the cycloalkylene group is optionally substituted and/or is optionally (partly) unsaturated and/or optionally comprises one or more heteroatoms in the ring, wherein the heteroatoms are independently selected from O, N and S, and/or wherein the cycloalkylene group is interrupted by one or more -S-S- groups outside the ring. Even more preferably, E is a C₁ - C₂₀ alkylene as defined above.

According to yet another preferred embodiment of the present invention, R⁴ is independently selected from the group consisting of H; C₁ - C₂₀ alkyl, wherein the alkyl group is optionally substituted and/or is optionally (partly) unsaturated and/or is optionally interrupted by one or more heteroatoms, wherein the heteroatoms are independently selected from O, N and S; and C₃ - C₂₀ cycloalkyl, wherein the cycloalkyl group is optionally substituted and/or is optionally (partly) unsaturated and/or optionally comprises one or more heteroatoms in the ring, wherein the heteroatoms are independently selected from O, N and S. The alkyl group is even more preferably a C₁ - C₁₂ alkyl group. The cycloalkyl group is even more preferably a C₃ - C₁₂ cycloalkyl group.

According to yet another preferred embodiment of the present invention, R⁶ is preferably independently selected from H; C₁ - C₁₂ alkyl, wherein the alkyl group is optionally substituted and/or is optionally (partly) unsaturated and/or is optionally interrupted by one or more heteroatoms, wherein the heteroatoms are independently selected from O, N and S; and C₃ - C₁₂ cycloalkyl, wherein the cycloalkyl group is optionally substituted and/or is optionally (partly) unsaturated and/or is optionally interrupted by one or more heteroatoms, wherein the heteroatoms are independently selected from O, N and S. The alkyl group is even more preferably a C₁ - C₁₂ alkyl group. The cycloalkyl group is even more preferably a C₃ - C₁₂ cycloalkyl group.

According to yet another preferred embodiment of the present invention, R⁷ is preferably independently selected from halogen; C₁ - C₁₂ alkyl, wherein the alkyl group is optionally substituted and/or is optionally (partly) unsaturated and/or is optionally interrupted by one or more heteroatoms, wherein the heteroatoms are independently selected from O, N and S; and C₃ - C₁₂ cycloalkyl, wherein the cycloalkyl group is optionally substituted and/or is optionally (partly) unsaturated and/or is optionally interrupted by one or more heteroatoms, wherein the heteroatoms are independently selected from O, N and S. The alkyl group is even more preferably a C₁ - C₁₂ alkyl group. The cycloalkyl group is even more preferably a C₃ - C₁₂ cycloalkyl group.

According to yet another preferred embodiment of the present invention, t = 0 - 2.

According to yet another preferred embodiment of the present invention, u = 1 - 6, more preferably 2 - 6, and most preferably 2 - 4.

According to yet another preferred embodiment of the present invention, v = 1 - 6, more preferably 1 - 4, and most preferably 1 - 2.

According to yet another preferred embodiment of the present invention, R⁸ is H.

According to yet another preferred embodiment of the present inverition, R⁹ is H.

A preferred group of the boronated polymers according to the present invention are those wherein s = 0. This group of the boronated polymers is then represented by Formula (3): 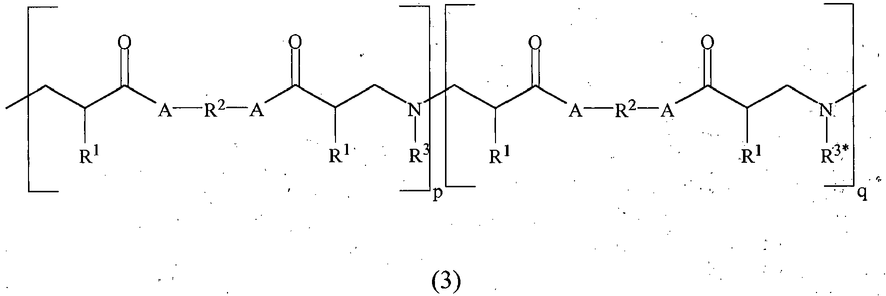 wherein R¹, R², R³, R^{3*}, A, p and q are as defined above.

Another preferred group of the boronated polymers according to the present invention are those wherein s = 1. These boronated polymers are represented by Formula (4):

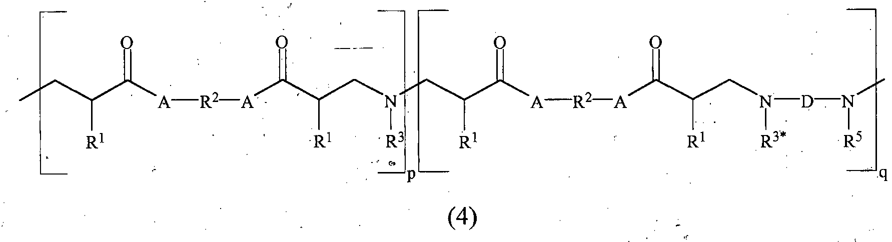

According to this embodiment, it is preferred that D is selected from group (a) defined for R². More in particular, D is a group -N(R¹⁰)-(CR⁴₂)₂-S-S-(CR⁴₂)₂-N(R¹⁰)-, wherein R¹⁰ is selected from the group consisting ofH and C₁-C₆ alkyl, preferably H and methyl, and wherein R⁴ is as defined above, preferably H.

Another preferred group of the boronated polymers according to the present invention are those wherein q = 0. This group of the boronated polymers is then represented by Formula (5): 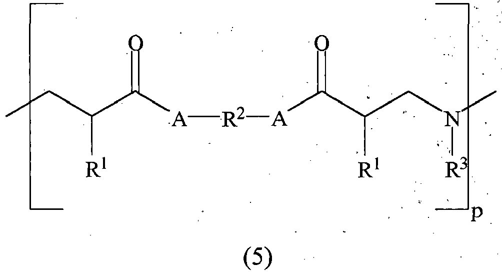 wherein R¹, R², R³, A and p are defined as above.

### Process for preparing the boronated polymer

According to an embodiment of the present invention, the boronated polymers may be prepared by a process wherein a monomer according to Formula (6): 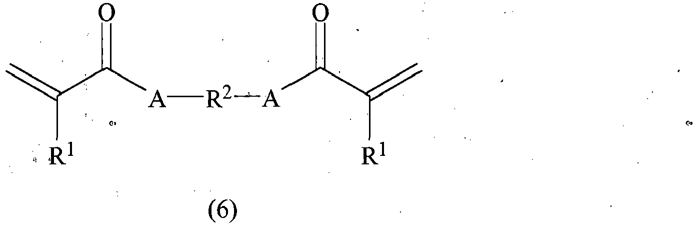 is polymerized with a monomer according to Formula (7):

H₂N-R³ (7)

and:
optionally in the presence of a monomer according to Formula (8):

   H₂N-R^{3*} (8)
or optionally in the presence of a monomer according to Formula (9): 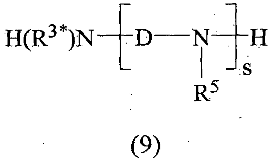
wherein R¹, R², R³, R^{3*}, R⁴, R⁵, A, r, D and s are as defined above. Consequently, according to this process, the boronated polymers according to the present invention may thus be prepared by polymerizing a monomer according to Formula (6) with a monomer according to Formula (7). Such a process provides boronated polymers according to Formula (5).

The boronated polymers according to the present invention may also be prepared by polymerizing a monomer according to Formula (6) with a monomer according to Formula (7) and with a monomer according to Formula (8). Such a process provides boronated polymers according to Formula (3).

The boronated polymers according to the present invention may also be prepared by polymerizing a monomer according to Formula (6) with a monomer according to Formula (7) and with a monomer according to Formula (9). Such a process provides boronated polymers according to Formula (1).

According to another embodiment of the present invention, the boronated polymers may be prepared by a process comprising the following steps:
(a) polymerizing a monomer according to Formula (6): 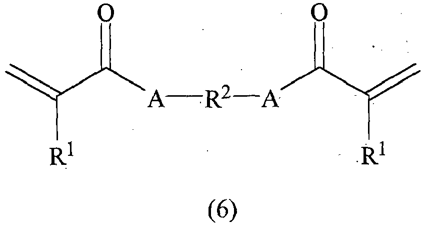 with a monomer according to Formula (10):

   ^{t}BuO-C(O)-N(H)-(CR⁸₂)_{w}-NH₂ (10)

   wherein w = 2 - 20 and R⁸ is independently selected from the group consisting of H and C₁ - C₂₀ alkyl, to obtain a polymer according to Formula (11): 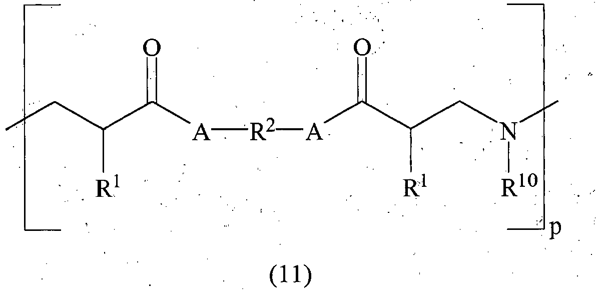 wherein R¹⁰ is ^{t}BuO-C(O)-N(H)-(CR⁸₂)_{w};
(b) reacting the polymer according to Formula (11) with an acid to obtain a polymer according to Formula (11) wherein R¹⁰ is H₂N-(CR⁸₂)_{w}-; and
(c) reacting the polymer as obtained in step (b) with a compound according to Formula (12): 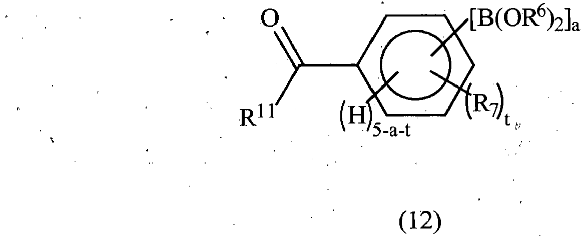 wherein:
   R⁶ is independently selected from
      (a) H;
      (b) C₁ - C₁₂ alkyl, wherein the alkyl group is optionally substituted and/or is optionally (partly) unsaturated and/or is optionally interrupted by one or more heteroatoms, wherein the heteroatoms are independently selected from O, N and S;
      (c) C₃ - C₁₂ cycloalkyl, wherein the cycloalkyl group is optionally substituted and/or is optionally (partly) unsaturated and/or is optionally interrupted by one or more heteroatoms, wherein the heteroatoms are independently selected from O, N and S;
      (d) C₆ - C₂₀ aryl, wherein the aryl group is optionally substituted;
      (e) C₆ - C₂₀ heteroaryl, wherein the heteroaryl group comprises 1 - 3 heteroatoms independently selected from O, N and S and/or wherein the heteroaryl group is optionally substituted;
      (f) C₇ - C₂₀ alkylaryl wherein the alkylaryl group is optionally substituted and/or wherein an alkyl part of the alkylaryl group is optionally (partly) unsaturated and/or is optionally interrupted by one or more heteroatoms, wherein the heteroatoms are independently selected from O, N and S; and
      (g) C₇ - C₂₀ alkylheteroaryl, wherein the alkylheteroaryl group comprises 1 - 3 heteroatoms independently selected from O, N and S and/or wherein the alkylheteroaryl group is optionally substituted, and/or wherein an alkyl part of the alkylheteroaryl group is optionally (partly) unsaturated and/or is optionally interrupted by one or more heteroatoms, wherein the heteroatoms are independently selected from O, N and S;
   R⁷ is independently selected from
      (a) halogen;
      (b) C₁ - C₁₂ alkyl, wherein the alkyl group is optionally substituted and/or is optionally (partly) unsaturated and/or is optionally interrupted by one or more heteroatoms, wherein the heteroatoms are independently selected from O, N and S;
      (c) C₃ - C₁₂ cycloalkyl, wherein the cycloalkyl group is optionally substituted and/or is optionally (partly) unsaturated and/or is optionally interrupted by one or more heteroatoms, wherein the heteroatoms are independently selected from O, N and S;
      (d) C₆ - C₂₀ aryl, wherein the aryl group is optionally substituted;
      (e) C₆ - C₂₀ heteroaryl, wherein the heteroaryl group comprises 1 - 3 heteroatoms independently selected from O, N and S and/or wherein the heteroaryl group is optionally substituted;
      (f) C₇ - C₂₀ alkylaryl wherein the alkylaryl group is optionally substituted and/or wherein an alkyl part of the alkylaryl group is optionally (partly) unsaturated and/or is optionally interrupted by one or more heteroatoms, wherein the heteroatoms are independently selected from O, N and S; and
      (g) C₇ - C₂₀ alkylheteroaryl, wherein the alkylheteroaryl, group comprises 1 - 3 heteroatoms independently selected from O, N and S and/or wherein the alkylheteroaryl group is optionally substituted, and/or wherein an alkyl part of the alkylheteroaryl group is optionally (partly) unsaturated and/or is optionally interrupted by one or more heteroatoms, wherein the heteroatoms are independently selected from O, N and S;
   R¹¹ is H or a leaving group;
   t = 0 - 4; and
   R¹, R² and A are as defined above.

Preferably, R¹¹ is selected from the group of H, OH and Cl.

It is also preferred that a = 1 which implies that the compound according to Formula (12) has the Formula (13):

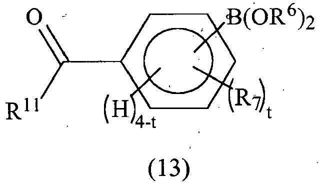

### The aggregate and the nanoparticle

The present invention also relates to an aggregate and to a nanoparticle comprising the boronated polymer according to the present invention. Preferably, the aggregate is a nanoparticle.

Preferably, the aggregate or the nanoparticle further comprises a biologically active component selected from the group of drugs, anionic polymers, DNA molecules or derivatives thereof, RNA molecules or derivatives thereof), peptides and derivatives thereof, and proteins and derivatives thereof. It is preferred that the weight ratio of the boronated polymer and the biologically active component is about 20 - 50 to 1.

According to the present invention, the nanoparticles according to the present invention have a particle size of about 10 to about 500 nm, more preferably about 30 to about 300 nm. The nanoparticles have a high stability and show a low tendency to aggregation as appears from a value of the ξ-potential of about 30 to about 60 mV, preferably about 35 to about 50 mV.

The aggregates and the nanoparticles according to the present invention are very suitable for delivery of a biologically active component to a mammal. This was established in transfection (a process for introducing nucleic acid derivatives into a cell) experiments which showed transfection efficiencies comparable with those obtained with linear poly(ethylene imine) (PEI; ExGen®). PEI is known to be a polymer having a high cationic-charge density, which effectively condenses DNA for highly efficient gene-delivery. Furthermore, PEI/DNA complexes are known to interact with cell surface proteoglycans (syndecans) resulting in internalization by endosomes. PEI is capable of acting as an effective proton sponge buffer within the endosome, thereby protecting the internalized DNA from lysosomal degradation. PEI and similar polymers used for this purpose are known from e.g. US 2010/041739, incorporated by reference.

Accordingly, the present invention also relates to the use of the boronated polymer according to the present invention as a transfection agent.

The present invention further relates to a method for delivering a biologically active component to a mammal, wherein a pharmaceutical composition comprising an aggregate or a nanoparticle comprising a boronated polymer according to the present invention and a biologically active component is administered to said mammal. This method in particular relates to delivering the biologically active component to a cell, preferably an eukaryotic cell.

The nanoparticles according to the present invention are preferably coated with a polyol. Preferred polyols include vicinal diols or components comprising a vicinal diol moiety. Preferred polyols also include carbohydrates, in particular monosaccharides, disaccharides and polysaccharides. Preferred monosaccharides include sorbitol, mannose and galactose. Preferred polysaccharides have a weight average molecular weight of about 10 kDa to about 20.000 kDa. The polysaccharides may be branched or unbranched and are preferably selected from the group consisting of glycosaminoglycans, glucans and galactomannans. The glycosaminoglycan is preferably an anionic glycosaminoglycan, more preferably an anionic, non-sulfated glycosaminoglycan. The glycosaminoglycan has preferably a molecular weight of about 50 kDa to about 20.000 kDa. Most preferably, the glycosaminoglycan is hyaluronic acid (also known as hyalurorlan). The glucan is preferably an α-glucan, more preferably a α-1,6-glucan. The glucan has preferably a molecular weight of from about 10 kDa to about 150 kDa. Most preferably, the glucan is dextran. Galactomannans are polysaccharides having a D-mannose backbone and D-galactose side groups. The galactomannan has preferably a mannose to galactose ratio of about 1 : 1 to about 4 : 1, more preferably about 1 : 1 to about 2 : 1. The galactomannan has preferably a molecular weight of about 100 kDa to about 300 kDa. Most preferably, the galactomannan is guar gum or a derivative thereof, e.g. hydroxypropyl guar gum and carboxymethyl guar gum.

Preferred polyols further include polyvinyl alcohols, polyethylene glycols and derivatives thereof, which preferably have weight average molecular weight of about 10 to about 300 kDa. A particularly preferred polyol is also polyvinyl alcohol.

The coated nanoparticles show higher transfection efficiencies than the non-coated nanoparticles. These coated nanoparticles are prepared by treating aqueous compositions of the non-coated nanoparticles with an aqueous composition of the polysaccharide or the polyol. Consequently, according to the present invention, the polyol is preferably selected from the group consisting of vicinal diols, components comprising a vicinal diol moiety, monosaccharides, disaccharides, polysaccharides, polyvinyl alcohols; polyethylene glycols and derivatives of these polyols.

The nanoparticles according to the present invention may also incorporate a polyol as described above. Preferably, the polyol is than a drug such as dopamine.

The nanoparticles according to the present invention may also incorporate a component that enables the control of the delivery of the biologically active component, e.g. a fluorescent dye.

The nanoparticles according to the present invention are pH-responsive, in particular within a pH-range of about 5 to less than about 8. The nanoparticles have therefore excellent endosomolytic properties.

It is furthermore preferred that in the boronated polymer R² is independently selected from the group consisting of:
(a) C₁ - C₂₀ alkylene, wherein the alkylene group is optionally substituted and/or is optionally (partly) unsaturated and/or is optionally interrupted by one or more heteroatoms, wherein the heteroatoms are independently selected from O, N and S, and/or wherein the alkylene group is interrupted by one or more -S-S- groups; and
(b) C₃ - C₂₀ cycloalkylene, wherein the cycloalkylene group is optionally substituted and/or is optionally (partly) unsaturated and/or optionally comprises one or more heteroatoms in the ring, wherein the heteroatoms are independently selected from O, N and S, and/or wherein the cycloalkylene group is interrupted by one or more -S-S- groups outside the ring.

### The hydrogel

The present invention also relates to a hydrogel comprising the boronated polymer according to the present invention. These hydrogels are pH sensible, thermoreversible, responsive to 1,2- and 1,3-dihydroxy and diamino groups, including carbohydrates, and have self-healing properties. The hydrogels can conveniently be used for controlled drug delivery at physiological pH or lower.

Preferably, the hydrogel further comprises a macromolecular polyol, wherein the macromolecular polyol is preferably selected from the group consisting of polyvinyl alcohols and polysaccharides. The polyvinyl alcohols have preferably a weight average molecular weight M_{w} of about 10 to about 300 kDa. The polysaccharides may be unbranched or branched and have preferably a molecular weight of about 10 kDa to about 20,000 kDa. More preferably, the polysaccharides are selected from the group consisting of glycosaminoglycans, glucans and galactomannans.

The glycosaminoglycan is preferably an anionic glycosaminoglycan, more preferably an anionic, non-sulfated glycosaminoglycan. The glycosaminoglycan has preferably a molecular weight of about 50 kDa to about 20,000 kDa. Most preferably, the glycosaminoglycan is hyaluronic acid (also known as hyaluronan).

The glucan is preferably an α-glucan, more preferably a α-1,6-glucan. The glucan has preferably a molecular weight of from about 10 kDa to about 150 kDa. Most preferably, the glucan is dextran.

Galactomannans are polysaccharides having a D-mannose backbone and D-galactose side groups. The galactomannan has preferably a mannose to galactose ratio of about 1 : 1 to about 4 : 1, more preferably about 1 : 1 to about 2 : 1. The galactomannan has preferably a molecular weight of about 100 kDa to about 300 kDa. Most preferably, the galactomannan is guar gum or a derivative thereof, e.g. hydroxypropyl guar gum and carboxymethyl guar gum.

Preferably, the hydrogel further comprises a carbohydrate.

It is also preferred that the hydrogel according to the present invention comprises a cross-linker.

It is furthermore preferred that in the boronated polymer R² is independently selected from the group consisting of:
(a) C₁ - C₂₀ alkylene, wherein the alkylene group is optionally substituted and is interrupted by one or more -S-S- groups; and
(b) C₃ - C₂₀ cycloalkylene, wherein the cycloalkylene group is optionally substituted and is interrupted by one or more -S-S- groups outside the ring.

Ivanov et al, Chem. Eur. J. 12, 7204 - 7214, 2006, incorporated by reference, discloses that boronic acid containing polymers have several advantages over borax as a crosslinker, such as higher shape stability and usability at lower pH. However, the polymers disclosed by Ivanov *et al* are not biodegradable and can only form hydrogels at relatively high pH, i.e. above physiological pH. The boronated polymers according to the present invention, however, offer the possibility to introduce specific properties to the hydrogel, including pH responsiveness, drug loading and release, and triggered release by biomolecules like glucose. The boronated polymers according to the present invention have peptide mimicking structures and are biocompatible, biodegradable and when disulfide moieties are incorporated in the polymer backbone they are bioreducible in the intracellular environment.

The present invention also relates to a hydrogel comprising poly(boronic acid) cross-linkers for fast en reversible gelation of macromolecular polyols, in particular polyvinyl alcohol, resulting in poly(boronic) compounds according to Formula (14) and Formula (15): 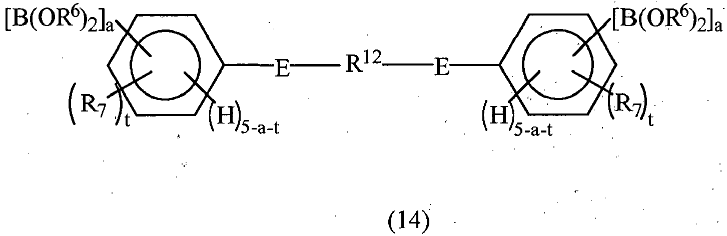 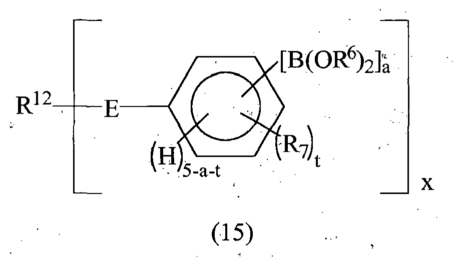 wherein:
x = 3 - 8;
a = 1 or 2;
R⁶ is independently selected from
   (a) H;
   (b) C₁ - C₁₂ alkyl, wherein the alkyl group is optionally substituted and/or is optionally (partly) unsaturated and/or is optionally interrupted by one or more heteroatoms, wherein the heteroatoms are independently selected from O, N and S;
   (c) C₃ - C₁₂ cycloalkyl, wherein the cycloalkyl group is optionally substituted and/or is optionally (partly) unsaturated and/or is optionally interrupted by one or more heteroatoms, wherein the heteroatoms are independently selected from O, N and S; C₆ - C₂₀ aryl, wherein the aryl group is optionally substituted;
   (d) C₆ - C₂₀ heteroaryl, wherein the heteroaryl group comprises 1 - 3 heteroatoms independently selected from O, N and S and/or wherein the heteroaryl group is optionally substituted;
   (e) C₇ - C₂₀ alkylaryl wherein the alkylaryl group is optionally substituted and/or wherein an alkyl part of the alkylaryl group is optionally (partly) unsaturated and/or is optionally interrupted by one or more heteroatoms, wherein the heteroatoms are independently selected from O, N and S; and
   (f) C₇ - C₂₀ alkylheteroaryl, wherein the alkylheteroaryl group comprises 1 - 3 heteroatoms independently selected from O, N and S and/or wherein the alkylheteroaryl group is optionally substituted, and/or wherein an alkyl part of the alkylheteroaryl group is optionally (partly) unsaturated and/or is optionally interrupted by one or more heteroatoms, wherein the heteroatoms are independently selected from O, N and S;
R⁷ is independently selected from
   (a) halogen;
   (b) C₁ - C₁₂ alkyl, wherein the alkyl group is optionally substituted and/or is optionally (partly) unsaturated and/or is optionally interrupted by one or more heteroatoms, wherein the heteroatoms are independently selected from O, N and S;
   (c) C₃ - C₁₂ cycloalkyl, wherein the cycloalkyl group is optionally substituted and/or is optionally (partly) unsaturated and/or is optionally interrupted by one or more heteroatoms, wherein the heteroatoms are independently selected from O, N and S;
   (d) C₆ - C₂₀ aryl, wherein the aryl group is optionally substituted;
   (e) C₆ - C₂₀ heteroaryl, wherein the heteroaryl group comprises 1 - 3 heteroatoms independently selected from O, N and S and/or wherein the heteroaryl group is optionally substituted;
   (f) C₇ - C₂₀ alkylaryl wherein the alkylaryl group is optionally substituted and/or wherein an alkyl part of the alkylaryl group is optionally (partly) unsaturated and/or is optionally interrupted by one or more heteroatoms, wherein the heteroatoms are independently selected from O, N and S; and
   (g) C₇ - C₂₀ alkylheteroaryl, wherein the alkylheteroaryl group comprises 1 - 3 heteroatoms independently selected from O, N and S and/or wherein the alkylheteroaryl group is optionally substituted, and/or wherein an alkyl part of the alkylheteroaryl group is optionally (partly) unsaturated and/or is optionally interrupted by one or more heteroatoms, wherein the heteroatoms are independently selected from O, N and S;
E is independently selected from the group consisting of:
   -(CR⁸₂)ᵤ-N(R⁹)-C(O)-,
   -C(O)-N(R⁹)-(CR⁸₂)ᵤ-,
   -(CR⁸₂)ᵤ-N=CR⁹-,
   -C(R⁹)=N-(CR⁸₂)ᵤ-, and
   -(CR⁸₂)ᵤ-N(R⁹)-(CR⁸₂)ᵥ-;
R⁸ is independently selected from the group consisting of H and C₁ - C₂₀ alkyl;
R⁹ is independently selected from the group consisting of H and C₁ - C₂₀ alkyl;
t = 0 - 4;
u = 1 - 10;
v = 1 - 4;
in Formula (14), R¹² is independently selected from
   (a) C₁ - C₁₂ alkylene, wherein the alkylene group is optionally substituted and/or is optionally (partly) unsaturated and/or is optionally interrupted by one or more heteroatoms, wherein the heteroatoms are independently selected from O, N and S;
   (b) C₃ - C₁₂ cycloalkylene, wherein the cycloalkylene group is optionally substituted and/or is optionally (partly) unsaturated and/or is optionally interrupted by one or more heteroatoms, wherein the heteroatoms are independently selected from O, N and S;
   (c) C₆ - C₂₀ arylene, wherein the arylene group is optionally substituted;
   (d) C₆ - C₂₀ heteroarylene, wherein the heteroarylene group comprises 1 - 3 heteroatoms independently selected from O, N and S and/or wherein the heteroarylene group is optionally substituted;
   (e) C₇ - C₂₀ alkylarylene wherein the alkylarylene group is optionally substituted and/or wherein an alkyl part of the alkylarylene group is optionally (partly) unsaturated and/or is optionally interrupted by one or more heteroatoms, wherein the heteroatoms are independently selected from O, N and S; and
   (f) C₇ - C₂₀ alkylheteroarylene, wherein the alkylheteroarylene group is optionally substituted, and/or wherein an alkyl part of the alkylheteroarylene group is optionally (partly) unsaturated and/or is optionally interrupted by one or more heteroatoms, wherein the heteroatoms are independently selected from O, N and S;
   (g) Polyalkylene having a weight average molecular weight M_{w} of about 150 to about 10,000, wherein the polyalkylene group may be linear or branched and is interrupted by one or more heteroatoms, wherein the heteroatoms, are independently selected from O, N and S;
in Formula (15), R¹² is :
   (h) Polyalkylene having a weight average molecular weight M_{w} of about 1,000 to about 50,000, wherein the polyoxyalkylene has a (hyper)branched, multi-arm and/or dendrimeric structure and is interrupted by one or more heteroatoms, wherein the heteroatoms are independently selected from O, N and S; or

The term "poly(boronic acid) cross-linkers" comprises cross-linkers having more than one boronic acid end-group, e.g. three, four, five or six boronic acid end-groups, wherein R¹² is group (h).

The poly(boronic acid) cross-linkers preferably have a sufficient hydrophilicity and comprise amine groups, preferably primary amine groups, as terminal groups, wherein these amine groups constitute a part of the groups defined for group E (in the formulas below the amine groups are sometimes omitted). The poly(boronic acid) cross-linkers may have different spacers which comprise one or more heteroatoms selected from the group.consisting of O, N and S, preferably O and N. The poly(boronic acid) cross-linkers in compounds according to formula (14) preferably have a linear or branched structure. The poly(boronic acid) cross-linkers in compounds according to formula (15) preferably have a (hyper)branched, star, multi-arm or dendritic structure. It is also preferred for certain embodiments that the poly(boronic acid) cross-linkers comprise moieties providing (additional) hydrophilicity, e.g. PEG moieties. Suitable base structures for the poly(boronic acid) cross-linkers are Boltorn® (Perstorp), Astramer® (DSM), JEFFAMINE® (Huntsman), PANAM, PAMAM, PPI, PEAN and PEAC polymers. The terms "PANAM" and "PAMAM" refer to poly(amido amine) polymers. The term "PPI" means polypropylene imine polymers. The term "PEAN" refers to poly(ester amine) polymers. The term "PEAC" refers to poly(ether amine) polymers.

Preferred polyalkylenes according to group (g) are represented by JEFFAMINE® D, ED and EDR polymers. These polymers are commercially available with a weight average molecular weight M_{w} of about 150 to about 4,000. As described above, it will be apparent to those skilled in the art that the terminal amine groups of these polymers are part of group E defined above.

Preferred polyalkylenes according to group (h) are the polymers represented by Formulas (16) and (17), wherein x and y are selected such that the weight average molecular weight M_{w} is about 1,000 to about 50,000: 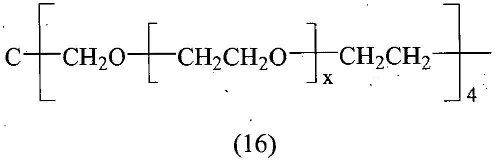 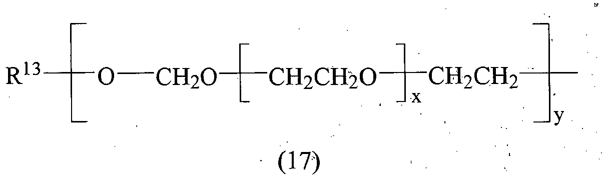 wherein R¹³ is a core structure derived from the group consisting of trimethylolpropane, pentaerythritol, ditrimethylolpropane, diglycerol, ditrimethylol-ethane, trimethylolpropane (hexaglycerol), tripentaerythritol, and mixtures thereof.

In formulas (16) and (17), it is preferred that y = 3 - 8, more preferably 4 or 8; wherein x is such that the compound according to Formula (16) or (17) has a weight average molecular weight M_{w} of about 1,000 to about 50,000. Such polyalkylenes may have up to eight arms and are commercially available from for example JenKem Technology and Creative PEGWorks with a weight average molecular weight M_{w} in the range of about 2,000 to about 40,000.

Another preferred group of polyalkylenes according to group (h) are the polymers of the JEFFAMINE® T series which are commercially available with a weight average molecular weight M_{w} in the range of about 440 to about 5,000.

Yet another group of preferred polyalkylenes according to group (h) are the polymers represented by Formula (18):

N{(R¹⁴)₃₋ₙ[(CR¹⁴₂)ₘ-N(R¹⁴)-(CR¹⁵₂)-]ₙ} (18)

wherein:
n = 2 or 3;
m = 2 - 12;
each R¹⁴ is independently selected from the group consisting of hydrogen atoms and C₁-C₆ alkyl groups;
R¹⁵ is independently selected from the group consisting of hydrogen atoms, C₁ - C₂₀ alkyl groups and groups of the formula (CR¹⁶₂)ₒN(R¹⁴)(CR¹⁷₂), wherein o = 1 - 11; each R¹⁶ is independently selected from the group consisting of hydrogen atoms and C₁ - C₆ alkyl groups; and R¹⁷ is selected from the group consisting of hydrogen atoms, C₁ - C₂₀ alkyl groups and groups of the formula -(CR¹⁸₂)ₚN(R¹⁷)(CR¹⁹₂), wherein p = 1 - 11, each R¹⁸ is as defined above for R¹⁶ and R¹⁹ is selected from the group consisting of hydrogen atoms, C₁ - C₂₀ alkyl groups and groups of the formula -(CR²⁰₂)_{q}N(R¹⁷)(CR²¹₂), wherein q = 1 - 11, each R²⁰ is as defined above for R¹⁶ and each R²¹ is as defined above for R¹⁶, wherein the alkyl groups may be linear or branched and may comprise one or more oxygen atoms.

### Examples

### Examples relating to nanoparticles

### Materials used

Dry toluene and dry THF were freshly distilled from Na/benzophenone. Cystamine bisacrylamide (CBA, Polysciences, USA), N-Boc-1,4-diaminobutane (Aldrich), benzoylchloride (Aldrich) andp-carboxy phenylboronic acid (4-CPBA, Aldrich) were of commercial grade and used without further purification. All reagents and solvents were of reagent grade and were used without further purification.

NMR spectra were recorded on a Varian Unity 300 (¹H NMR 300 MHz) using the solvent residual peak as the internal standard. FAB-MS spectra were recorded on a Finningan MAT 90 spectrometer with *m*-nitrobenzyl alcohol (NBA) as the matrix.

### Example 1

Polymers P1 - P4 according to Formula (18):: 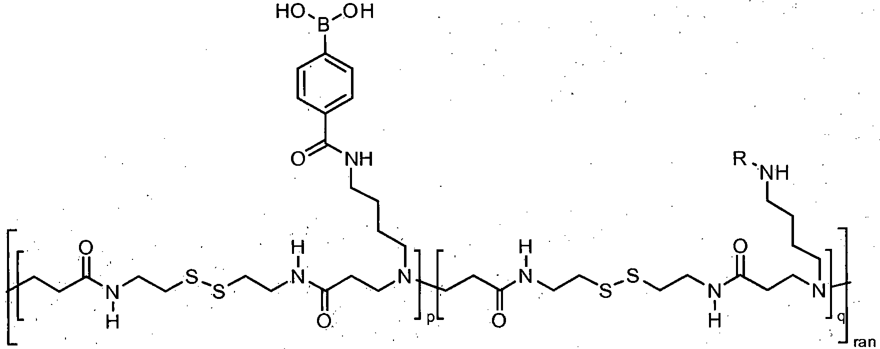
**P1**: R=H, p/q=20/80
**P2**: R=H, p/q=25/75
**P3**: R=H, p/q=35/70
**P4**: R=Ac, p/q=70/30
were prepared according to a process shown in Scheme 1.

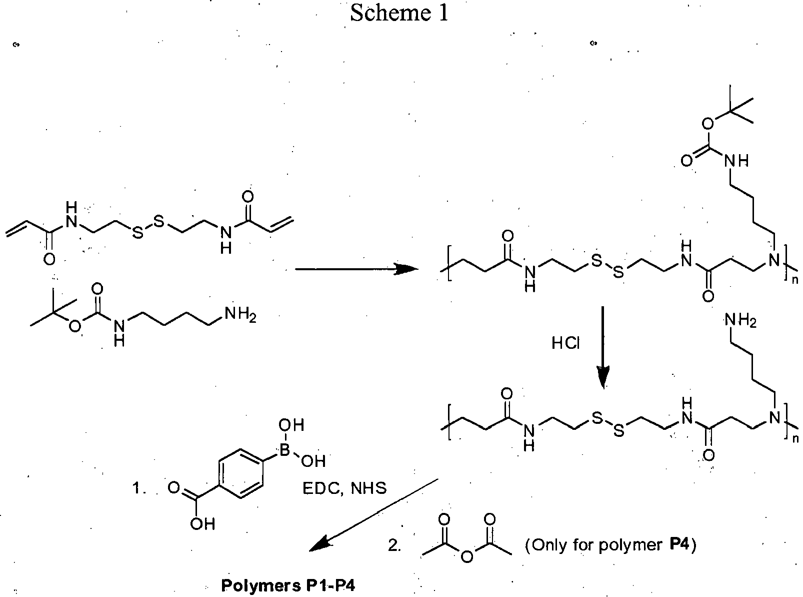

The first step was a Michael addition-polymerization of N-Boc-1,4-diaminobutane with equimolar amounts of cystamine bisacrylamide. Typically, 0.5 - 1.0 g of N-Boc-1,4-diaminobutane and cystamine bisacrylamide were added with 2 equivalents of triethylamine into a brown reaction flask with methanol : water 4:1 as the solvent to a final concentration of 2 M. The polymerization was carried out in the dark at 45 °C in a nitrogen atmosphere. The reaction mixture became homogeneous in less than 1 hour and the reaction was allowed to proceed for 6 - 10 days, yielding a viscous solution. Subsequently, 10 mol % excess of N-Boc-1,4-diaminobutane was added to consume any unreacted acrylamide groups and stirring was continued for 2 days at 45 °C. The resulting solution was diluted with water to about 30 ml, acidified with 4 M HCl to pH ~ 4, and then purified using a ultrafiltration membrane (MWCO 3000 g/mol). After freeze drying, the BOC-protected poly(amido amine) (BOC is N-tert-butoxycarbonyl) was collected as the HCl-salt. The composition of the BOC-protected poly(amido amine) was established by ¹H NMR (D₂O, 300 MHz). Next, the BOC-protected poly(amido amine) was dissolved in about 30 ml methanol and fully deprotected by bubbling dry HCl-gas through the solution for 20 minutes. The methanol was removed at a rotary evaporater and the polymer was redissolved in about 30 ml water, the pH was adjusted to ~ 4 using 4 M NaOH (aq). The polymer was then purified again using an ultrafiltration membrane (MWCO 3000g/mol). After freeze-drying, the poly(amido amine) was collected as the HCl-salt. The complete removal of the BOC groups was confirmed by ¹H NMR (D₂O, 300 MHz).

In a next step, p-carboxyphenylboronic acid (4-CPBA) (0.124g, 0.746mmol) was dissolved in 5 ml methanol by slightly increasing the temperature to 50 °C. About 2 equivalents of 1-ethyl-3-[3-dimethylaminopropyl]carbodiimide hydrochloride (EDC) (0.221g, 1.15mmol) dissolved in 5 ml millipore H₂O was added to the reaction mixture at ambient temperature. After a period of about 10 minutes, about 1.8 equivalents of N-hydroxy sulfosuccinimide (NHS) (0.126 g, 1.05 mmol) dissolved in 5 ml millipore H₂O was added. The mixture became a little turbid and the pH decreased to 5. The deprotected poly(amido amine) (0.232 g, 0.604 mmol NH₂) was dissolved in 25 ml millipore H₂O and added to the reaction mixture after another 30 minutes at ambient temperature. The reaction proceeded for 6 hours at ambient temperature under nitrogen in the absence of light. The polymer solution was then purified again using an ultrafiltration membrane (MWCO 3000g/mol). After freeze drying, the poly(amido amine) was collected as the HCl-salt. The yield was 78 % (0.206 g) and the degree of substitution was determined by ¹H NMR (D₂O, 300 MHz).

¹H NMR (Polymer **P1,** HCl salt, D₂O, 300 MHz):
δ 7.6-7.8 (0.8H, dd, Ar*H*), δ = 3.65 (4H, t, (2H, t, NHCO-CH₂-C*H*₂-N), δ = 3.55 (4H, t, SS-CH₂-C*H*₂-N), δ = 3.30. (1.6H, t, CH₂-C*H*₂-NH₃⁺), δ = 3.30 (0.4H, t, CH₂-C*H*₂-NHCO-Ar), δ = 3.15 (2H, t, R₂-N-C*H*₂-CH₂), δ = 2.90 (4H, t, NHCO-C*H*2-CH₂-N), δ = 2.75 (4H, t, C*H₂*-SS-C*H₂*), δ = 1.75 (2H, m, N-CH₂-C*H*₂-CH₂-CH₂-NH), δ = 1.55 (2H, m, N-CH₂-CH₂-C*H₂*-CH₂-NH)

¹H NMR (Polymer **P2**, HCl salt, D₂O, 300 MHz):
δ 7.6-7.8 (1H, dd, Ar*H*), δ = 3.65 (4H, t, (2H, t, NHCO-CH₂-C*H*₂-N), δ = 3.55 (4H, t, SS-CH₂-C*H*₂-N), δ = 3.30 (1.5H, t, CH₂-C*H₂*-NH₃⁺), δ = 3.30 (0.5H, t, CH₂-C*H₂*-NHCO-Ar), δ = 3.15 (2H, t, R₂-N-C*H₂*-CH₂), δ = 2.90 (4H, t, NHCO-C*H₂*-CH₂-N), δ = 2.75 (4H, t, C*H₂*=SS-C*H₂*), δ = 1.75 (2H, m, N-CH₂-C*H₂*-CH₂-CH₂-NH), δ = 1.55 (2H, m, N-CH₂-CH₂-C*H₂*-CH₂-NH)

¹H NMR (Polymer **P3**, HCl salt, D₂O, 300 MHz):
δ 7.6-7.8 (1.4H, dd, Ar*H*), δ = 3.65 (4H, t, (2H, t, NHCO-CH₂-C*H₂*-N), δ = 3.55 (4H, t, SS-CH₂-C*H₂*-N), δ = 3.30 (1.3H, t, CH₂-C*H₂*-NH₃⁺), δ = 3.30 (0.7H, t, CH₂-C*H₂*-NHCO-Ar), δ = 3.15 (2H; t, R₂-N-C*H₂*-CH₂), δ = 2.90 (4H, t, NHCO-C*H₂*-CH₂-N), δ = 2.75 (4H, t, C*H₂*-SS-C*H₂*), δ = 1.75 (2H, m, N-CH₂-C*H₂*-CH₂-CH₂-NH), δ = 1.55 (2H, m, N-CH₂-CH₂-C*H₂*-CH₂-NH)

In a next step, acetylation of the non-functionalised primary amino groups using acetic anhydride was performed, wherein the poly(amido amine) was dissolved in 40 ml methanol, excess acetic anhydride (four equivalents) and triethylamine (three equivalents) were added and the mixture was stirred overnight at 60 °C. The methanol was evaporated and the polymer was purified again using an ultrafiltration membrane (MWCO 3000 g/mol). After freeze drying, NMR showed complete acetylation of the primary amino groups.

¹H NMR (Polymer **P4**, HCl salt, D₂O, 300 MHz):
δ 7.6-7.8 (1.2H, dd, Ar*H*), δ = 3.65 (4H, t, (2H, t, NH-CO-CH₂-C*H₂*-N), δ = 3.55 (4H, t, SS-CH₂-C*H₂*-N), δ = 3.30 (1.4H, t, CH₂-C*H₂*-NHCO-CH₃), δ = 3.30 (0.6H, t, CH₂-C*H₂*-NHCO-Ar), δ = 3.15 (2H, t, R₂-N-C*H₂*-CH₂), δ = 2.90 (4H, t, NHCO-C*H₂*-CH₂-N), δ = 2.75 (4H, t, C*H₂*-SS-C*H₂*), δ = 2.05 (2.1H, t, NHCO-C*H₃*), δ = 1.75 (2H, m, N-CH₂-C*H₂*-CH₂-CH₂-NH), δ = 1.55 (2H, m, N-CH₂-CH₂-C*H₂*-CH₂-NH)

According to this procedure, the following polymers were prepared, wherein the amount of boronic acid groups was varied.

**Table 1**

| Polymer | p= (mol % 4-CPBA) | q= (mol % NHR) | R= | M_{w} (10³ g/mol) |
|---|---|---|---|---|
| **P1** | 20 | 80 | H | 3.4 |
| **P2** | 25 | 75 | H | 3.2 |
| **P3** | 35 | 65 | H | 4.9 |
| **P4** | 30 | 70 | Acetyl | 4.0 |

Molecular weights were determined using a Viscotek GPC System Model TDA-302 (operated without a column) equipped with a TDA-302 triple detector array consisting of a refractive index (RI) detector, a light scattering detector (7° and 90°) and a viscosity detector, together with OmniSec 4.1 software provided by Viscotek (Oss, The Netherlands). First, the samples were dried overnight over sicapent, and subsequently stirred to dissolve for 24 hours at a concentration of 5 mg/ml in a mixture of water/methanol 1/4 (v/v). Next, 20 µl of the sample was injected and dn/dc was calculated based on the RI response using the FIPA method.

### Example 2

This example shows that boronic acid reduces toxicity of particles formed with DNA. Polymers from Example 1 were used to form nanoparticles with DNA. It way found that the boronated polymers from Example 1 all formed nanosized polyplexes with positive surface charge. The influence of grafting on the cell viability in COS7 cells was systematically studied, see Table 2. It was found that the boronic acid polymers with low degree of functionalization show significantly improved cell viabilities.

**Table 2**

| Polymer | Functionalization (%) | Particle size^{a} (nm) | ζ-potential^{a} (mV) | Cell viability^{b} (%) |
|---|---|---|---|---|
| **P1** | 20 | 87.3±0.9 | 42.6±1.2 | 28±12 |
| **P2** | 25 | 97.4±0.3 | 28.9±3.8 | 51±11 |
| **P3** | 35 | 246.2±4.27 | 22.4±2.5 | 75±10 |
| **P4** | 30 | 70.7±0.6 | 30.0±1.4 | 105±17 |

| | | | | |
|---|---|---|---|---|
| ^{a} Determined at a polymer/DNA weight ratio of 24/1 ^{b} Determined at a polymer/DNA weight ratio of 24/1 in absence of serum | | | | |

### Example 3

### Synthesis of 2-((4-aminobutylamino)methyl)phenyl boronic acid (monomer M1)

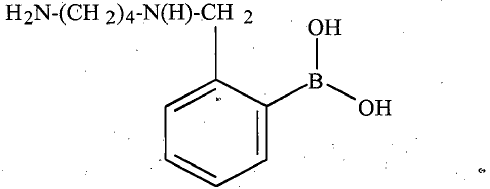

2-Formylphenylboronic acid (5.10 g, 0.0340 mol) was dissolved in 45 ml methanol, together with N-BOC-1,4-diaminobutane (6.69 g, 0.0347 mol) and 3 equivalents triethylamine (10.08 g, 0.0996 mol) and stirred overnight under nitrogen. The mixture turned yellow and ¹H-NMR confirmed the formation of the Schiff's base. Next, 3 equivalents NaBH₄ (3.91 g, 0.103 mol) were added to the reaction mixture to reduce the Schiff's base and the mixture turned colorless. The product was thrice extracted from water/chloroform. The organic layer was dried over MgSO₄ and evaporated under reduced pressure. The product was an off-white solid (8.78 g, 75 % yield). The BOC-protected product (3.74 g, 0.0116 mol) was dissolved in 30 ml of methanol and deprotected by bubbling HCl-gas through the reaction mixture for 30 minutes. The solvent was evaporated to dryness and ¹H-NMR showed complete deprotection. The monomer was used without further purification, but the titer was calculated to be 50 % using p-toluene sulfonic acid and ¹H-NMR. Impurities were due to bound water to the boronic acid and counterions.

¹H-NMR (monomer **M1**, HCl-salt, D₂O, 300 MHz):
δ - 7.1-7.4 (4H, m, Ar*H*), δ = 3.950 (2H, s, ArH-C*H*₂-NH); δ = 2.905 (2H, t, 3J(H-H) = 7.2 Hz, NH-C*H₂*-CH₂), δ = 2.825 (2H, t, 3J(H-H) = 7.5 Hz, CH₂-C*H₂*-NH₂), δ = 1.673 (2H, q, 5J(H-H) = 7.7 Hz, CH₂-CH₂-C*H₂*-CH₂-NH₂), δ = 1.601 (2H, q, 5J(H-H) = 8.4 Hz, CH₂-C*H₂*-CH₂-CH₂-NH₂).

MS (ESP-TOF): m/z= 239.3 (100) ([M+OH+ H⁺], calculated for [C₁₁H₁₉BN₂O₂.OH]: 239.15).

### Example 4

This example shows that boronated polymers according to the present invention have improved DNA condensation, and endosomal escape properties resulting in improved transfection efficiencies. Four different structures were compared. The first three were synthesized by polymerization of cystamine bisacrylamide and *N*-BOC protected diaminobutane followed by deprotection of the pending amines, according to the procedure described under Example 1. Different batches of the resulting polymer pCBA/DAB with M_{w} ranging between 4-6 kDa were pooled. From this parent batch three polymers functionalized with 30 % of either acetyl groups (Ac) (**P5**); benzoyl groups (Bz) (**P6**) or with 4-carbamoylphenylboronic acid (4-CPBA) (**P7**) were synthesized in the same way as described in Example 1. In a similar procedure cystamine bisacrylamide was copolymerizated with 30 % monomer **M1** (cf. Example 3) and 70 %*N*-BOC protected diaminobutane followed by deprotection with HCl(g), resulting in **P8**, with 30 % 2((amino)methyl)phenylboronicacid (30 %2AMPBA).

¹H NMR (Polymer **P5**, HCl salt, D₂O, 300 MHz):
δ = 3.65 (4H, t, (2H, t, NHCO-CH₂-C*H₂*-N), δ = 3.55 (4H, t, SS-CH₂-C*H₂*-N), δ = 3.30 (1.4H, t, CH₂-C*H₂*-NH₃⁺), δ = 3.30 (0.6H, t, CH₂-C*H₂*-NHCO-CH₃), δ = 3.15 (2H, t, R₂-N-CH₂-CH₂), δ = 2.90 (4H, t, NHCO-C*H₂*-CH₂-N), δ = 2.75 (4H, t, C*H₂*=SS-C*H₂*), δ = 2.05 (0.9H, t, NHCO-C*H₃*), δ = 1.75 (2H, m, N-CH₂-C*H₂*-CH₂-CH₂), δ = 1.55 (2H, m, N-CH₂-CH₂-C*H₂*-CH₂)

¹H NMR (Polymer **P6**, HCl salt, D₂O, 300 MHz):
δ 7.6-7.8 (1.5H, m, Ar*H*), δ = 3.65 (4H, t, (2H, t, NHCO-CH₂-C*H₂*-N), δ = 3.55 (4H, t, SS-CH₂-CH₂-N), δ = 3.30 (1.4H, t, CH₂-C*H₂*-NH₃⁺), δ = 3.30 (0.6H, t, CH₂-C*H₂*-NHCO-Ar), δ = 3.15 (2H, t, R₂-N-CH₂-CH₂), δ = 2.90 (4H, t, NHCO-C*H₂*-CH₂-N), δ = 2.75 (4H, t, C*H₂*-SS-C*H₂*), δ = 1.75 (2H, m, N-CH₂-C*H₂*-CH₂-CH₂), δ = 1.55 (2H, m, N-CH₂-CH₂-C*H*₂-CH₂)

¹H NMR (Polymer **P7**, HCl salt, D₂O, 300 MHz):
δ 7.6-7.8 (1.2H, dd, Ar*H*), δ = 3.65 (4H, t, (2H, t, NHCO-CH₂-C*H₂*-N), δ = 3.55 (4H, t, SS-CH₂-C*H₂*-N), δ = 3.30 (1.4H, t, CH₂-C*H₂*-NH₃⁺), δ = 3.30 (0.6H, t, CH₂-C*H₂*-NHCO-Ar), δ = 3.15 (2H, t, R₂-N-C*H₂*-CH₂), δ = 2.90 (4H, t, NHCO-C*H₂*-CH₂-N), δ = 2.75 (4H, t, C*H₂*-SS-C*H₂*), δ = 1.75 (2H, m, N-CH₂-C*H₂*-CH₂-CH₂), δ = 1.55 (2H, m, N-CH₂-CH₂-C*H₂*-CH₂)

¹H NMR (Polymer **P8**, HCl salt, D₂O, 300 MHz):
δ 7.7-7.8 (0.3H, m, Ar*H*), δ = 7.4-7.5 (0.9H, m, Ar*H*), δ = 4.2-4.4 (0.6H, m, ArH-C*H₂*-NH), δ = 3.65 (4H, t, (2H, t, NHCO-CH₂-C*H₂*-N), δ = 3.55 (4H, t, SS-CH₂-C*H₂*-N), δ = 3.30 (1.4H, t, CH₂-C*H₂*-NH₃⁺), δ = 3.30 (0.6H, t, CH₂-C*H₂*-NHCO-Ar), δ = 3.15 (2H, t, R₂-N-C*H₂*-CH₂), δ = 2.90 (4H, t, NHCO-C*H₂*-CH₂-N), δ = 2.75 (4H, t, C*H₂*-SS-C*H₂*), δ = 1.75 (2H, m, N-CH₂-C*H₂*-CH₂,-CH₂), δ = 1.55 (2H, m, N-CH₂-CH₂-C*H*₂-CH₂),

Polymers P1 - P4 according to Formula (20):

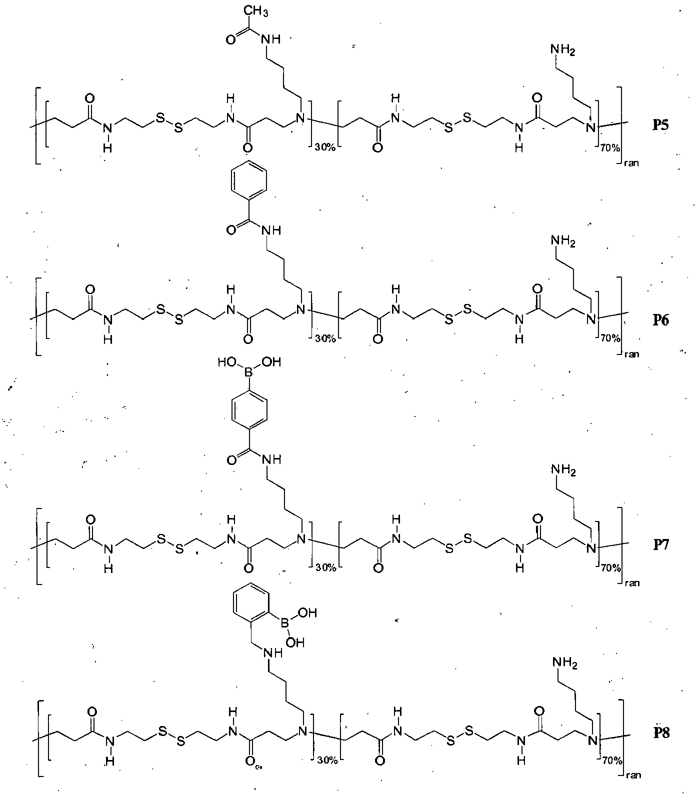

The synthesis of these polymers was according to Scheme 2:

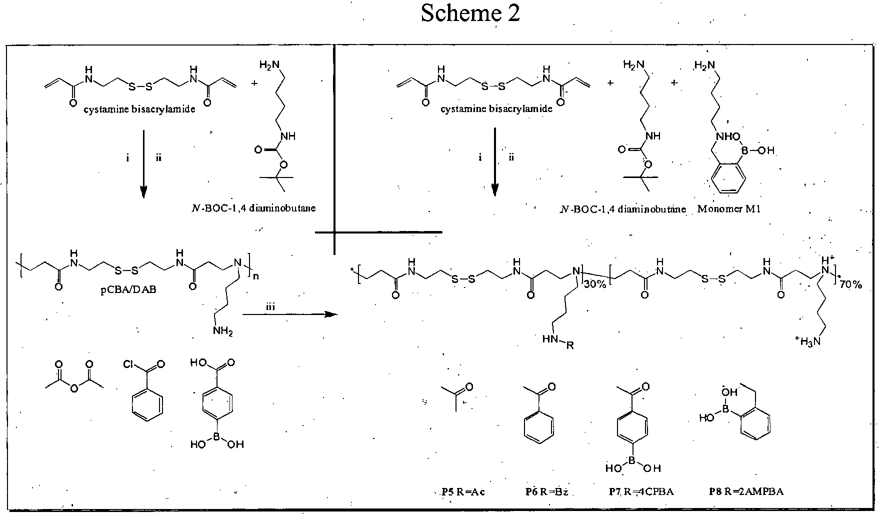

Polyplexes were prepared with polymer **P5 - P8** and polyplex size and ζ-potential were determined by light scattering, see Table 3. It was previously observed that functionalization of primary amines by acetylation results in somewhat larger particles with lower ζ-potentials. In polymers **P5 - P8** the primary amines were only partially functionalized. The benzoylated polymers form larger particles with increasing polymer concentration (at higher weight ratios). Without being bound by theory, this may be due to the increased hydrophobicity arising from the benzoylic groups. This does not apply to either of the boronic acid polymers, since they are able to form stable and nanoparticles smaller than 100nm.

The transfection efficiencies of these polyplexes were investigated in COS7 cells both in the presence and absence of serum. The transfection results were generally comparable to PEI, which is the gold standard for pDNA transfection. The transfection efficiency of the boronated polymer **P7** (30 % 4-CPBA) was slightly higher than **P5** (30 % Ac) or **P6** (30 % Bz) in the presence of serum, see Table 3. The boronated polymer **P7** is a very promising gene delivery vector (30 % 4-CPBA), since it shows similar transfection efficiencies both in absence and presence of serum. **P8** (30 % 2-AMPBA) results in very small and stable particles.

**Table 3**

| Polymer | Structure | Particle size^{a} (nm) | ζ-potential^{a} (mV) | Transfection efficiency^{b} (%) | Cell viability^{c} (%) |
|---|---|---|---|---|---|
| **P5** | 30% Ac | 83.3 (±0.2) | 38.9 (±1.0) | 22 (±1) | 94 (±5) |
| **P6** | 30% Bz | 256 (±9.2) | 46.5 (±1.6) | 55 (±4) | 89 (±6) |
| **P7** | 30% 4-CPBA | 67.7 (±1.0) | 37.8 (±1.0) | 66 (±7) | 74 (±12) |
| **P8** | 30% 2-AMPBA | 77.9 (±0.4) | 32.9 (±0.9) | 6 (±2) | 35 (±5) |

| | | | | | |
|---|---|---|---|---|---|
| ^{a} Determined at a polymer/DNA weight ratio of 24/1 ^{b} Determined at a polymer/DNA weight ratio of 24/1 in presence of serum, relative to PEI ^{c} Determined at a polymer/DNA weight ratio of 24/1 in presence of serum, relative to untreated control | | | | | |

### Example 5

This Example shows that the intracellular fate of the boronated polymers is altered and endosomal escape is improved due to the boronic acid moiety. The COS7 cells transfected with polyplexes from **P6** (30 % Bz) and **P7** (30 % 4-CPBA), see Example 4, were trypsinated after 2 days and these cells were analyzed by FACS. It was observed that a significant number of cells treated with polyplexes **P6** at the 48/1 polymer/DNA weight ratio cells appeared to have an apoptotic morphology. From the cells of the healthy population the percentage of GFP positive cells were determined as shown in Table 4. Both polymers **P6** and **P7** were able to transfect up to 94 % of the healthy cells. From this, it appears that both **P6** and **P7** are very efficient delivery vectors.

**Table 4**

| Polymer | Structure | 6/1 ratio | 12/1 ratio | 24/1 ratio | 48/1 ratio |
|---|---|---|---|---|---|
| **P6** | 30% Bz | 18.7% | 49.8% | 94.4% | n.d. |
| **P7** | 30% 4-CPBA | 13.4% | 12.3% | 29.3% | 89.3% |

The difference in Transfection efficiency between **P6** and **P7** was further investigated. Polyplexes were titrated from pH 7.4 to 5.1 in order to mimic the endosomal acidification process. In this experiment 10 ml of polyplexes at a 48/1 polymer/DNA weight ratio were prepared and titrated with 0.25 M NaOH (aq) from pH 5.1 to 7.4 and then back with 0.25 M HCl (aq) using the MPT-2 Autotitrator of the Zetasizer Nano (Malvern, UK).

The results are shown in Table 5. Both **P6** and **P7** form stable particles that are stable for several days at 37°C. Polyplexes of **P6** did not change in size in the range of pH 7.4 to 5.1. **P7** showed a reversible transition in size at pH 6.8 upon titration from pH 7.4 to 5.1 and back. The pH responsive properties of polyplexes of **P7** may favorably contribute to better endosomolytic properties of these polyplexes.

**Table 5**

| Polymer | Structure | pH 7.4 Size (nm) | pH 5.1 Size (nm) |
|---|---|---|---|
| **P6** | 30%Bz | 110 | 130 |
| **P7** | 30% 4-CPBA | 150 | 80 |

### Example 6

This Example shows that boronated polymers can bind with the glycoproteins on the cell membrane resulting in improved cell adhesion. Polymer **P7** (30 %CPBA) from Example 4 was used to prepare polyplexes at a 24/1 and 48/1 polymer/DNA weight ratio. Next, 1 % w/v sorbitol was added to the polyplex solution to block the boronic acid functionality. By blocking the boronic acid with the strong binding sorbitol, the gene expression (fluorescence signal caused by the GFP expressed) was significantly reduced. Especially for polyplexes formed at a 24/1 polymer/DNA weight ratio the transfection was almost completely reduced, see Table 6.

**Table 6.**

| Polymer **P7** | GFP expression Fluorescent intensity (a.u.) | |
|---|---|---|
| DNA/polymer ratio | No sugar added | 1 % (w/v) Sorbitol added |
| 24/1 | 1630 (±520) | 2 (±4) |
| 48/1 | 1410 (±450) | 810 (±120) |

### Example 7

Cystamine bisacrylamide (cf. Example 1), N-aminobutanol and the monomer **M1** according to Example 3 were polymerized to polymer **P9** (cf. Scheme 3).

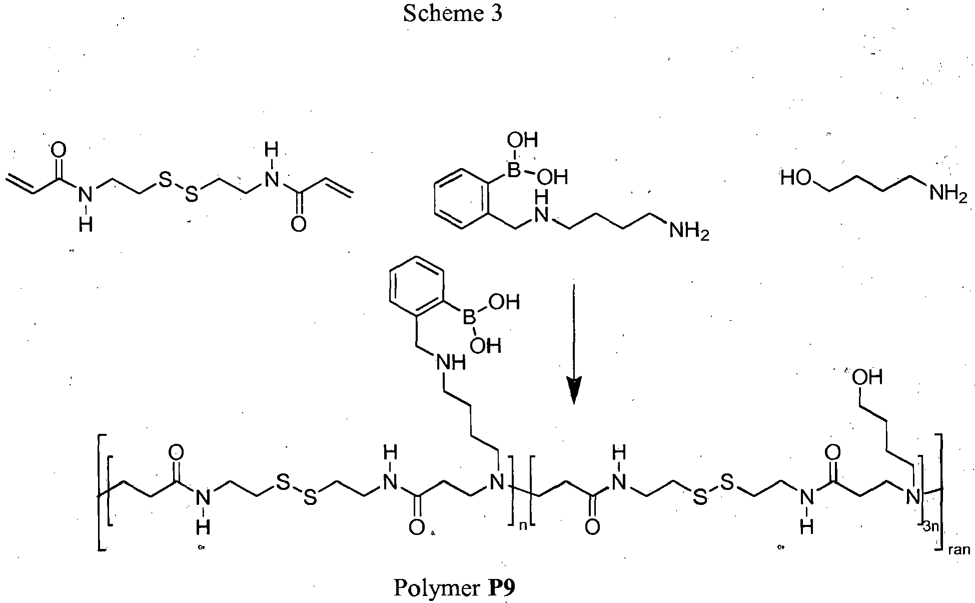

The polymer was synthesized by the Michael addition of CBA (1.18 g, 4.38 mmol), 75 % of N-aminobutanol (0.31 g, 3.37 mmol) and 25 % of monomer B2 (0.23 g, 1.0547 mmol). The reactants were dissolved in a mixture of methanol (1.6 ml), water (0.4 ml) and triethylamine (0.5 ml, 3.59 mmol). After 8 days reacting at 45 °C under nitrogen in the absence of light, the reaction was terminated by addition of excess monomer B2 (0.33 g, 1.47 mmol) dissolved in methanol (0.9 ml), water (0.6 ml) and triethylamine (0.23 ml). The termination reaction was left to proceed for another 5 days and the polymer was isolated using an ultrafiltration membrane (MWCO 3000 g/mol), yielding 1.14 g of off-white solid (66.5 % yield). The composition of the polymer was established by ¹H NMR (D₂O, 300 MHz) and the content of boronic acid side groups was 23 % with respect to the total amount of side groups.

¹H NMR (Polymer **P9**, HCl salt, D₂O, 300 MHz): δ 7.7-7.8 (0.23H, m, Ar*H*), δ = 7.4-7.5 (0.69H, m, Ar*H*), δ = 4.2-4.4 (0.46H, m, ArH-C*H*₂-NH), δ = 3.65 (4H, t, (2H, t, NHCO-CH₂-C*H₂*-N), δ = 3.55 (4H, t, SS-CH₂-C*H₂*-N), δ = 3.30 (1.64H, t, CH₂-C*H*₂-OH), δ = 3.30 (0.46H, t, CH₂-C*H₂*-NH-CH₂-Ar), δ = 3.15 (2H, t, R₂-N-C*H₂*-CH₂), δ = 2.90 (4H, t, NHCO-C*H₂*-CH₂-N), δ = 2.75 (4H, t, C*H₂*-SS-C*H₂*), δ = 1.75 (2H, m, N-CH₂-C*H₂*-CH₂-CH₂), δ = 1.55 (2H, m, N-CH₂-CH₂-C*H₂*-CH₂)

The molecular weight was 4,100 g/mol according to SLS with the FIPA method (cf. Example 1). The buffer capacity of 42 % was determined according to potentiometric titration (cf. Example 1).

This polymer was capable of forming stable aggregates by itself, with plasmid DNA and with siRNA. The properties of these nanoparticles are shown in Table 7.

**Table 7**

| | Polymer only | | | Polyplexes with pDNA | | Polyplexes with siRNA | |
|---|---|---|---|---|---|---|---|
| Conc. **P9** mg/ml | Size (nm) | ζ-potential (mV) | Ratio (w/w) | Size (nm) | ζ-potential (mV) | Size (nm) | ζ-potential (mV) |
| 0.09 | 116 (±1.1) | 35.9 (±2.43) | 6/1 | 624 (±7.8) | 12.0 (±0.4) | 695 (±46.7) | 12.3 (±0.1) |
| 0.18 | 95.3 (±1.4) | 35.6 (±3.03) | 12/1 | 147 (±0.7) | 21.5 (±1.0) | 243 (±3.83) | 20.7(±0.8) |
| 0.36 | 94.1 (±0.8) | 38.2 (±1.53) | 24/1 | 142 (±1.4) | 25.2 (±0.9) | 115 (±1.1) | 36.6 (±1.9) |
| 0.72 | 98.3 (±2.06) | 46.2 (±1.1) | 48/1 | 110 (±0.5) | 30.3 (±2.2) | 96.5 (±0.3) | 42.7 (±1.4) |

### Example 8

This Example shows that polymer **P9** has good transfection properties using plasmid DNA as well as good gene knockdown with siRNA. Transfection experiments were carried out with the DNA polyplexes of **P9** (cf. Example 7). An ONGP assay was performed to determine the transfection efficiency of particles with plasmid DNA encoding the LACz gene. It was observed that the **P9** gives a transfection efficiency similar to the positive control PEI (Exgen^{®}). In addition **P9** showed no significant cytotoxicity under these circumstances.

**Table 8**

| | With pDNA | | With siRNA | |
|---|---|---|---|---|
| Polymer/DNA ratio | Transfection rel. to PEI (%) | Cell viability (%) | Knockdown (%) | Cell viabilty (%) |
| 12/1 | 20 (±26) | 69 (±8) | 49.2 (±11.7) | 833 (±23) |
| 24/1 | 93 (±29) | 80 (±13) | 51.4 (±7.2) | 87 (±19) |
| 48/1 | 31 (±29) | 51 (±17) | 65.8 (±12.2) | 132 (±40) |

### Example 9

The boronic acid functionality in the polymers can be used for controlled delivery of drugs capable to form boronic ester groups. In this example, Alizarin Red S is taken as a model compound. The scheme below shows boronic ester formation of P9 (see Example 7) with Alizarin Red S (ARS).

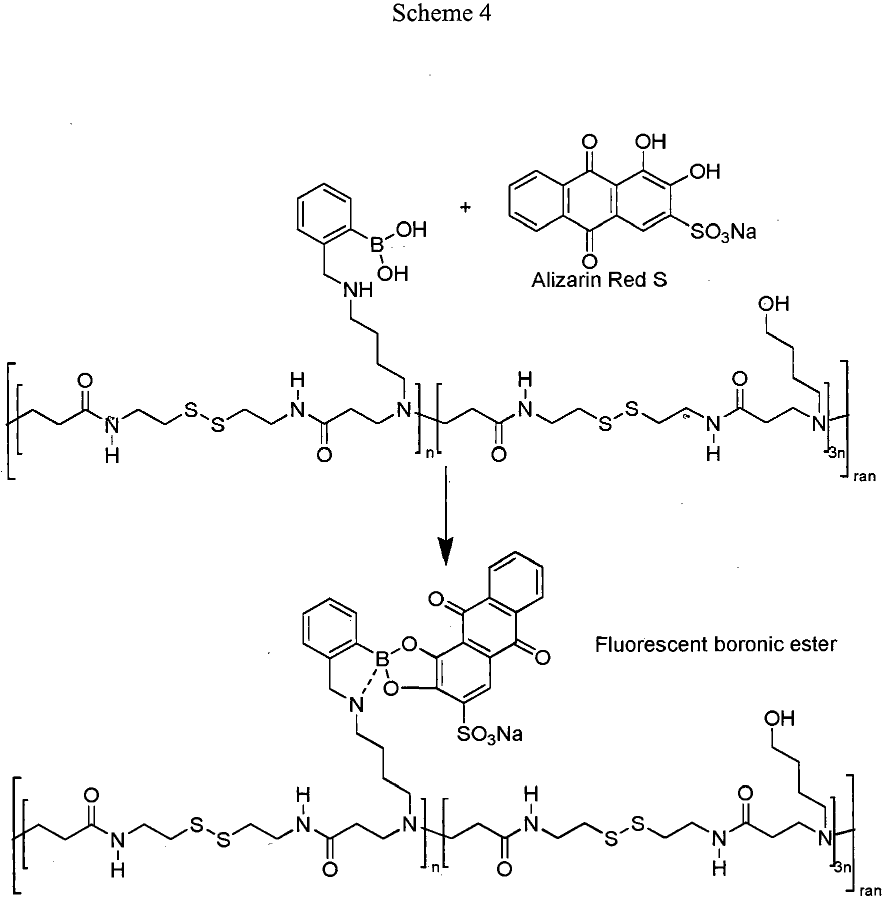

A binding constant of Kₐₛₛ = 2400 (±270) for the polymer-ARS complex was measured which is significantly higher than the Kₐₛₛ = 1600 (±170) determined for the monomer **M1** (cf. Example 3). Control experiments have shown that relevant amounts of ARS did not alter polyplex properties including size, charge, transfection or cell viability.

The boron-ARS complex is fluorescent and it was used for internalization studies to demonstrate the drug delivery capacities of the polymer. ARS was added to polymeric nanoparticles (as well as to nanosized polyplexes formed with pDNA). The presence of the fluorescent nanoparticles (with and without pDNA) in the cells was observed using confocal microscopy. The percentage of ARS positive cells was also determined with Fluorence Assisted Cell Sorting, see Table 9. Cell adhesion and internalization of the nanoparticles occurred within one hour for ca. 80 % of the cells.

**Table 9**

| Incubation time (hr) | (%) ARS positive cells |
|---|---|
| 0.5 | 68.4 |
| 1.0 | 83.3 |
| 2.0 | 76.0 |
| 4.0 | 78.1 |

### Example 10

This Example shows that polymers with a B-N interaction give diol binding at lower pH. The electron pair donating interaction of an adjacent amine base with a boronic acid group (B-N interaction) enhances the Lewis acidity of the boron center and facilitates the formation of boronic esters with diols at a lower (physiological) pH. Polymer **P9** (cf. Example 7) was used to form polyplexes which were stronger pH responsive than particles formed with polymer **P7** (cf. Example 4).

DNA-polyplexes prepared with polymer **P9** responded much stronger when different sugars were added to the polyplex solutions (to a final concentration of 1 % w/v). The binding of the sugars was evident from the decrease of ζ-potential of the polyplexes, see Table 10. This effect was not observed for polyplexes with polymer **P7**, lacking the dative B-N interaction.

**Table 10**

| | No sugar | Glucose | Galactose | Mannose | Sorbitol | Dextran |
|---|---|---|---|---|---|---|
| ζ-potential (mV) | 32.4 | 28.2 | 25.3 | 22.8 | 25.5 | 25.5 |

| | | | | | | |
|---|---|---|---|---|---|---|
| ^{a}polymer/DNA weight ratio 48/1 | | | | | | |

### Example 11

This Example shows that post-modification of particles with sugars enables receptor mediated uptake of boronated particles. Using boronated nanoparticles enables relatively easy post-modification with sugars for targeting purposes, without additional complicated synthetic procedures..

Polyplexes formed with polymer **P9** and plasmid DNA at a 48/1 polymer/IDNA weight ratio (cf. Example 8) were used in transfection studies in COS7 cells, which is a kidney cell line derived from green African monkeys. Table 11 shows that addition of sugars generally results in enhancement of the transfection (in particular for galactose), while maintaining good cell viability.

**Table 11**

| | No sugar | Glucose | Mannose | Galactose | Sorbitol | Dextran |
|---|---|---|---|---|---|---|
| Transfection rel. to PEI (%) | 35 (±3) | 41 (±23) | 34 (±10) | 147 (±43) | 67 (±19) | 89 (±4) |
| Cell viability (%) | 113 (±2) | 90 (±8) | 95 (±6) | 104 (±9) | 106 (±7) | 112 (±1) |

### Example 12

This Example discloses dextran coated nanoparticles with boronated polymers. The effect of particle coating by dextran was studied with atomic force microscopy (AFM) and scanning electron microscopy (SEM). To polyplexes of polymer **P9** and pDNA at 48/1 and 24/1 polymer/DNA weight ratio dextran was added to a final concentration of 1 % w/v. Polyplexes without dextran and Polyplexes with dextran, together with the control of dextran only, were put on a gold substrate and washed with water and then dried to the air. In the presence of dextran, uniform nanoparticles were observed on the gold surface with similar sizes as found in DLS experiments. Without being bound by theory, this is a strong indication that dextran forms a stabilizing coating around the nanoparticles, thereby preventing the disulfide bonds from interaction with the gold surface. In a control experiment it was shown that dextran alone does not form nanosized particles by itself.

The dextran particle coating was confirmed in a transfection study using a neuroblastoma cell line (SH-SY5Y). The viability of these cells is very vulnerable towards cationic vectors. Particles with polymer **P9** were prepared with pDNA encoding for GFP at 24/1 polymer/DNA weight ratio, both in absence and presence of 1 % (w/v) dextran. GFP gene expression was measured using a fluorescent plate reader or an automated confocal microscope observing co-localization of GFP and a DAPI staining of nuclei (absolute % transfected cells). For the dextran coated particles, a doubling of the transfection efficiency was observed, while the cells kept their healthy morphology.See Table 12.

**Table 12**

| Polyplex pretreatment | GFP expression (a.u.) | Absolute (%) transfection |
|---|---|---|
| Nothing added | 48 (±8) | 20.1 |
| Dextran (1 % w/v) | 210 (±59) | 40.6 |

### Example 13

WO 95/20591 discloses that boronic acids can form relatively strong esters with salicylhydroxamic acids (SHA). This enables a route to reversible, pH-sensitive, attachment of compounds functionalized with the SHA group to the boronated poly(amido amine)s . This Example shows p*ost*-PEGylation with SHA-PEG of boronated nanoparticles based on polymer P9 (cf. Scheme 5).

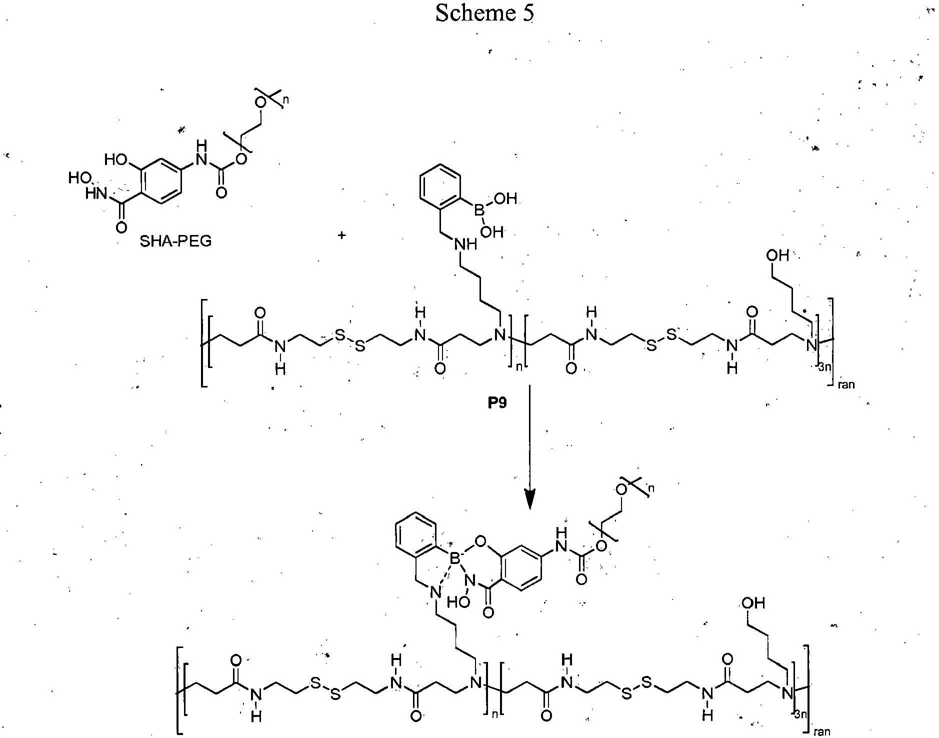

SHA-PEG was added stepwise to a polyplex solution of polymer **P9** at a 48/1 polymer/DNA weight ratio (see Example 7 for experimental details). A gradual decrease in ζ-potential was observed, indicating an increased shielding of the surface charge by the PEG ligation to the polyplexes (Table 13).

**Table 13.**

| SHA-PEG (mg/ml) | Size (nm) | ζ-potential (mV) |
|---|---|---|
| 0.00 | 113 (±1.7) | 45.2 (±1.8) |
| 0.35 | 116 (±1.6) | 41.7 (±1.1) |
| 0.70 | 124 (±0.3) | 37.3 (±1:0) |
| 1.05 | 121 (±5.3) | 34.4 (±1.5) |
| 2.10 | 161 (±4.5) | 3.2.9 (±1.1) |

### Example 14

This Example shows that boronated nanoparticles can be post-modified with polyvinyl alcohol (PVA). Examples 12 and 13 show dynamic covalent binding between dextran and PEG coated nanoparticles. This example further shows dynamic covalent binding between polymer **P9** and polyvinyl alcohol. Polymer **P9** forms spontaneously nanoparticles in Hepes buffer solution (pH=7.4) and to this solution, containing 0.9 mg/ml **P9** nanoparticles, different amounts of polyvinyl alcohol (PVA) were added, leading to PVA concentrations in the range of 0.1-3.3 % w/v. A moderate increase in nanoparticle size was measured (after correction for the increase of the viscosity of the solution due to PVA addition). Moreover, a significant reduction of the ζ-potential was observed which was not observed for nanoparticles from polymers without boronic acid functionalities. See Table 14. Without being bound by theory, this indicates that PVA forms a charge-shielding coating around the nanoparticle. At PVA concentrations of 3.33 % w/v and higher the beginning of gelation was observed. These effects were also observed for the other boronated polymers, including **P7**, but not for polymers without boronic acid moieties. Without being bound by theory, this is a strong indication that the boronated poly(amido amine)s can also be applied for the preparation of functionalized hydrogels.

**Table 14**

| Polymer **P9** (0.9 mg/ml) | | |
|---|---|---|
| PVA (w/v %) | Size (nm) | ζ-potential (mV) |
| 0.00 | 67.7 (±1.0) | 44.1 (±1.1) |
| 0.10 | 71.1 (±0.9) | 39.6 (±1.5) |
| 0.91 | 79.4 (±2.2) | 15,4 (±0.8) |
| 3.33 | 85.6 (±0.8) | 1,58 (±0.43) |

Besides the unloaded nanoparticles formed by self assembly of polymer **P9**, as discussed above, also polyplexes of **P9**/DNA (48/1 w/w) were investigated. The effects of PVA addition on the transfection and cell viability are low, up to PVA concentrations of 0.9 %. However, at higher concentration of 3.3 % PVA, the ζ-potential drops to almost neutral, indicating the shielding of the surface charge of the Polyplexes by the formation of a PVA corona (see Table 15). As expected, the gel-like PVA corona around these polyplexes prevented cellular uptake and consequently no transfection or cytotoxicity was observed for these particles.

**Table 15**

| PVA (w/v %) | Polyplex size (nm) | ζ-potential (mV) | Transfection rel. to PEI (%) | Cell viability (%) |
|---|---|---|---|---|
| 0.00 | 116.0 (±1.1) | 41.0 (±0.8) | 340 (±150) | 45 (±5) |
| 0.10 | 125.6 (±0.8) | 35.4 (±1.6) | 390 (±140) | 68 (±5) |
| 0.91 | 139.0 (±0.3) | 14.6 (±0.4) | 310 (±40) | 64 (±6) |
| 3.33 | 130.0 (±1.4) | 2.43 (±0.25) | 10 (±10) | 110 (±5) |

This Example demonstrates that after coating of the boronated nanoparticles with polyvinyl alcohol (PVA), gel formation starts to occur at higher PVA concentrations. PVA is a commonly used polymeric polyol that can interact strongly with boronic acids.

### Examples relating to hydrogels

The following examples will deal with boronated polymers based on methylene bisacrylamide (MBA). Firstly, some examples of the dynamic reversible covalent crosslinking of polyvinyl alcohol based hydrogels are given using the novel boronated poly(amido amine)s. In addition, highly water-soluble dimeric boronic acid crosslinkers (either based on JEFFamines or a xylene dipyridinium linker) were synthesized in order to produce bulk hydrogels with PVA alone or to serve as additional crosslinkers to reinforce PAA-PVA hydrogels.

### Example 15

Methylene bisacrylamide (MBA), N-Boc-1,4-diaminobutane were polymerized to pMBA/DAB and functionalized with 2-formyl phenyl boronic acid (2FPBA), 4-formyl phenyl boronic acid (4FPBA) or benzaldehyde through reductive amination to yield polymer **P10, P11** and **P12** respectively (cf. Scheme 6).

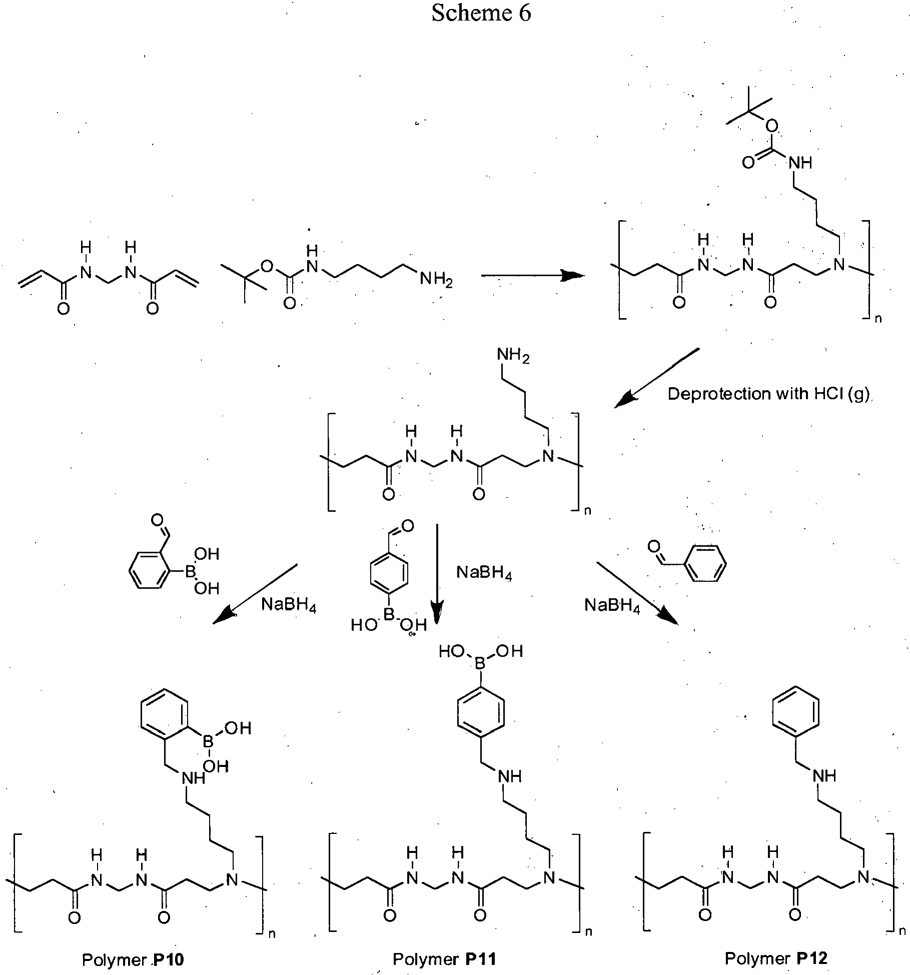

Polymers P10, **P11** and **P12** were prepared according to the following method. MBA (3.5 g, 22.5 mmol) and N-Boc-1,4-diaminobutane (4.37 g, 22.5 mmol) were added into a brown reaction flask with methanol/deionized water (11.6 ml, 171, v/v) as solvent mixture. The reaction system was stirred at 45 °C in the dark, under nitrogen-atmosphere. Within 10 minutes the reaction mixture was homogeneous and the polymerization was left to proceed for seven days, until the solution had become viscous. Subsequently, 12 mol % excess (0.52 g, 2.68 mmol) of N-Boc-1,4-diaminobutane in 3.6 ml methanol/deionized water (1/1, v/v) was added to terminate the reaction for three days. Water was added to the polymerization mixture. The pH of the resulting suspension was set to approximately 5 with aqueous HCl (1M) until the solution became clear again and was then purified by ultrafiltration through a membrane (3000 Dalton M_{w} cut-off) with deionized water (pH ~ 5). After freeze-drying, the base polymer pMBA/N-Boc 1,4 diaminobutane was collected as a white foam-like material.

¹H NMR (D₂O, 300 MHz): δ = 4.4 (2H, s, CONH-C*H*₂-NHCO),; δ = 3.3 (4H, t, (2H, t, CONH-CH₂-C*H₂*-N), δ = 3.1 (2H, t, CONH-CH₂-CH₂)₂-N-C*H₂*), δ = 2.9 (2H, t, ArH-CH₂-NH-C*H₂*, δ = 2.6 (4H, t, N-CH₂-C*H₂*-CONH), δ = 1.6 (2H, t, N-CH₂-C*H₂*-CH₂-CH₂-NH), δ = 1.4 (2H, t, N-CH₂-CH₂-C*H₂*-CH₂-NHCO), δ = 1.2 (9H, s, NHCO-O-C(C*H₃*)₃)

For the deprotection of the parent polymer, 3.65 g was dissolved in methanol (100 ml) and HCl gas was bubbled through the solution for 1 hr. The solvent was evaporated and the residue dried *in vacuo* for 2 days. The HCl-salt of the deprotected base polymer was obtained as a white powder and stored in the freezer after drying. Polymers **P10** and **P11** were synthesized by post-modification of the deprotected parent polymer with 2-formyl phenylboronic acid (2-FPBA) and 4-formyl phenylboronic acid (4-FPBA), respectively, from a single parent batch. For all syntheses 0.85 g (3.5 mmol of the repeating unit) of the HCl salt of the deprotected parent polymer was dissolved in methanol (20 ml) and an excess oftriethylamine was added to deprotonate the (primary) amines of the polymer. Excess of 2-FPBA (1.26 g, 8.14 mmol) and 4-FPBA (1.24 g, 7.90 mmol) were added to the polymer solutions, respectively. The reaction mixture was stirred at ambient temperature under nitrogen atmosphere. The reaction was allowed to proceed for two days, in which a yellow darkening was observed for the solutions with 2- and 4-FPBA which indicated the formation of the Schiff's base. Reduction of the Schiff's bases occurred through an excess addition of NaBH₄ (0.38 g, 10 mmol) to the solution portion wise to control the heat generation and hydrogen gas evolution. Another color change was observed in the reaction flasks with 2- and 4-FPBA (lighter). The solvent was removed by rotation evaporation under reduced pressure. Deionized water was added to the polymerization mixtures, resulting in suspensions. The pH was adjusted to approximately 5 with aqueous HCl (4 M) until the turbid suspensions became clear solutions and some insoluble material was removed by vacuum filtration. The filtrates were then purified by ultrafiltration with a 1000 Dalton cut-off membrane with deionized water (pH - 5). The solution with **P10** remained slightly turbid. After freeze-drying, the functionalized polymers were collected as white/yellowish foam-like material (**P10**) and as a white foam-like material (**P11**).

Polymer **P12** was prepared according to the same method with benzaldehyde (0.88 g, 8.25 mmol) as the aldehyde compound in the *post*-modification step. The reaction mixture with turned turbid and became clear again during the reduction. **P12** was isolated as a white solid material. Yield of the polymers were around 50 % after ultrafiltration.

¹H NMR (Polymer **P10**, HCl salt, D₂O, 300 MHz): δ 7.7-7.8 (1H, m, Ar*H*), δ = 7.4-7.5 (3H, m, Ar*H*), δ = 4.55 (2H, s, CONH-C*H*₂-NHCO), δ = 4.2-4.4 (2H, m, ArH-C*H*₂-NH), δ = 3.45 (4H, t, NHCO-CH₂-C*H₂*-N), δ = 3.25 (2H, t, NHCO-CH₂-CH₂)₂-N-C*H₂*), δ = 3.10 (2H, t, ArH-CH₂-NH-C*H₂*, δ = 2.75 (4H, t, N-CH₂-C*H₂*-CONH), δ = 1.75 (4H, m, N-CH₂-C*H₂*C*H₂*-CH₂-NH)

¹H NMR (Polymer **P11**, HCl salt, D₂O, 300 MHz): δ = 7.4-7.7 (4H, dd, Ar*H*), δ = 4.45 (2H, s, CONH-C*H*₂-NHCO), δ = 4.18 (2H, s, ArH-C*H*₂-NH); δ = 3.35 (4H, t, NHCO-CH₂-C*H₂*-N), δ = 3.08 (2H, t, NHCO-CH₂-CH₂)₂-N-C*H*₂), δ = 3.08 (2H, t, ArH-CH₂-NH-C*H₂*, δ =2.65 (4H, t, N-CH₂-C*H₂*-CONH), δ = 1.75 (4H, m, N-CH₂*-CH₂*-C*H₂*-CH₂-NH)

¹H NMR (Polymer **P12**, HCl salt, D₂O 300 MHz): δ = 7.4-7.8 (5H, m, Ar*H*), δ = 4.55 (2H, s, CONH-C*H*₂-NHCO), δ = 4.2 (2H, s, ArH-C*H*₂-NH); δ = 3.4 (4H, t, NHCO-CH₂-C*H₂*-N), δ 3.15 (2H, t, NHCO-CH₂-CH₂)₂-N-C*H₂*), δ = 3.08 (2H, t, ArH-CH₂-NH-C*H₂*, δ = 2.75 (4H, t, N-CH₂-C*H₂*-CONH), δ = 1.75 (4H, m, N-CH₂-C*H₂*-C*H₂*-CH₂-NH)

### Example 16

This Example shows that boronated poly(amido amine)s form pH-responsible hydrogels with polyvinyl alcohol that can be varied in mechanical strength from weak to ultra-rigid. Hydrogels of boronated polymers with polyvinyl alcohols were prepared as follows. Solutions with different concentrations of polyvinyl alcohol (M_{w} = 16, 27, 47, 72, 125 and 195 kDa) were prepared by dissolving the PVA in deionized water at 80 °C to obtain stock solutions of 10 % w/v that were diluted (10 %, 7.5 %, 5 %, 2.5 % w/v). Stock solutions of the polymers **P10 - P12** (10 %, 7.5 %, 5 %, 2.5 % w/v) were also prepared by dissolving the polymer in deionized water followed by dilution.

The pH of the stock solutions of **P10 - P12** was measured and in appropriate cases adjusted with aqueous HCl (1 M) or NaOH (1 M). To investigate gelation, the solutions were mixed and vortexed for 5 seconds in closed vials. **P10** and **P11** almost instantaneously formed hydrogels upon mixing with polyvinyl alcohol solutions, and in all cases gelation was observed within 5 seconds of vortexing. The resultant gels were allowed to equilibrate for at least 48 hours at room temperature before any rheological measurements were performed. However, **P12** (cf. Example 17), lacking the boronic acid moieties, did not form hydrogels with polyvinyl alcohol under any of the circumstances studied.

The results for **P10** are summarized in Table 16. From this it is shown that the gels are strongly pH-responsive and that, dependent on the concentration and pH, reversible gels can be made that vary in mechanical strength from weak to ultra-rigid (hard) material.

**Table 16**

| pH | **Polymer P10** (concentration % w/v) | **PVA** (**M_{w}**, conc. % w/v) | Relative gel strength^{a} |
|---|---|---|---|
| *Effect of M_{w} of PVA* | | | |
| 4.84 | 5 | 16 kDa, 5 | ++ |
| 4.84 | 5 | 47 kDa, 5 | ++ |
| 4.84 | 5 | 72 kDa, 5 | +++ |
| 4.84 | 5 | 125 kDa, 5 | +++ |
| 4.84 | 5 | 195 kDa, 5 | +++ |
| *Effect of pH* | | | |
| 4.02 | 5 | 47 kDa, 5 | +++ |
| 5.01 | 5 | 47 kDa, 5 | ++ |
| 6.00 | 5 | 47 kDa, 5 | + |
| 7.08 | 5 | 47 kDa, 5 | - |
| *Effect of PVA concentration* | | | |
| 4.96 | 7.5 | 47 kDa, 2.5 | - |
| 4.96 | 7.5 | 125 kDa, 2.5 | + |
| 4.96 | 7.5 | 125 kDa, 5.0 | +++ |
| 4.96 | 7.5 | 125 kDa, 7.5 | ++++ |
| 4.96 | 7.5 | 125 kDa, 10.0 | +++++ |

| | | | |
|---|---|---|---|
| ^{a}+++++ = ultra rigid; ++++ = very rigid; +++ = rigid; ++ = soft; + very soft; - = no gel. | | | |

### Example 17

### Dynamic rheology of the hydrogels

Rheology experiments were performed on a US 200 Rheometer (Anton Paar). Parallel plates with 25 mm diameter, between which the gels were squeezed and a gap of 1 mm (gel thickness) were used in all experiments. The gels were subjected to oscillating rotational deformations with angular frequencies (ω) from 0.1 tot 500 s⁻¹ (frequency sweeps) at a strain (γ) of 2 %. For each gel a strain sweep from 0.01 to 100 % at an angular frequency of 10 s⁻¹ was taken afterwards, to determine the linear viscoelastic region, as the frequency sweep should be run in this range. To check the reproducibility samples were prepared in duplo and each sample was measured 2-4 times. Measurements were performed at 25 °C, and for a number of samples also at 37 °C after temperature equilibration, in order to evaluate the effects of the temperature. Before each new frequency sweep, oscillatory time sweeps at 2 % strain and low angular frequency (1 s⁻¹) conditions were performed until the gel was stabilized (constant value of the storage and loss modulus in time). To prevent evaporation of water from the gel, a thin layer of silicon oil was applied on top. From each frequency sweep a crossover frequency (ω_{c}) was determined at the angular frequency where G' = G". From this crossover frequency the characteristic relaxation time (τ) could be calculated, with the formula: τ = 2π/ ωc. Another characteristic value that was obtained from the frequency sweep is the plateau value of the storage modulus (G'max) at high frequencies.

The data obtained for hydrogels made from **P10** and **P11** (5 % w/v) (cf. Example 18) and PVA 47 kDa (5 % w/v) at pH ~ 5 and at 25 °C are given in Table 17.

**Table 17**

| | Polymer **P10** | Polymer **P11** |
|---|---|---|
| Storage modulus (G') | 1880 | 2410 |
| Relaxation time (s) | 1.48 | 8.58 |

Both polymers **P10** and **P11** formed hydrogels with identical plateau gel strengths (G' plateau) of about 2 kPa at pH 5. The hydrogel prepared with polymer **P10** was more dynamic, since the relaxation time (1.48 s) was significantly lower than for the more shape-stable hydrogel prepared with **P11** (8.58 s). Hence, the dynamic (self-healing) properties of the hydrogels can be tuned by the type of boronic acid present in the polymers.

The hydrogel made from **P10** was tested at various temperatures and various pH values as well (Table 18). The data shown in this table demonstrate that the decrease in pH results in a significant increase in hydrogel strength for the **P10** polymer.

**Table 18**

| T (°C) | pH | G'max (Pa) | Relaxation time (s) |
|---|---|---|---|
| 25 | 6 | 160 | 0.47 |
| 25 | 5 | 800 | 0.94 |
| 25 | 4 | 2040 | 1.95 |
| 37 | 6 | * | * |
| 37 | 5 | 790 | 0.52 |
| 37 | 4 | 1730 | 1.17 |

| | | | |
|---|---|---|---|
| *No gel formed | | | |

### Example 18

This Example shows the influence of the PVA concentration on the gel strength (G'plateau) of the PAA/PVA hydrogels. Hydrogels were made from **P10** (7.5 % w/v) and PVA (125 kDa) at different concentrations at pH ~ 5 and 25 °C according to the general method of Example 18 (cf. Table 19). By varying the PVA concentration from 2.5 to 10 % w/v, the gel strength could be varied from weak to strong, whereas the self-healing character remained intact. The latter is observed by the relaxation time, which is not significantly influenced by the PVA concentration.

**Table 19**

| PVA (125 kDa) concentration (% w/v) | G'max (Pa) | Relaxation time (s) |
|---|---|---|
| 2.5 | 570 | 2.6 |
| 5.0 | 5500 | 3.8 |
| 7.5 | 13800 | 4.1 |
| 10 | 19400 | 3.8 |

### Example 19

This Example shows the influence of the PVA molecular weight on the gel strength (G' plateau) of the PAA/PVA hydrogels. Hydrogels were made from **P10** (5 % w/v) and PVA (5 % w/v) at different molecular weights at pH ~ 5 and 25 °C according to the general method of Example 18 (cf. Table 20). It was observed that by varying the M_{w} of the PVA from 27 kDa to 195 kDa the gel strength can be varied from weak to strong. Moreover, the self healing character can be tuned by varying the M_{w} of PVA. The increased shape stability is indicated by the relaxation time, which is significantly higher (tenfold) for the 195 kDa PVA compared to the 27 kDa PVA.

**Table 20**

| PVA (M_{w}, kD) | G'max (Pa) | Relaxation time (s) |
|---|---|---|
| 27 | 630 | 0.54 |
| 47 | 1100 | 0.95 |
| 72 | 4050 | 2.73 |
| 125 | 4200 | 3.34 |
| 195 | 4760 | 5.63 |

### Example 20

This Example shows that PAA/PVA hydrogels have thermoreversible behavior. The thermoreversibility of a hydrogel made from **P10** (5 % w/v) and PVA (47 kDa, 5 % w/v) according to the general method of Example 16 was tested by performing multiple frequency sweeps at alternating temperatures (25 °C and 37 °C). G'max and relaxtion time were determined for each measurement. The data are given in Table 21. The sample reached the original plateau value (G'max) upon the second cooling and heating, demonstrating thermoreversible behavior of the gel.

**Table 21**

| *Temperature run:* | *heating* | | *cooling* | |
|---|---|---|---|---|
| | 25 °C | 37 °C | 25 °C | 37 °C |
| G'max (Pa) | 1200 | 1040 | 1180 | 1030 |
| Relaxation time (s) | 0.80 | 0.44 | 0.81 | 0.45 |

### Example 21

This Examples shows that the swelling and degradation of the PAA/PVA hydrogels is influenced by the addition of glucose. For swelling and degradation tests, gels were prepared according to the general method of Example 18 from **P10** (5 % w/v) and PVA (195 kD, 7.5 % w/v) at pH ~ 5. The gel was transferred into empty vials of known weight and the initial weight of the gels was determined (t=0). Aqueous glucose solutions (10 ml) of five different concentrations (10 %, 5 %, 2.5 %, 1 % and 0 % w/v) were put on top of the gels. The gels were stored at ambient temperature, and after decantation of the supernatant the remaining gels were weighed. This procedure was repeated at regular time intervals in which fresh solutions were added to the gels after each weighing. The swelling ratio is defined as the weight of the swollen gel (Wₜ) after time t (1 hour, 4 hours and 7 hours, respectively) divided by the initial weight of the gel at t = 0 (Wo). The decrease of the swelling ratio in time from 1 hr to 4-7 hrs is an indication for the rate of gel degradation under the conditions given. From Table 22 it can be observed that increasing the glucose concentration from 0-10 % (w/v) will result in faster degradation of the gels. This is demonstrated by the swelling ratio after 4 hours, where the gels with 0 % and 1 % (w/v) glucose are still intact and the gels with 5 % and 10 % (w/v) glucose are mostly degraded.

**Table 22**

| [Glucose] (% w/v) | Swelling ratio after 1hour (%) | Swelling ratio after 4 hours (%) | Swelling ratio after 7 hours (%) |
|---|---|---|---|
| 0 | 121±16 | 90±1 | 19±2 |
| 1 | 121±6 | 99±6 | 15±4 |
| 2.5 | 108±4 | 69±12 | 16±3 |
| 5 | 72±16 | 37±2 | 11±1 |
| 10 | 44±2 | 29±1 | 13±1 |

| | | | |
|---|---|---|---|
| ^{a} 5 % (w/v) **P10** and 7.5 % (w/v) PVA | | | |

### Example 22

This Example discloses the synthesis of bifunctional crosslinkers based on α,ω-di(boronic acid) substituted oligoethylene oxid/oligopropyleneoxide (JEFFamine). JEFFamines ED600, ED900 and ED1900 (Scheme 7) were functionalized through reductive amination procedure described in Example 15.

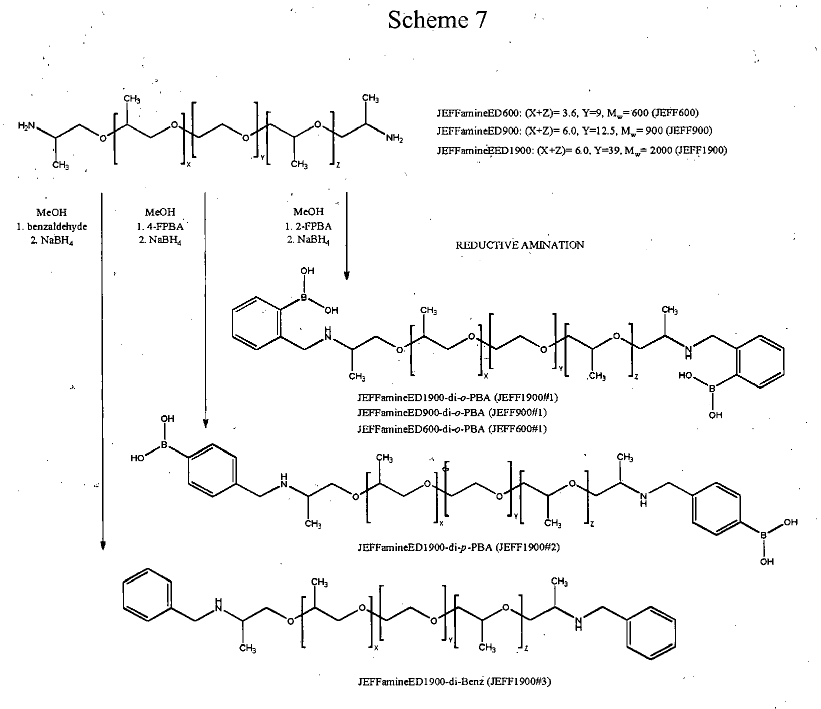

All linkers were synthesized via reductive amination of the corresponding aldehyde with the appropriate Jeffamine. The obtained variations in linker allows evaluation of spacer length and nature of the α,ω-boronic acid groups (ortho vs para-substituted with respect to the amine group), i.e. phenylboronic acid with intramolecular B-N interaction (JEFF1900#1) vs. phenylboronic acid without intramolecular B-N interaction (JEFF1900#2) vs. aromatic system without boronic acid functionality (JEFF1900#3).

### Example 23

This Example provides gel properties of PVA with α,ω-di(boronic acid) functionalized JEFFamineED1900. Bis(*o*-boronic acid) functionalized JEFFamineED1900 (20 % w/v JEFF1900#1, cf Example 21) was used to form gels with 195kDa PVA (5 % w/v) at pH 5 (cf. Example 16) and the swelling behavior was measured (cf. Example 21).

**Table 23^{a}**

| [Glucose] (% w/v) | Swelling ratio after 1 hour (%) | Swelling ratio after 4 hours (%) | Swelling ratio after 8 hours (%) |
|---|---|---|---|
| 0 | 1.30 (±2) | 1.18 (±1) | 82 (±11) |
| 1 | 1.22 (±2) | 1.11 (±2) | 57 (±6) |
| 2.5 | 1.21 (±3) | 1.20 (±2) | 70 (±18) |
| 5 | 1.12 (±1) | 1.11 (±7) | 69 (±15) |
| 10 | 0.94 (±9) | 0.91 (±17) | 38 (±1) |

| | | | |
|---|---|---|---|
| ^{a} 20 % (w/v) JEFF1900#1 and 5 % (w/v). PVA(195 kDa). | | | |

The data of Table 23 show that increasing the glucose concentration from 0 -10 % w/v will result in faster degradation of the gels. This is demonstrated by the swelling ratio after 8 hours, where the gels with 0-5 % w/v glucose are mostly intact and the gel with 10% w/v glucose is mostly degraded.

### Example 24

This Example shows that reductive amination of 8-armed PEG-amine (25 kDa, 8NH₂) with 4FPBA results in 25 kDa PEG-(4-aminomethyl phenyl boronic acid)₈

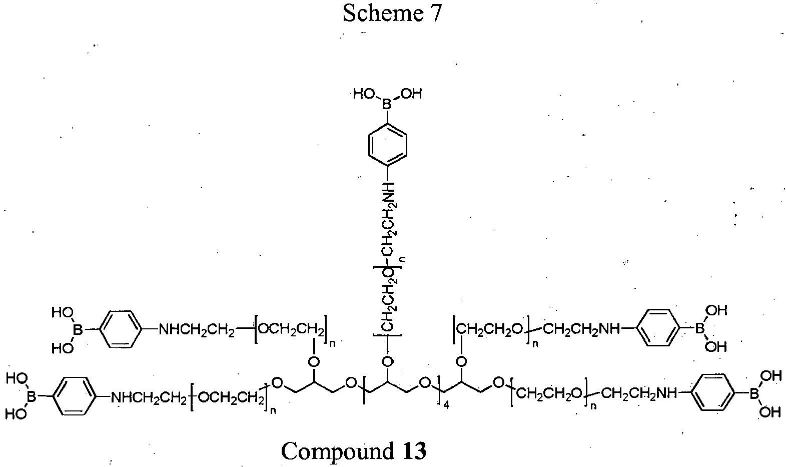

Starting material eight-arm PEG amine 25 kDa was synthesized from commercial eight-arm PEG hydroxyl 25 kDa (cf. D.L. Elbert, J.A. Hubbell, Conjugate addition reactions combined with free-radical cross-linking for the design of materials for tissue engineering, Biomacromolecules 2 (2001) 430-441). The completely functionalized eight-arm PEG amine (1,003 g; 0,04 mmol) was mixed with 4-formylphenylboronic acid (58 mg; 0.39 mmol) in a round bottom flask and dissolved in 5 ml methanol. The reaction was stirred at room temperature for four days under nitrogen-atmosphere. Subsequently, NaBH₄ (36 mg; 0.96 mmol) was added portion-wise to the yellow solution, followed by an observed colour-change from yellow to pale-yellow. This reduction was allowed to proceed to completion for two hours; indicated by the cease of hydrogen gas formation. The remaining methanol was then evaporated under reduced pressure, and the product mixture was dissolved in milli-Q water and purified by dialysis using 10000 MWCO ultrafiltration membrane. The purified retentate 25 kDa PEG-(4AMPBA)₈ was then freeze-dried and obtained as white spongy material. Yield: 85 %.

¹H-NMR (CD₃OD): 7,4 ppm (*d*, 16H, Ar), 7,1 ppm (*d*, 16H, Ar), 3, 9 ppm (s, 16H, Ar-*CH₂*-NH-), 3,05-3,8 ppm (*m*, 1830H, -O-*CH₂-CH₂-*O-), 2,96 ppm (*t*,16H, -NH-*CH₂*-CH₂-O-).

### Example 25

This Example shows that compound 13 can form rigid hydrogels with PVA under physiological conditions. Compound 13 (cf. Example 26) was dissolved and added to a 195 kD PVA solution of 10 % (w/v) to a final concentration of 10 % (w/y) crosslinker in PBS (10 mM, 75 mM NaCl, pH7.4). A very strong gel was observed both at 25°C and 37 °C (G'max >13,800 and >11,100 Pa respectively), with self healing properties (relaxation time of 1.1 s at 25 °C and 0.6 s at 37 °C). Thermoreversible behaviour was demonstrated, by repeatedly keeping the gel for 1 hr at 25 and 37 °C (cf. Table 24).

**Table 24**

| Time (hr) | 1 | 2 | 3 | 4 | 5 | 6 | 7 | 8 | 9 | 10 | 11 | 12 |
|---|---|---|---|---|---|---|---|---|---|---|---|---|
| Temp (°C) | 25 | 37 | 25 | 37 | 25 | 37 | 25 | 37 | 25 | 37 | 25 | 37 |
| G'max (kPa) | 13.8 | 11.1 | 14.1 | 11.3 | 14.2 | 1.1 | 14.3 | 11.5 | 14.3 | 11.5 | 14.4 | 11.5 |
| G"max (kPa) | 1.37 | 1.83 | 1.39 | 1.86 | 1.41 | 1.87 | 1.41 | 1.88 | 1.41 | 1.88 | 1.41 | 1.89 |

### Example 26

This example shows the differences in gel properties as function of the pH of PVA gels with the *ortho-* and *para*-aminomethyl phenylboronic acid Jeffamines, Jeff1900#1 and Jeff1900#2, respectively, allowing modulation of (mixtures of) the gels as function of the pH.

Two frequency-dependent moduli as measured by oscillatory rheology within the linear viscoelastic region were determined to characterize the viscoelasticity of the dynamic network of these gels: the elastic modulus G' (storage modulus) and the viscous modulus G" (loss modulus). The hydrogels exhibit viscous behaviour at a long time scale (at low angular frequency), at which the network of hydrogel has sufficient time to reorganize and can flow accordingly (G' < G"). In contrast, these gels exhibit elastic behaviour on a short time scale (at high angular frequency), at which the crosslinks of the network can not completely dissociate and the network is more rigid (G' > G"). Moreover, at higher frequencies, the elastic modulus G' becomes frequency-independent and reaches a plateau. In these Jeffamine-PVA gels the pH dramatically affects the viscoelastic behavior, since the equilibrium of boronic/boronate ester formation and the resulting crosslink density of the network is strongly pH dependant. Two main parameters, as a function of angular frequency, quantify the rheological behaviour of viscoelastic hydrogels: the relaxation time (τ) reflecting the lifetime of the crosslink and the plateau value of the storage modulus G' obtained at high angular frequencies (G'ₘₐₓ), reflecting the maximal strength attainable. The relaxation time τ was determined by τ = 2π/ω_{c}), where ω_{c} is the crossover angular frequency at which G' equals to G", and τ could be viewed as the average lifetime of the crosslinks. Gels with longer relaxation times show more elastic behaviour and have a higher shape-stability.

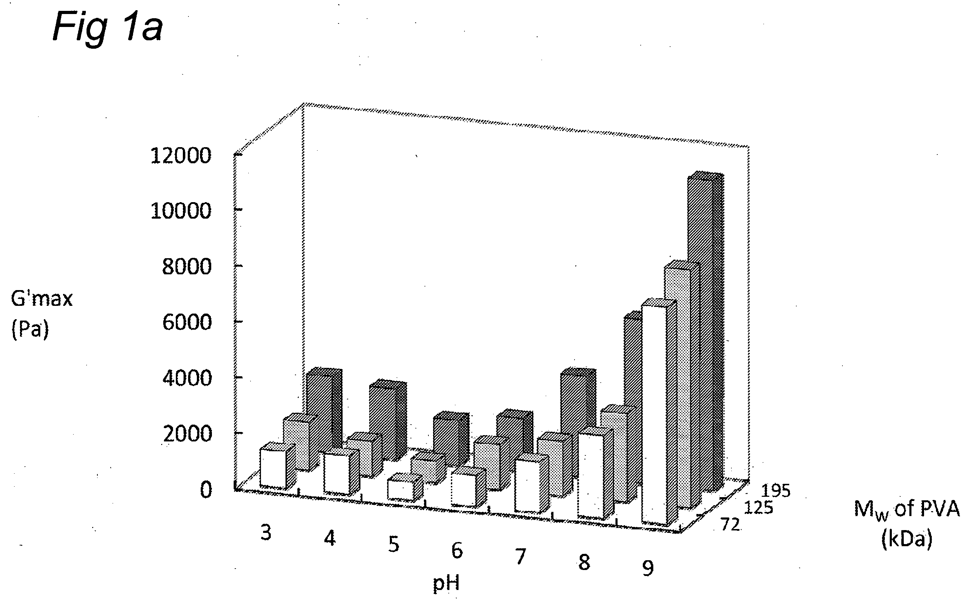

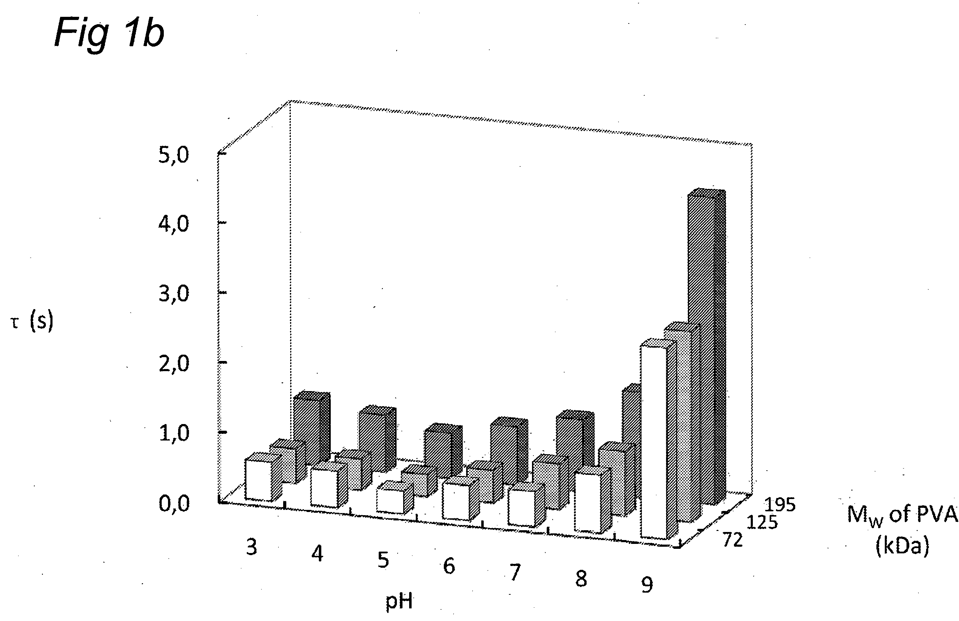

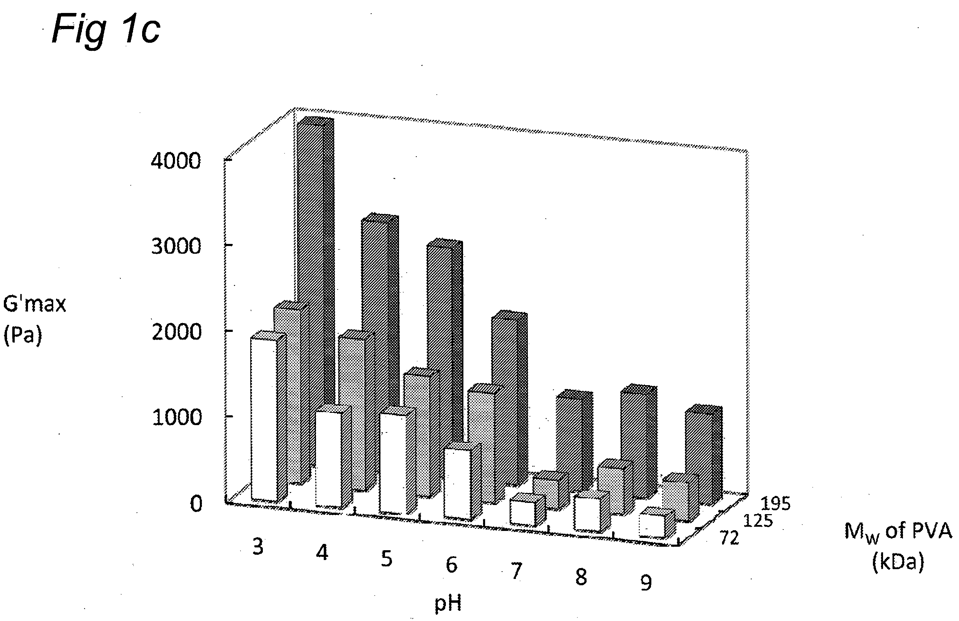

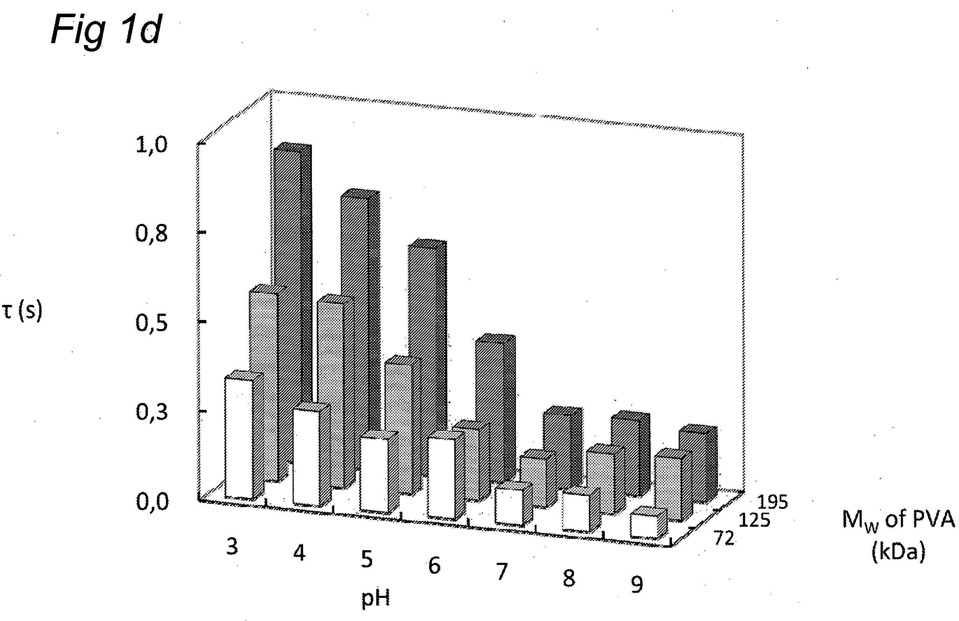

It was observed that gel formation occurs immediately upon mixing of the diphenylboronic acid functionalized Jeffamine crosslinkers with PVA and the resulting hydrogels were transparent. The mixing of Jeff1900#1 and Jeff1900#2 crosslinkers with PVA yielded strong hydrogels, depending on the pH and molecular weight of the PVA used, see Figure 1 which shows plateau value of storage modulus G'ₘₐₓ (a, c) and relaxation time τ (b, d) for hydrogels prepared by mixing of 10% w/v Jeff1900#2 (a, b) and leff1900#1 (c, d) crosslinkers and 10% w/v PVA with different M_{w} (72, 125, and 195 kDa) at pH ranging from 3 - 9 at 25 °C.

From the G'ₘₐₓ values in Figure 1, it is clear that under all conditions stronger gels are formed with higher molecular weight PVA, which can be explained by an increase in polymer entanglement, thereby enhancing the gel strength. With increasing M_{w} of PVA an increase in viscosity was observed that is slowing the relaxation process and thus explains the longer relaxation times observed in Figure 1.

Remarkably different behavior was observed for the pH dependency of the two types of Jeffamine-based hydrogels. For Jeff1900#2 the strongest gels were formed in basic media (Figure 1, a and b), whereas the Jeff1900#1 hydrogels showed increasing gel strength in acidic media (Figure 1, c and d). For example, for Jeff1900#2 hydrogels with PVA 195 kDa, the gel strength G'ₘₐₓ increases from 3000 (± 230) Pa at pH 3 to 11100(± 180) Pa at pH 9, with a concomitant increase of the crosslinking lifetime τ from 0.93 (± 0.06) s to 4.38 (± 0.07) s. A minimum in gel strength was observed at pH 5, where G' plateau was only 1600 ± 20 Pa and τ was 0.65 ± 0.04 s. For the hydrogels prepared with Jeff1900#1, the strongest gels were formed at acidic pH. For example, mixing PVA 195 kDa with leff1900#1 at pH 3 immediately gave a strong hydrogel with G'ₘₐₓ = 4000 (± 130) Pa, and τ = 0.87 (± 0.02) s, which is stronger than the gel observed for Jeff1900#2 under the same conditions.

## Claims

1. A boronated polymer according to Formula (1): 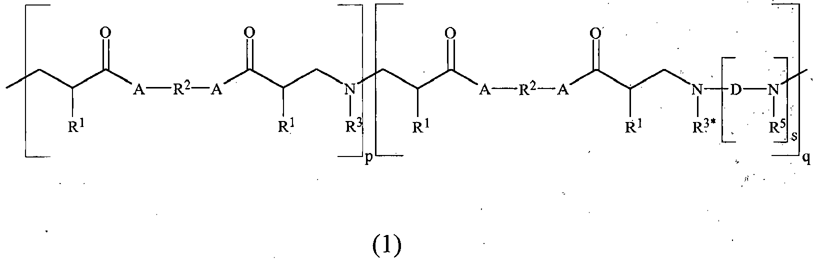 wherein:
A is independently selected from a direct carbon-carbon single bond, O, N and S;
R¹ is independently selected from H and CH₃;
R² is independently selected from the group consisting of:
(a) C₁ - C₂₀ alkylene, wherein the alkylene group is optionally substituted and/or is optionally (partly) unsaturated and/or is optionally interrupted by one or more heteroatoms, wherein the heteroatoms are independently selected from O, N and S, and/or wherein the alkylene group is interrupted by one or more -S-S- groups;
(b) C₃ - C₂₀ cycloalkylene, wherein the cycloalkylene group is optionally substituted and/or is optionally (partly) unsaturated and/or optionally comprises one or more heteroatoms in the ring, wherein the heteroatoms are independently selected from O, N and S, and/or wherein the cycloalkylene group is interrupted by one or more -S-S- groups outside the ring;
(c) C₆ - C₂₀ arylene, wherein the arylene group is optionally substituted;
(d) C₆ - C₂₀ heteroarylene, wherein the heteroarylene group comprises 1 - 3 heteroatoms independently selected from O, N and S and/or wherein the heteroarylene group is optionally substituted;
(e) C₇ - C₂₀ alkylarylene wherein the alkylarylene group is optionally substituted and/or wherein an alkyl part of the alkylarylene group is optionally (partly) unsaturated and/or is optionally interrupted by one or more heteroatoms, wherein the heteroatoms are independently selected from O, N and S, and/or wherein an alkyl part of the alkylarylene group is interrupted by one or more -S-S- groups;
(f) C₇ - C₂₀ alkylheteroarylene, wherein the alkylheteroarylene group comprises 1 - 3 heteroatoms independently selected from O, N and S and/or wherein the alkylheteroarylene group is optionally substituted, and/or wherein an alkyl part of the alkylheteroarylene group is optionally (partly) unsaturated and/or is optionally interrupted by one or more heteroatoms, wherein the heteroatoms are independently selected from O, N and S, and/or wherein an alkyl part of the alkylheteroarylene group is interrupted by one or more -S-S- groups; and
(g) a group wherein two (hetero)arylene groups and/or alkyl(hetero)arylene groups are connected to each other by a -S-S- group;
D is selected from the group consisting of -(CR⁴2)r- and the groups (a), (b), (c), (d), (e), (f) and (g) defined for R²;
R⁴ is independently selected from the group consisting of:
(a) H;
(b) C₁ - C₂₀ alkyl, wherein the alkyl group is optionally substituted and/or is optionally (partly) unsaturated and/or is optionally interrupted by one or more heteroatoms, wherein the heteroatoms are independently selected from O, N and S;
(c) C₃ - C₂₀ cycloalkyl, wherein the cycloalkyl group is optionally substituted and/or is optionally (partly) unsaturated and/or optionally comprises one or more heteroatoms in the ring, wherein the heteroatoms are independently selected from O, N and S;
(d) C₆ - C₂₀ aryl, wherein the aryl group is optionally substituted;
(e) C₆ - C₂₀ heteroaryl, wherein the heteroaryl group comprises 1 - 3 heteroatoms independently selected from O, N and S and/or wherein the heteroaryl group is optionally substituted;
(f) C₇ - C₂₀ alkylaryl wherein the alkylaryl group is optionally substituted and/or wherein an alkyl part of the alkylaryl group is optionally (partly) unsaturated and/or is optionally interrupted by one or more heteroatoms, wherein the heteroatoms are independently selected from O, N and S; and
(g) C₇ - C₂₀ alkylheteroaryl, wherein the alkylheteroaryl group comprises 1 - 3 heteroatoms independently selected from O, N and S and/or wherein the alkylheteroaryl group is optionally substituted, and/or wherein an alkyl part of the alkylheteroaryl group is optionally .(partly) unsaturated and/or is optionally interrupted by one or more heteroatoms, wherein the heteroatoms are independently selected from O, N and S;
R⁵ is independently selected from the group consisting of H and C₁ - C₂₀ alkyl, wherein the alkyl group is optionally substituted and/or is optionally (partly) unsaturated and/or is optionally interrupted by one or more heteroatoms, wherein the heteroatoms are independently selected from O, N and S;
p = 1 to 100;
q = 0 to 100;
r = 2 - 6;
s = 0 - 5;
R³ has the Formula (2): 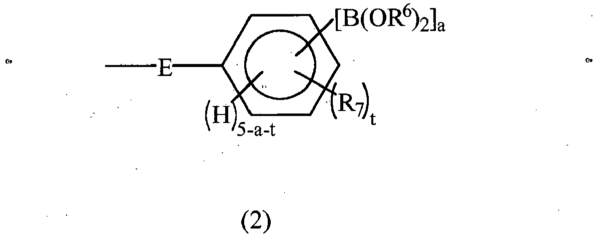
wherein:
R⁶ is independently selected from
(a) H;
(b) C₁ - C₁₂ alkyl, wherein the alkyl group is optionally substituted and/or is optionally (partly) unsaturated and/or is optionally interrupted by one or more heteroatoms, wherein the heteroatoms are independently selected from O, N and S ;
(c) C₃ - C₁₋₂ cycloalkyl, wherein the cycloalkyl group is optionally substituted and/or is optionally (partly) unsaturated and/or is optionally interrupted by one or more heteroatoms, wherein the heteroatoms are independently selected from O, N and S;
(d) C₆ - C₂₀ aryl, wherein the aryl group is optionally substituted;
(e) C₆ - C₂₀ heteroaryl, wherein the heteroaryl group comprises 1 - 3 heteroatoms independently selected from O, N and S and/or wherein the heteroaryl group is optionally substituted;
(f) C₇ - C₂₀ alkylaryl wherein the alkylaryl group is optionally substituted and/or wherein an alkyl part of the alkylaryl group is optionally (partly) unsaturated and/or is optionally interrupted by one or more heteroatoms, wherein the heteroatoms are independently selected from O, N and S; and
(g) C₇ - C₂₀ alkylheteroaryl, wherein the alkylheteroaryl group comprises 1 - 3 heteroatoms independently selected from O, N and S and/or wherein the alkylheteroaryl group is optionally substituted, and/or wherein an alkyl part of the alkylheteroaryl group is optionally (partly) unsaturated and/or is optionally interrupted by one or more heteroatoms, wherein the heteroatoms are independently selected from O, N and S;
R⁷ is independently selected from
(a) halogen;
(b) C₁ - C₁₂ alkyl, wherein the alkyl group is optionally substituted and/or is optionally (partly) unsaturated and/or is optionally interrupted by one or more heteroatoms, wherein the heteroatoms are independently selected from O, N and S;
(c) C₃ - C₁₂ cycloalkyl, wherein the cycloalkyl group is optionally substituted and/or is optionally (partly) unsaturated and/or is optionally interrupted by one or more heteroatoms, wherein the heteroatoms are independently selected from O, N and S;
(d) C₆ - C₂₀ aryl, wherein the aryl group is optionally substituted;
(e) C₆ - C₂₀ heteroaryl, wherein the heteroaryl group comprises 1 - 3 heteroatoms independently selected from O, N and S and/or wherein the heteroaryl group is optionally substituted;
(f) C₇ - C₂₀ alkylaryl wherein the alkylaryl group is optionally substituted and/or wherein an alkyl part of the alkylaryl group is optionally (partly) unsaturated and/or is optionally interrupted by one or more heteroatoms, wherein the heteroatoms are independently selected from O, N and S; and
(g) C₇ - C₂₀ alkylheteroaryl, wherein the alkylheteroaryl group comprises 1 - 3 heteroatoms independently selected from O, N and S and/or wherein the alkylheteroaryl group is optionally substituted, and/or wherein an alkyl part of the alkylheteroaryl group is optionally (partly) unsaturated and/or is optionally interrupted by one or more heteroatoms, wherein the heteroatoms are independently selected from O, N and S;
E is independently selected from the group consisting of:
-(CR⁸₂)ᵤ-N(R⁹)-C(O)-,
-C(O)-N(R⁹)-(CR⁸2)ᵤ-,
-(CR⁸₂)ᵤ-N=CR⁹-,
-CR⁹=N-(CR⁸₂)ᵤ-, and
-(CR⁸₂)ᵤ-N(R⁹)-(CR⁸₂)ᵥ-;
t = 0 - 4;
u = 1 - 10;
v = 1 - 4;
R⁸ is independently selected from the group consisting of H and C₁ - C₂₀ alkyl;
R⁹ is independently selected from the group consisting of H and C₁ - C₂₀ alkyl; and
when s = 0, then R^{3*} is independently selected from the group consisting of
(a) H;
(b) C₁ - C₁₂ alkyl, wherein the alkyl group is optionally substituted and/or is optionally (partly) unsaturated and/or is optionally interrupted by one or more heteroatoms, wherein the heteroatoms are independently selected from O, N and S;
(c) C₃ - C₁₂ cycloalkyl, wherein the cycloalkyl group is optionally substituted and/or is optionally (partly) unsaturated and/or is optionally interrupted by one or more heteroatoms, wherein the heteroatoms are independently selected from O, N and S;
(d) C₆ - C₂₀ aryl, wherein the aryl group is optionally substituted;
(e) C₆ - C₂₀ heteroaryl, wherein the heteroaryl group comprises-1 - 3 heteroatoms independently selected from O, N and S and/or wherein the heteroaryl group is optionally substituted;
(f) C₇ - C₂₀ alkylaryl wherein the alkylaryl group is optionally substituted and/or wherein an alkyl part of the alkylaryl group is optionally (partly) unsaturated and/or is optionally interrupted by one or more heteroatoms, wherein the heteroatoms are independently selected from O, N and S; and
(g) C₇ - C₂₀ alkylheteroaryl, wherein the alkylheteroaryl group comprises 1 - 3 heteroatoms independently selected from O, N and S and/or wherein the alkylheteroaryl group is optionally substituted, and/or wherein.an alkyl part of the alkylheteroaryl group is optionally (partly) unsaturated and/or is optionally interrupted by one or more heteroatoms, wherein the heteroatoms are independently selected from O, N and S;
when s = 1 - 5, then R^{3*} is independently selected from the group consisting of
(a) H;
(b) C₁ - C₆ alkyl, wherein the alkyl group is optionally substituted;
(c) C₃ - C₆ cycloalkyl, wherein the cycloalkyl group is optionally substituted; C₆ - C₁₂ aryl, wherein the aryl group is optionally substituted;
(d) C₆ - C₁₂ heteroaryl, wherein the heteroaryl group comprises 1 - 3 heteroatoms independently selected from O, N and S and/or wherein the heteroaryl group is optionally substituted;
(e) C₇ - C₁₂ alkylaryl wherein the alkylaryl group is optionally substituted; and
(f) C₇ - C₁₂ alkylheteroaryl, wherein the alkylheteroaryl group comprises 1 - 3 heteroatoms independently selected from O, N and S and/or wherein the alkylheteroaryl group is optionally substituted.

2. The boronated polymer according to Formula (1), wherein the boronated polymer has a number average molecular weight Mₙ in the range of about 1,000 to about 100,000.

3. The boronated polymer according to Claim 1 or Claim 2, wherein s = 0.

4. The boronated polymer according to any one of Claims 1 - 3, wherein q = 0.

5. The boronated polymer according to any one of Claims 1 - 4, wherein v is 1 or 2.

6. The boronated polymer according to any one of Claims 1 - 5, wherein a is 1.

7. The boronated polymer according to any one of Claims 1 - 6, wherein t is 0 - 2.

8. A process for preparing a boronated polymer according to any one of Claims 1 - 7, said process comprising polymerizing a monomer according to Formula (6): 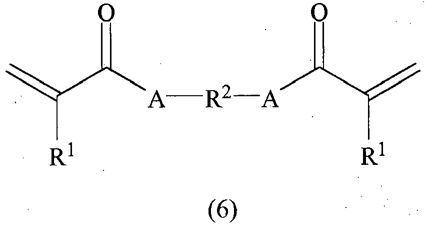 with a monomer according to Formula (7):
H₂N-R³ (7)
and:
optionally in the presence of a monomer according to Formula (8):
H₂N-R^{3*} (8)
or optionally in the presence of a monomer according to Formula (9): 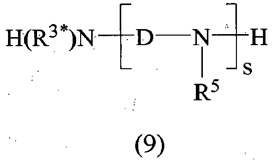
wherein R¹, R², A, R³, R^{3*}, R⁴, R⁵, r and s have the meaning as defined in Claim 1.

9. A process for preparing a boronated polymer according to any one of Claims 1 - 7, said process comprising:
(a) polymerizing a monomer according to Formula (6): 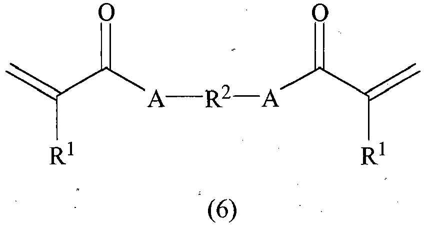 with a monomer according to Formula (10):
^{t}BuO-C(O)-N(H)-(CR⁸₂)_{w}-NH₂ (10)
wherein w = 2 - 20 and R⁸ is independently selected from the group consisting of H and C₁ - C₂₀ alkyl, to obtain a polymer according to Formula (11): 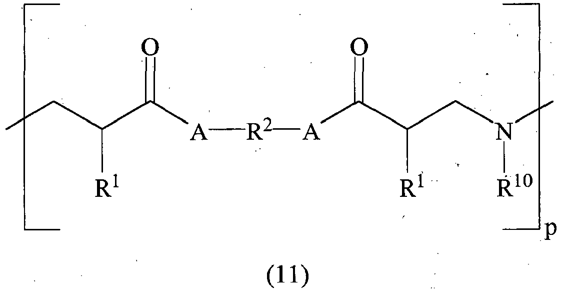 wherein R¹⁰ is ^{t}BuO-C(O)-N(H)-(CR⁸₂)-NH-;
(b) reacting the polymer according to Formula (11) with an acid to obtain a formula according to Formula (11) wherein R¹⁰ is H₂N-(CR⁸₂)-NH-; and
(c) reacting the polymer as obtained in step (b) with a compound according to Formula (12): 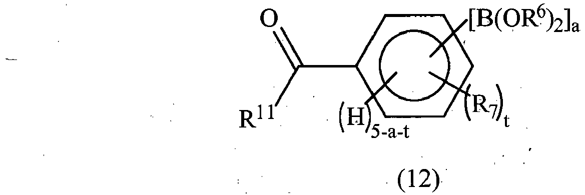 wherein:
R⁶ is independently selected from
(a) H;
(b) C₁ - C₁₂ alkyl, wherein the alkyl group is optionally substituted and/or is optionally (partly) unsaturated and/or is optionally interrupted by one or more heteroatoms, wherein the heteroatoms are independently selected from O, N and S;
(c) C₃ - C₁₂ cycloalkyl, wherein the cycloalkyl group is optionally substituted and/or is optionally (partly) unsaturated and/or is optionally interrupted by one or more heteroatoms, wherein the heteroatoms are independently selected from O, N and S;
(d) C₆ - C₂₀ aryl, wherein the aryl group is optionally substituted;
(e) C₆ - C₂₀ heteroaryl, wherein the heteroaryl group comprises 1 - 3 heteroatoms independently selected from O, N and S and/or wherein the heteroaryl group is optionally substituted;
(f) C₇ = C₂₀ alkylaryl wherein the alkylaryl group is optionally substituted and/or wherein an alkyl part of the alkylaryl group is optionally (partly) unsaturated and/or is optionally interrupted by one or more heteroatoms, wherein the heteroatoms are independently selected from O, N and S; and
(g) C₇ - C₂₀ alkylheteroaryl, wherein the alkylheteroaryl group comprises 1 - 3 heteroatoms independently selected from O, N and S and/or wherein the alkylheteroaryl group is optionally substituted, and/or wherein an alkyl part of the alkylheteroaryl group is optionally (partly) unsaturated and/or is optionally interrupted by one or more heteroatoms, wherein the heteroatoms are independently selected from O, N and S;
R⁷ is independently selected from
(a) halogen;
(b) C₁ - C₁₂ alkyl, wherein the alkyl group is optionally substituted and/or is optionally (partly) unsaturated and/or is optionally interrupted by one or more heteroatoms, wherein the heteroatoms are independently selected from O, N and S;
(c) C₃ - C₁₂ cycloalkyl, wherein the cycloalkyl group is optionally substituted and/or is optionally (partly) unsaturated and/or is optionally interrupted by one or more heteroatoms, wherein the heteroatoms are independently selected from O, N and S;
(d) C₆ - C₂₀ aryl, wherein the aryl group is optionally substituted;
(e) C₆ - C₂₀ heteroaryl, wherein the heteroaryl group comprises 1 - 3 heteroatoms independently selected from O, N and S and/or wherein the heteroarylene group is optionally substituted;
(f) C₇ - C₂₀ alkylaryl wherein the alkylaryl group is optionally substituted and/or wherein an alkyl part of the alkylaryl group is optionally (partly) unsaturated and/or is optionally interrupted by one or more heteroatoms, wherein the heteroatoms are independently selected from O, N and S; and
(g) C₇ - C₂₀ alkylheteroaryl, wherein the alkylheteroaryl group comprises 1 - 3 heteroatoms independently selected from O, N and S and/or wherein the alkylheteroaryl group is optionally substituted, and/or wherein an alkyl part of the alkylheteroaryl group is optionally (partly) unsaturated and/or is optionally interrupted by one or more heteroatoms, wherein the heteroatoms are independently selected from O, N and S;
R¹¹ is H or a leaving group;
t = 0 - 4;
and wherein R¹, R² and A are as defined in Claim 1.

10. The process according to Claim 9, wherein any pendant amino groups are subjected to acylation.

11. A boronated polymer obtainable by the process according to Claim 8 or obtainable by the process according to Claim 9 or Claim 10.

12. A drug delivery system comprising the boronated polymer according to any one of Claims 1 - 7 or Claim 11.

13. An aggregate or a nanoparticle comprising the boronated polymer according to any one of Claims 1 - 7 or Claim 11.

14. The aggregate or the nanoparticle according to Claim 13, wherein the nanoparticle comprises a biologically active component selected from the group of drugs, anionic polymers, DNA molecules or derivatives thereof, RNA molecules or derivatives thereof, peptides and derivatives thereof, and proteins and derivatives thereof.

15. The nanoparticle according to Claim 13 or Claim 14, wherein R² is independently selected from the group consisting of:
(a) C₁ - C₂₀ alkylene, wherein the alkylene group is optionally substituted and is interrupted by one or more -S-S- groups; and
(b) C₃ - C₂₀ cycloalkylene, wherein the cycloalkylene group is optionally substituted and is interrupted by one or more -S-S- groups outside the ring.

16. A hydrogel comprising the boronated polymer according to any one of Claims 1 - 7 or Claim 11.

17. The hydrogel according to Claim 16, further comprising a polyvinyl alcohol.

18. The hydrogel according to Claim 16 or Claim 17, further comprising a carbohydrate.

19. The hydrogel according to any one of Claims 16 - 18, further comprising a crosslinker.

20. The hydrogel according to any one of Claims 16 - 19, wherein R² is independently selected from the group consisting of:
(a) C₁- C₂₀ alkylene, wherein the alkylene group is optionally substituted and is interrupted by one or more -S-S- groups; and
(b) C₃ - C₂₀ cycloalkylene, wherein the cycloalkylene group is optionally substituted and is interrupted by one or more -S-S- groups outside the ring.

## Patentansprüche

1. Boriertes Polymer gemäß Formel (1): 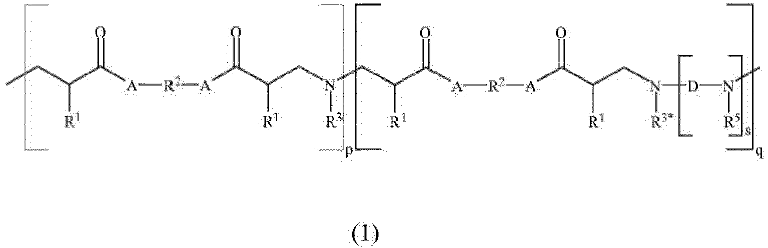 wobei:
A unabhängig ausgewählt ist aus einer direkten Kohlenstoff-Kohlenstoff-Einfachbindung, O, N und S;
R¹ unabhängig ausgewählt ist aus H und CH₃;
R² unabhängig ausgewählt ist aus der Gruppe bestehend aus:
(a) C₁-C₂₀-Alkylen, wobei die Alkylengruppe gegebenenfalls substituiert ist und/oder gegebenenfalls (teilweise) ungesättigt ist und/oder gegebenenfalls durch ein oder mehrere Heteroatome unterbrochen ist, wobei die Heteroatome unabhängig ausgewählt sind aus O, N und S, und/oder wobei die Alkylengruppe durch eine oder mehrere -S-S- Gruppen unterbrochen ist;
(b) C₃-C₂₀-Cycloalkylen, wobei die Cycloalkylengruppe gegebenenfalls substituiert ist und/oder gegebenenfalls (teilweise) ungesättigt ist und/oder gegebenenfalls ein oder mehrere Heteroatome in dem Ring umfasst, wobei die Heteroatome unabhängig ausgewählt sind aus O, N und S, und/oder wobei die Cycloalkylengruppe durch eine oder mehrere -S-S- Gruppen außerhalb des Rings unterbrochen ist;
(c) C₆-C₂₀-Arylen, wobei die Arylengruppe gegebenenfalls substituiert ist;
(d) C₆-C₂₀-Heteroarylen, wobei die Heteroarylengruppe 1 - 3 Heteroatome, unabhängig ausgewählt aus O, N und S, umfasst und/oder wobei die Heteroarylengruppe gegebenenfalls substituiert ist;
(e) C₇-C₂₀-Alkylarylen, wobei die Alkylarylengruppe gegebenenfalls substituiert ist und/oder wobei ein Alkylteil der Alkylarylengruppe gegebenenfalls (teilweise) ungesättigt ist und/oder gegebenenfalls durch ein oder mehrere Heteroatome unterbrochen ist, wobei die Heteroatome unabhängig ausgewählt sind aus O, N und S, und/oder wobei ein Alkylteil der Alkylarylengruppe durch eine oder mehrere -S-S-Gruppen unterbrochen ist;
(f) C₇-C₂₀-Alkylheteroarylen, wobei die Alkylheteroarylengruppe 1 - 3 Heteroatome, unabhängig ausgewählt aus O, N und S, umfasst und/oder wobei die Alkylheteroarylengruppe gegebenenfalls substituiert ist, und/oder wobei ein Alkylteil der Alkylheteroarylengruppe gegebenenfalls (teilweise) ungesättigt ist und/oder gegebenenfalls durch ein oder mehrere Heteroatome unterbrochen ist, wobei die Heteroatome unabhängig ausgewählt sind aus O, N und S, und/oder wobei ein Alkylteil der Alkylheteroarylengruppe durch eine oder mehrere -S-S- Gruppen unterbrochen ist; und
(g) eine Gruppe, wobei zwei (Hetero)arylengruppen und/oder Alkyl(hetero)arylengruppen durch eine -S-S- Gruppe miteinander verbunden sind;
D ausgewählt ist aus der Gruppe bestehend aus -(CR⁴₂)r- und den Gruppen (a), (b), (c), (d), (e), (f) und (g), die für R² definiert sind;
R⁴ unabhängig ausgewählt ist aus der Gruppe bestehend aus:
(a) H;
(b) C₁-C₂₀-Alkyl, wobei die Alkylgruppe gegebenenfalls substituiert ist und/oder gegebenenfalls (teilweise) ungesättigt ist und/oder gegebenenfalls durch ein oder mehrere Heteroatome unterbrochen ist, wobei die Heteroatome unabhängig ausgewählt sind aus O, N und S;
(c) C₃-C₂₀-Cycloalkyl, wobei die Cycloalkylgruppe gegebenenfalls substituiert ist und/oder gegebenenfalls (teilweise) ungesättigt ist und/oder gegebenenfalls ein oder mehrere Heteroatome in dem Ring umfasst, wobei die Heteroatome unabhängig ausgewählt sind aus O, N und S;
(d) C₆-C₂₀-Aryl, wobei die Arylgruppe gegebenenfalls substituiert ist;
(e) C₆-C₂₀-Heteroaryl, wobei die Heteroarylgruppe 1 - 3 Heteroatome, unabhängig ausgewählt aus O, N und S, umfasst und/oder wobei die Heteroarylgruppe gegebenenfalls substituiert ist;
(f) C₇-C₂₀-Alkylaryl, wobei die Alkylarylgruppe gegebenenfalls substituiert ist und/oder wobei ein Alkylteil der Alkylarylgruppe gegebenenfalls (teilweise) ungesättigt ist und/oder gegebenenfalls durch ein oder mehrere Heteroatome unterbrochen ist, wobei die Heteroatome unabhängig ausgewählt sind aus O, N und S; und
(g) C₇-C₂₀-Alkylheteroaryl, wobei die Alkylheteroarylgruppe 1 - 3 Heteroatome, unabhängig ausgewählt aus O, N und S, umfasst und/oder wobei die Alkylheteroarylgruppe gegebenenfalls substituiert ist, und/oder wobei ein Alkylteil der Alkylheteroarylgruppe gegebenenfalls (teilweise) ungesättigt ist und/oder gegebenenfalls durch ein oder mehrere Heteroatome unterbrochen ist, wobei die Heteroatome unabhängig ausgewählt sind aus O, N und S;
R⁵ unabhängig ausgewählt ist aus der Gruppe bestehend aus H und C₁-C₂₀-Alkyl, wobei die Alkylgruppe gegebenenfalls substituiert ist und/oder gegebenenfalls (teilweise) ungesättigt ist und/oder gegebenenfalls durch ein oder mehrere Heteroatome unterbrochen ist, wobei die Heteroatome unabhängig ausgewählt sind aus O, N und S;
p = 1 bis 100;
q = 0 bis 100;
r=2-6;
s=0-5;
R³ hat die Formel (2): 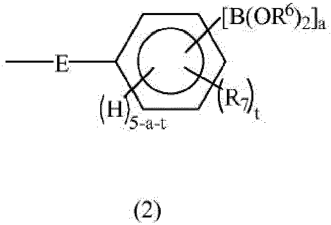
wobei:
a 1 oder 2 ist;
R⁶ unabhängig ausgewählt ist aus:
(a) H;
(b) C₁-C₁₂-Alkyl, wobei die Alkylgruppe gegebenenfalls substituiert ist und/oder gegebenenfalls (teilweise) ungesättigt ist und/oder gegebenenfalls durch ein oder mehrere Heteroatome unterbrochen ist, wobei die Heteroatome unabhängig ausgewählt sind aus O, N und S;
(c) C₃-C₁₂-Cycloalkyl, wobei die Cycloalkylgruppe gegebenenfalls substituiert ist und/oder gegebenenfalls (teilweise) ungesättigt ist und/oder gegebenenfalls durch ein oder mehrere Heteroatome unterbrochen ist, wobei die Heteroatome unabhängig ausgewählt sind aus O, N und S;
(d) C₆-C₂₀-Aryl, wobei die Arylgruppe gegebenenfalls substituiert ist;
(e) C₆-C₂₀-Heteroaryl, wobei die Heteroarylgruppe 1 - 3 Heteroatome, unabhängig ausgewählt aus O, N und S, umfasst und/oder wobei die Heteroarylgruppe gegebenenfalls substituiert ist;
(f) C₇-C₂₀-Alkylaryl, wobei die Alkylarylgruppe gegebenenfalls substituiert ist und/oder wobei ein Alkylteil der Alkylarylgruppe gegebenenfalls (teilweise) ungesättigt ist und/oder gegebenenfalls durch ein oder mehrere Heteroatome unterbrochen ist, wobei die Heteroatome unabhängig ausgewählt sind aus O, N und S; und
(g) C₇-C₂₀-Alkylheteroaryl, wobei die Alkylheteroarylgruppe 1 - 3 Heteroatome, unabhängig ausgewählt aus O, N und S, umfasst und/oder wobei die Alkylheteroarylgruppe gegebenenfalls substituiert ist, und/oder wobei ein Alkylteil der Alkylheteroarylgruppe gegebenenfalls (teilweise) ungesättigt ist und/oder gegebenenfalls durch ein oder mehrere Heteroatome unterbrochen ist, wobei die Heteroatome unabhängig ausgewählt sind aus O, N und S;
R⁷ unabhängig ausgewählt ist aus
(a) Halogen;
(b) C₁-C₁₂-Alkyl, wobei die Alkylgruppe gegebenenfalls substituiert ist und/oder gegebenenfalls (teilweise) ungesättigt ist und/oder gegebenenfalls durch ein oder mehrere Heteroatome unterbrochen ist, wobei die Heteroatome unabhängig ausgewählt sind aus O, N und S;
(c) C₃-C₁₂-Cycloalkyl, wobei die Cycloalkylgruppe gegebenenfalls substituiert ist und/oder gegebenenfalls (teilweise) ungesättigt ist und/oder gegebenenfalls durch ein oder mehrere Heteroatome unterbrochen ist, wobei die Heteroatome unabhängig ausgewählt sind aus O, N und S;
(d) C₆-C₂₀-Aryl, wobei die Arylgruppe gegebenenfalls substituiert ist;
(e) C₆-C₂₀-Heteroaryl, wobei die Heteroarylgruppe 1 - 3 Heteroatome, unabhängig ausgewählt aus O, N und S, umfasst und/oder wobei die Heteroarylgruppe gegebenenfalls substituiert ist;
(f) C₇-C₂₀-Alkylaryl, wobei die Alkylarylgruppe gegebenenfalls substituiert ist und/oder wobei ein Alkylteil der Alkylarylgruppe gegebenenfalls (teilweise) ungesättigt ist und/oder gegebenenfalls durch ein oder mehrere Heteroatome unterbrochen ist, wobei die Heteroatome unabhängig ausgewählt sind aus O, N und S; und
(g) C₇-C₂₀-Alkylheteroaryl, wobei die Alkylheteroarylgruppe 1 - 3 Heteroatome, unabhängig ausgewählt aus O, N und S, umfasst und/oder wobei die Alkylheteroarylgruppe gegebenenfalls substituiert ist, und/oder wobei ein Alkylteil der Alkylheteroarylgruppe gegebenenfalls (teilweise) ungesättigt ist und/oder gegebenenfalls durch ein oder mehrere Heteroatome unterbrochen ist, wobei die Heteroatome unabhängig ausgewählt sind aus O, N und S;
E unabhängig ausgewählt ist aus der Gruppe bestehend aus:
-(CR⁸₂)ᵤ-N(R⁹)-C(O)-,
-C(O)-N(R⁹)-(CR⁸₂)ᵤ-,
-(CR⁸₂)ᵤ-N=CR⁹-,
-CR⁹=N-(CR⁸₂)ᵤ-, und
-(CR⁸₂)ᵤ-N(R⁹)-(CR⁸₂)ᵥ-;
t=0-4;
u=1-10;
v=1-4;
R⁸ unabhängig ausgewählt ist aus der Gruppe bestehend aus H und C₁-C₂₀-Alkyl;
R⁹ unabhängig ausgewählt ist aus der Gruppe bestehend aus H und C₁-C₂₀-Alkyl; und
wenn s = 0, dann ist R^{3*} unabhängig ausgewählt aus der Gruppe bestehend aus:
(a) H,
(b) C₁-C₁₂-Alkyl, wobei die Alkylgruppe gegebenenfalls substituiert ist und/oder gegebenenfalls (teilweise) ungesättigt ist und/oder gegebenenfalls durch ein oder mehrere Heteroatome unterbrochen ist, wobei die Heteroatome unabhängig ausgewählt sind aus O, N und S;
(c) C₃-C₁₂-Cycloalkyl, wobei die Cycloalkylgruppe gegebenenfalls substituiert ist und/oder gegebenenfalls (teilweise) ungesättigt ist und/oder gegebenenfalls durch ein oder mehrere Heteroatome unterbrochen ist, wobei die Heteroatome unabhängig ausgewählt sind aus O, N und S;
(d) C₆-C₂₀-Aryl, wobei die Arylgruppe gegebenenfalls substituiert ist;
(e) C₆-C₂₀-Heteroaryl, wobei die Heteroarylgruppe 1 - 3 Heteroatome, unabhängig ausgewählt aus O, N und S, umfasst und/oder wobei die Heteroarylgruppe gegebenenfalls substituiert ist;
(f) C₇-C₂₀-Alkylaryl, wobei die Alkylarylgruppe gegebenenfalls substituiert ist und/oder wobei ein Alkylteil der Alkylarylgruppe gegebenenfalls (teilweise) ungesättigt ist und/oder gegebenenfalls durch ein oder mehrere Heteroatome unterbrochen ist, wobei die Heteroatome unabhängig ausgewählt sind aus O, N und S; und
(g) C₇-C₂₀-Alkylheteroaryl, wobei die Alkylheteroarylgruppe 1 - 3 Heteroatome, unabhängig ausgewählt aus O, N und S, umfasst und/oder wobei die Alkylheteroarylgruppe gegebenenfalls substituiert ist, und/oder wobei ein Alkylteil der Alkylheteroarylgruppe gegebenenfalls (teilweise) ungesättigt ist und/oder gegebenenfalls durch ein oder mehrere Heteroatome unterbrochen ist, wobei die Heteroatome unabhängig ausgewählt sind aus O, N und S;
wenn s = 1 - 5, dann ist R^{3*} unabhängig ausgewählt aus der Gruppe bestehend aus
(a) H;
(b) C₁-C₆-Alkyl, wobei die Alkylgruppe gegebenenfalls substituiert ist;
(c) C₃-C₆-Cycloalkyl, wobei die Cycloalkylgruppe gegebenenfalls substituiert ist;
(d) C₆-C₁₂-Aryl, wobei die Arylgruppe gegebenenfalls substituiert ist;
(e) C₆-C₁₂-Heteroaryl, wobei die Heteroarylgruppe 1 - 3 Heteroatome, unabhängig ausgewählt aus O, N und S, umfasst und/oder wobei die Heteroarylgruppe gegebenenfalls substituiert ist;
(f) C₇-C₁₂-Alkylaryl, wobei die Alkylarylgruppe gegebenenfalls substituiert ist; und
(g) C₇-C₁₂-Alkylheteroaryl, wobei die Alkylheteroarylgruppe 1 - 3 Heteroatome, unabhängig ausgewählt aus O, N und S, umfasst und/oder wobei die Alkylheteroarylgruppe gegebenenfalls substituiert ist.

2. Boriertes Polymer gemäß Formel (1), wobei das borierte Polymer ein Zahlenmittel des Molekulargewichts Mₙ im Bereich von ungefähr 1000 bis ungefähr 100000 aufweist.

3. Boriertes Polymer gemäß Anspruch 1 oder Anspruch 2, wobei s = 0.

4. Boriertes Polymer gemäß einem der Ansprüche 1 - 3, wobei q = 0.

5. Boriertes Polymer gemäß einem der Ansprüche 1 - 4, wobei v 1 oder 2 ist.

6. Boriertes Polymer gemäß einem der Ansprüche 1 - 5, wobei a 1 ist.

7. Boriertes Polymer gemäß einem der Ansprüche 1 - 6, wobei t 0 - 2 ist.

8. Verfahren zum Herstellen eines borierten Polymers gemäß einem der Ansprüche 1 - 7, wobei das Verfahren das Polymerisieren eines Monomers gemäß Formel (6): 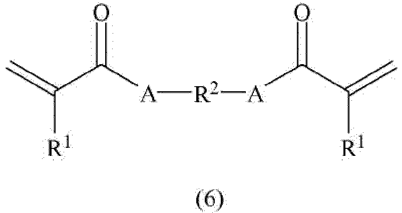 mit einem Monomer gemäß Formel (7):
H₂N-R³ (7)
und:
gegebenenfalls in Gegenwart eines Monomers gemäß Formel (8):
H₂N-R^{3*} (8)
oder gegebenenfalls in Gegenwart eines Monomers gemäß Formel (9): 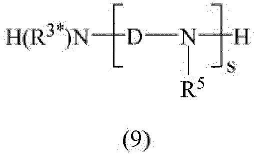
umfasst, wobei R¹, R², A, R³, R^{3*}, R⁴, R⁵, r und s die Bedeutung wie in Anspruch 1 definiert haben.

9. Verfahren zum Herstellen eines borierten Polymers gemäß einem der Ansprüche 1 - 7, wobei das Verfahren umfasst:
(a) Polymerisieren eines Monomers gemäß Formel (6): 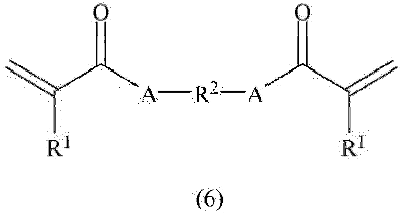 mit einem Monomer gemäß Formel (10):
^{t}BuO-C(O)-N(H)-(CR⁸₂)_{w}-NH₂ (10)
wobei w = 2 - 20 und R⁸ unabhängig ausgewählt ist aus der Gruppe bestehend aus H und C₁-C₂₀-Alkyl, um ein Polymer gemäß Formel (11) zu erhalten: 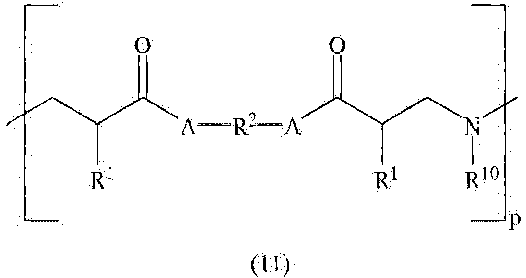 wobei R^{10 t}BuO-C(O)-N(H)-(CR⁸₂)-NH- ist;
(b) Umsetzen des Polymers gemäß Formel (11) mit einer Säure, um eine Formel gemäß Formel (11) zu erhalten, wobei R¹⁰ H₂N-(CR⁸₂)-NH- ist; und
(c) Umsetzen des Polymers, wie es in Schritt (b) erhalten wurde, mit einer Verbindung gemäß Formel (12): 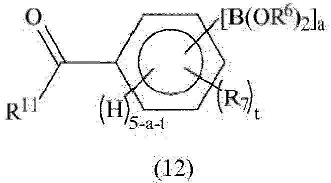 wobei:
a 1 oder 2 ist;
R⁶ unabhängig ausgewählt ist aus
(a) H;
(b) C₁-C₁₂-Alkyl, wobei die Alkylgruppe gegebenenfalls substituiert ist und/oder gegebenenfalls (teilweise) ungesättigt ist und/oder gegebenenfalls durch ein oder mehrere Heteroatome unterbrochen ist, wobei die Heteroatome unabhängig ausgewählt sind aus O, N und S;
(c) C₃-C₁₂-Cycloalkyl, wobei die Cycloalkylgruppe gegebenenfalls substituiert ist und/oder gegebenenfalls (teilweise) ungesättigt ist und/oder gegebenenfalls durch ein oder mehrere Heteroatome unterbrochen ist, wobei die Heteroatome unabhängig ausgewählt sind aus O, N und S;
(d) C₆-C₂₀-Aryl, wobei die Arylgruppe gegebenenfalls substituiert ist;
(e) C₆-C₂₀-Heteroaryl, wobei die Heteroarylgruppe 1 - 3 Heteroatome, unabhängig ausgewählt aus O, N und S, umfasst und/oder wobei die Heteroarylgruppe gegebenenfalls substituiert ist;
(f) C₇-C₂₀-Alkylaryl, wobei die Alkylarylgruppe gegebenenfalls substituiert ist und/oder wobei ein Alkylteil der Alkylarylgruppe gegebenenfalls (teilweise) ungesättigt ist und/oder gegebenenfalls durch ein oder mehrere Heteroatome unterbrochen ist, wobei die Heteroatome unabhängig ausgewählt sind aus O, N und S; und
(g) C₇-C₂₀-Alkylheteroaryl, wobei die Alkylheteroarylgruppe 1 - 3 Heteroatome, unabhängig ausgewählt aus O, N und S, umfasst und/oder wobei die Alkylheteroarylgruppe gegebenenfalls substituiert ist und/oder wobei ein Alkylteil der Alkylheteroarylgruppe gegebenenfalls (teilweise) ungesättigt ist und/oder gegebenenfalls durch ein oder mehrere Heteroatome unterbrochen ist, wobei die Heteroatome unabhängig ausgewählt sind aus O, N und S;
R⁷ unabhängig ausgewählt ist aus
(a) Halogen;
(b) C₁-C₁₂-Alkyl, wobei die Alkylgruppe gegebenenfalls substituiert ist und/oder gegebenenfalls (teilweise) ungesättigt ist und/oder gegebenenfalls durch ein oder mehrere Heteroatome unterbrochen ist, wobei die Heteroatome unabhängig ausgewählt sind aus O, N und S;
(c) C₃-C₁₂-Cycloalkyl, wobei die Cycloalkylgruppe gegebenenfalls substituiert ist und/oder gegebenenfalls (teilweise) ungesättigt ist und/oder gegebenenfalls durch ein oder mehrere Heteroatome unterbrochen ist, wobei die Heteroatome unabhängig ausgewählt sind aus O, N und S;
(d) C₆-C₂₀-Aryl, wobei die Arylgruppe gegebenenfalls substituiert ist;
(e) C₆-C₂₀-Heteroaryl, wobei die Heteroarylgruppe 1 - 3 Heteroatome, unabhängig ausgewählt aus O, N und S, umfasst und/oder wobei die Heteroarylgruppe gegebenenfalls substituiert ist;
(f) C₇-C₂₀-Alkylaryl, wobei Alkylarylgruppe gegebenenfalls substituiert ist und/oder wobei ein Alkylteil der Alkylarylgruppe gegebenenfalls (teilweise) ungesättigt ist und/oder gegebenenfalls durch ein oder mehrere Heteroatome unterbrochen ist, wobei die Heteroatome unabhängig ausgewählt sind aus O, N und S; und
(g) C₇-C₂₀-Alkylheteroaryl, wobei die Alkylheteroarylgruppe 1 - 3 Heteroatome, unabhängig ausgewählt aus O, N und S, umfasst und/oder wobei die Alkylheteroarylgruppe gegebenenfalls substituiert ist, und/oder wobei ein Alkylteil der Alkylheteroarylgruppe gegebenenfalls (teilweise) ungesättigt ist und/oder gegebenenfalls durch ein oder mehrere Heteroatome unterbrochen ist, wobei die Heteroatome unabhängig ausgewählt sind aus O, N und S;
R¹¹ H oder eine Abgangsgruppe ist;
t=0-4;
und wobei R¹, R² und A wie in Anspruch 1 definiert sind.

10. Verfahren nach Anspruch 9, wobei jegliche seitenständige Aminogruppen einer Acylierung unterworfen werden.

11. Boriertes Polymer, erhältlich durch das Verfahren nach Anspruch 8 oder erhältlich durch das Verfahren nach Anspruch 9 oder Anspruch 10.

12. Arzneimittelverabreichungssystem, umfassend das borierte Polymer nach einem der Ansprüche 1 - 7 oder Anspruch 11.

13. Aggregat oder Nanopartikel, umfassend das borierte Polymer nach einem der Ansprüche 1 - 7 oder Anspruch 11.

14. Aggregat oder Nanopartikel gemäß Anspruch 13, wobei das Nanopartikel eine biologisch aktive Komponente, ausgewählt aus der Gruppe von Arzneimitteln, anionischen Polymeren, DNA-Molekülen oder Derivaten davon, RNA-Molekülen oder Derivaten davon, Peptiden und Derivaten davon und Proteinen und Derivaten davon, umfasst.

15. Nanopartikel nach Anspruch 13 oder Anspruch 14, wobei R² unabhängig ausgewählt ist aus der Gruppe bestehend aus:
(a) C₁-C₂₀-Alkylen, wobei die Alkylengruppe gegebenenfalls substituiert ist und durch eine oder mehrere -S-S- Gruppen unterbrochen ist; und
(b) C₃-C₂₀-Cycloalkylen, wobei die Cycloalkylengruppe gegebenenfalls substituiert ist und durch eine oder mehrere -S-S- Gruppen außerhalb des Rings unterbrochen ist.

16. Hydrogel, umfassend das borierte Polymer nach einem der Ansprüche 1 - 7 oder Anspruch 11.

17. Hydrogel nach Anspruch 16, außerdem umfassend einen Polyvinylalkohol.

18. Hydrogel nach Anspruch 16 oder Anspruch 17, außerdem umfassend ein Kohlenhydrat.

19. Hydrogel nach einem der Ansprüche 16 - 18, außerdem umfassend einen Vernetzer.

20. Hydrogel nach einem der Ansprüche 16 - 19, wobei R² unabhängig ausgewählt ist aus der Gruppe bestehend aus:
(a) C₁-C₂₀-Alkylen, wobei die Alkylengruppe gegebenenfalls substituiert ist und durch eine oder mehrere -S-S- Gruppen unterbrochen ist; und
(b) C₃-C₂₀-Cycloalkylen, wobei die Cycloalkylengruppe gegebenenfalls substituiert ist und durch eine oder mehrere -S-S- Gruppen außerhalb des Rings unterbrochen ist.

## Revendications

1. Un polymère contenant du bore présentant la formule (1) 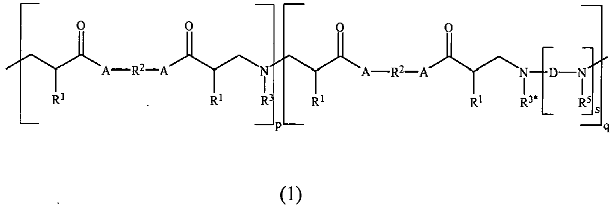 dans laquelle:
- A est sélectionné, indépendamment les uns des autres, parmi une liaison simple carbone-carbone directe, O, N et S;
- R¹ est sélectionné, indépendamment les uns des autres, parmi H et CH₃;
- R² est sélectionné, indépendamment les uns des autres, dans le groupe consistant en:
(a) C₁-C₂₀ alkylène, dans lequel le groupe alkylène est éventuellement substitué et/ou est éventuellement (partiellement) insaturé et/ou est éventuellement interrompu par un ou plusieurs hétéroatomes, dans lequel les hétéroatomes sont sélectionnés, indépendamment les uns des autres, parmi O, N et S et/ou dans lequel le groupe alkylène est interrompu par un ou plusieurs groupes -S-S-;
(b) C₃-C₂₀ cycloalkylène, dans lequel le groupe cycloalkylène est éventuellement substitué et/ou est éventuellement (partiellement) insaturé et/ou comporte éventuellement un ou plusieurs hétéroatomes dans le cycle, où les hétéroatomes sont sélectionnés, indépendamment les uns des autres, parmi O, N et S, et/ou dans lequel le groupe cycloalkylène est interrompu par un ou plusieurs groupes -S-S- extérieur au noyau;
(c) C₆-C₂₀ arylène, dans lequel le groupe arylène est éventuellement substitué;
(d) C₆-C₂₀ hétéroarylène, dans lequel le groupe hétéroarylène comprend 1 à 3 hétéroatomes sélectionnés, indépendamment les uns des autres, parmi O, N et S et/ou dans lequel le groupe hétéroarylène est éventuellement substitué;
(e) C₇-C₂₀ alkylarylène dans lequel le groupe alkylarylène est éventuellement substitué et/ou dans lequel une partie alkyle du groupe alkylarylène est éventuellement (partiellement) insaturé et/ou est éventuellement interrompu par un ou plusieurs hétéroatomes, dans lequel les hétéroatomes sont sélectionnés, indépendamment les uns des autres, parmi O, N et S, et/ou dans lequel une partie alkyle du groupe alkylarylène est interrompu par un ou plusieurs groupes -S-S-;
(f) C₇-C₂₀ alkylhétéroarylène, dans lequel le groupe alkylhétéroarylène comprend de 1 à 3 hétéroatomes sélectionnés, indépendamment les uns des autres, parmi O, N et S et/ou dans lequel le groupe alkylhétéroarylène est éventuellement substitué, et/ou dans lequel une partie alkyle du groupe alkylhétéroarylène est éventuellement (partiellement) insaturé et/ou est éventuellement interrompu par un ou plusieurs hétéroatomes, dans lequel les hétéroatomes sont sélectionnés, indépendamment les uns des autres, parmi O, N et S, et/ou dans lequel une partie alkyle du groupe alkylhétéroarylène est interrompu par un ou plusieurs groupes -S-S-;
et
(g) un groupe dans lequel deux groupes (hétéro)-arylène et/ou groupes alkyl-(hétéro)-arylène sont reliés les uns aux autres par un groupe -S-S-;
D est sélectionné dans le groupe constitué par -(CR⁴₂)r- et les groupes (a), (b), (c), (d), (e), (f) et (g) défini pour R²;
R⁴ est sélectionné, indépendamment les uns des autres, dans le groupe consistant en:
(a) un atome d'hydrogène,
(b) C₁-C₂₀ alkyle, dans lequel le groupe alkyle est éventuellement substitué et/ou est éventuellement (partiellement) insaturé et/ou est éventuellement interrompu par un ou plusieurs hétéroatomes, dans lequel les hétéroatomes sont sélectionnés, indépendamment les uns des autres, parmi O, N et S;
(c) C₃-C₂₀ cycloalkyle, dans lequel le groupe cycloalkyle est éventuellement substitué et/ou est éventuellement (partiellement) insaturé et/ou comporte éventuellement un ou plusieurs hétéroatomes dans le cycle, dans lequel les hétéroatomes sont sélectionnés, indépendamment les uns des autres, parmi O, Net S;
(d) C₆-C₂₀ aryle, dans lequel le groupe aryle est éventuellement substitué;
(e) C₆-C₂₀ hétéroaryle, où le groupe hétéroaryle comprend 1 à 3 hétéroatomes sélectionnés, indépendamment les uns des autres, parmi O, N et S et/ou dans lequel le groupe hétéroaryle est éventuellement substitué;
(f) C₇-C₂₀ alkylaryle dans lequel le groupe alkylaryle est éventuellement substitué et/ou dans lequel une partie alkyle du groupe alkylaryle est éventuellement (partiellement) insaturé et/ou est éventuellement interrompu par un ou plusieurs hétéroatomes, dans lequel les hétéroatomes sont sélectionnés, indépendamment les uns des autres, parmi O, N et S; et
(g) C₇-C₂₀ alkylhétéroaryle, dans lequel le groupe alkylhétéroaryle comprend de 1 à 3 hétéroatomes sélectionnés, indépendamment les uns des autres, parmi O, N et S et/ou dans lequel le groupe alkylhétéroaryle est éventuellement substitué, et/ou dans lequel une partie alkyle du groupe alkylhétéroaryle est éventuellement (partiellement) insaturé et/ou est éventuellement interrompu par un ou plusieurs hétéroatomes, dans lequel les hétéroatomes sont sélectionnés, indépendamment les uns des autres, parmi O, N et S;
R⁵ est sélectionné, indépendamment les uns des autres, dans le groupe consistant en un atome d'hydrogène et C₁-C₂₀ alkyle, dans lequel le groupe alkyle est éventuellement substitué et/ou est éventuellement (partiellement) insaturé et/ou est éventuellement interrompu par un ou plusieurs hétéroatomes, dans lequel les hétéroatomes sont sélectionnés, indépendamment les uns des autres, parmi O, N et S;
p = 1 à 100;
q = 0 à 100;
r = 2-6;
s = 0-5;
R³ présente la formule (2): 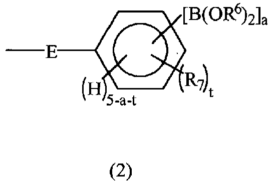
dans laquelle:
a est 1 ou 2;
R⁶ est sélectionné, indépendamment les uns des autres, parmi
(a) un atome d'hydrogène,
(b) C₁-C₁₂ alkyle, dans lequel le groupe alkyle est éventuellement substitué et/ou est éventuellement (partiellement) insaturé et/ou est éventuellement interrompu par un ou plusieurs hétéroatomes, dans lequel les hétéroatomes sont sélectionnés, indépendamment les uns des autres, parmi O, N et S;
(c) C₃-C₁₂ cycloalkyle, dans lequel le groupe cycloalkyle est éventuellement substitué et/ou est éventuellement (partiellement) insaturé et/ou est éventuellement interrompu par un ou plusieurs hétéroatomes, dans lequel les hétéroatomes sont sélectionnés, indépendamment les uns des autres, parmi O, Net S;
(d) C₆-C₂₀ aryle, où le groupe aryle est éventuellement substitué;
(e) C₆-C₂₀ hétéroaryle, dans lequel le groupe hétéroaryle comprend 1 à 3 hétéroatomes sélectionnés, indépendamment les uns des autres, parmi O, N et S et/ou dans lequel le groupe hétéroaryle est éventuellement substitué;
(f) C₇-C₂₀ alkylaryle dans lequel le groupe alkylaryle est éventuellement substitué et/ou dans lequel une partie alkyle du groupe alkylaryle est éventuellement (partiellement) insaturé et/ou est éventuellement interrompu par un ou plusieurs hétéroatomes, dans lequel les hétéroatomes sont sélectionnés, indépendamment les uns des autres, parmi O, N et S; et
(g) C₇-C₂₀ alkylhétéroaryle, dans lequel le groupe alkylhétéroaryle comprend de 1 à 3 hétéroatomes sélectionnés, indépendamment les uns des autres, parmi O, N et S et/ou dans lequel le groupe alkylhétéroaryle est éventuellement substitué, et/ou dans lequel une partie alkyle du groupe alkylhétéroaryle est éventuellement (partiellement) insaturé et/ou est éventuellement interrompu par un ou plusieurs hétéroatomes, dans lequel les hétéroatomes sont sélectionnés, indépendamment les uns des autres, parmi O, N et S;
R⁷ est sélectionné, indépendamment les uns des autres, parmi
(a) un atome d'halogène;
(b) C₁-C₁₂ alkyle, dans lequel le groupe alkyle est éventuellement substitué et/ou est éventuellement (partiellement) insaturé et/ou est éventuellement interrompu par un ou plusieurs hétéroatomes, dans lequel les hétéroatomes sont sélectionnés, indépendamment les uns des autres, parmi O, N et S;
(c) C₃-C₁₂ cycloalkyle, dans lequel le groupe cycloalkyle est éventuellement substitué et/ou est éventuellement (partiellement) insaturé et/ou est éventuellement interrompu par un ou plusieurs hétéroatomes, dans lequel les hétéroatomes sont sélectionnés, indépendamment les uns des autres, parmi O, Net S;
(d) C₆-C₂₀ aryle, dans lequel le groupe aryle est éventuellement substitué;
(e) C₆-C₂₀ hétéroaryle, dans lequel le groupe hétéroaryle comprend 1 à 3 hétéroatomes sélectionnés, indépendamment les uns des autres, parmi O, N et S et/ou dans lequel le groupe hétéroaryle est éventuellement substitué;
(f) C₇-C₂₀ alkylaryle dans lequel le groupe alkylaryle est éventuellement substitué et/ou dans lequel une partie alkyle du groupe alkylaryle est éventuellement (partiellement) insaturé et/ou est éventuellement interrompu par un ou plusieurs hétéroatomes, dans lequel les hétéroatomes sont sélectionnés, indépendamment les uns des autres, parmi O, N et S; et
(g) C₇-C₂₀ alkylhétéroaryle, dans lequel le groupe alkylhétéroaryle comprend de 1 à 3 hétéroatomes sélectionnés, indépendamment les uns des autres, parmi O, N et S et/ou dans lequel le groupe alkylhétéroaryle est éventuellement substitué, et/ou dans lequel une partie alkyle du groupe alkylhétéroaryle est éventuellement (partiellement) insaturé et/ou est éventuellement interrompu par un ou plusieurs hétéroatomes, dans lequel les hétéroatomes sont sélectionnés, indépendamment les uns des autres, parmi O, N et S;
E est sélectionné, indépendamment les uns des autres, dans le groupe constitué par:
-(CR⁸₂)ᵤ - N(R⁹) - C(O)-,
-C(O)-N(R⁹)-(CR⁸₂)ᵤ-,
-(CR⁸2)ᵤ - N = CR⁹ -,
-CR₉ = N-(CR⁸₂)ᵤ -, et
-(CR⁸₂)ᵤN(R⁹) - (CR⁸₂)ᵥ-;
t=0-4;
u=1-10;
v=1-4;
R⁸ est sélectionné, indépendamment les uns des autres, dans le groupe consistant en l'atome d'hydrogène et C₁-C₂₀ alkyle;
R⁹ est sélectionné, indépendamment les uns des autres, dans le groupe consistant en un atome d'hydrogène et C₁-C₂₀ alkyle;
et
lorsque s = 0, alors R^{3*} est sélectionné, indépendamment les uns des autres, dans le groupe consistant en
(a) un atome d'hydrogène,
(b) C₁-C₁₂ alkyle, dans lequel le groupe alkyle est éventuellement substitué et/ou est éventuellement (partiellement) insaturé et/ou est éventuellement interrompu par un ou plusieurs hétéroatomes, dans lequel les hétéroatomes sont sélectionnés, indépendamment les uns des autres, parmi O, N et S;
(c) C₃-C₁₂ cycloalkyle, dans lequel le groupe cycloalkyle est éventuellement substitué et/ou est éventuellement (partiellement) insaturé et/ou est éventuellement interrompu par un ou plusieurs hétéroatomes, dans lequel les hétéroatomes sont sélectionnés, indépendamment les uns des autres, parmi O, Net S;
(d) C₆-C₂₀ aryle, où le groupe aryle est éventuellement substitué;
(e) C₆-C₂₀ hétéroaryle, où le groupe hétéroaryle comprend 1 à 3 hétéroatomes sélectionnés, indépendamment les uns des autres, parmi O, N et S et/ou dans lequel le groupe hétéroaryle est éventuellement substitué;
(f) C₇-C₂₀ alkylaryle dans lequel le groupe alkylaryle est éventuellement substitué et/ou dans lequel une partie alkyle du groupe alkylaryle est éventuellement (partiellement) insaturé et/ou est éventuellement interrompu par un ou plusieurs hétéroatomes, dans lequel les hétéroatomes sont sélectionnés, indépendamment les uns des autres, parmi O, N et S; et
(g) C₇-C₂₀ alkylhétéroaryle, dans lequel le groupe alkylhétéroaryle comprend de 1 à 3 hétéroatomes sélectionnés, indépendamment les uns des autres, parmi O, N et S et/ou dans lequel le groupe alkylhétéroaryle est éventuellement substitué, et/ou dans lequel une partie alkyle du groupe alkylhétéroaryle est éventuellement (partiellement) insaturée et/ou est éventuellement interrompu par un ou plusieurs hétéroatomes, dans lequel les hétéroatomes sont sélectionnés, indépendamment les uns des autres, parmi O, N et S;
lorsque s = 1-5, alors R^{3*} est sélectionné, indépendamment les uns des autres, dans le groupe consistant en
(a) un atome d'hydrogène,
(b) C₁-C₆ alkyle, dans lequel le groupe alkyle est éventuellement substitué;
(c) C₃-C₆ cycloalkyle, dans lequel le groupe cycloalkyle est éventuellement substitué;
(d) C₆-C₁₂ aryle, dans lequel le groupe aryle est éventuellement substitué;
(e) C₆-C₁₂ hétéroaryle, dans lequel le groupe hétéroaryle comprend 1 à 3 hétéroatomes sélectionnés, indépendamment les uns des autres, parmi O, N et S et/ou dans lequel le groupe hétéroaryle est éventuellement substitué;
(f) C₇-C₁₂ alkylaryle dans lequel le groupe alkylaryle est éventuellement substitué; et
(g) C₇-C₁₂ alkylhétéroaryle, dans lequel le groupe alkylhétéroaryle comprend de 1 à 3 hétéroatomes sélectionnés, indépendamment les uns des autres, parmi O, N et S et/ou dans lequel le groupe alkylhétéroaryle est éventuellement substitué.

2. Le polymère contenant du bore selon la formule (1), dans lequel le polymère contenant du bore a un poids moléculaire moyen en nombre Mn dans la gamme d'environ 1000 à environ 100000.

3. Le polymère contenant du bore selon la revendication 1 ou la revendication 2, dans lequel s = 0.

4. Le polymère contenant du bore selon l'une quelconque des revendications 1 à 3, dans lequel q = 0.

5. Le polymère contenant du bore selon l'une quelconque des revendications 1-4, dans lequel v vaut 1 ou 2.

6. Le polymère contenant du bore selon l'une quelconque des revendications 1 à 5, dans lequel a est 1.

7. Le polymère contenant du bore selon l'une quelconque des revendications 1 à 6, dans lequel t = 0 - 2.

8. Un procédé de préparation d'un polymère contenant du bore selon l'une quelconque des revendications 1 à 7, ledit procédé comprenant la polymérisation d'un monomère selon la formule (6): 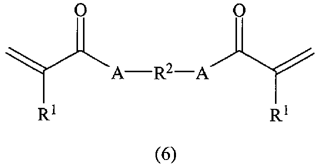 avec un monomère selon la formule (7):
H₂N - R³ (7)
et:
éventuellement en présence d'un monomère selon la formule (8):
H₂N - R^{3*} (8)
ou éventuellement en présence d'un monomère répondant à la formule (9): 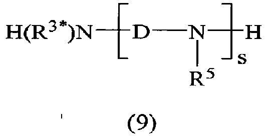
dans laquelle R¹, R², A, R³, R^{3*}, R⁴, R⁵, r et sont la signification telle que définie dans la revendication 1.

9. Procédé de préparation d'un polymère contenant du bore selon l'une quelconque des revendications 1 à 7, ledit procédé comprenant:
(a) la polymérisation d'un monomère selon la formule (6): 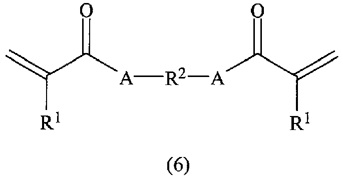 avec un monomère selon la formule (10):
^{t}BuO-C(O) -N(H) - (CR⁸₂)_{w} - NH₂ (10)
formule dans laquelle w = 2-20 et R⁸ est sélectionné, indépendamment les uns des autres, dans le groupe consistant en un atome d'hydrogène et C₁-C₂₀ alkyle, pour obtenir un polymère selon la formule (11): 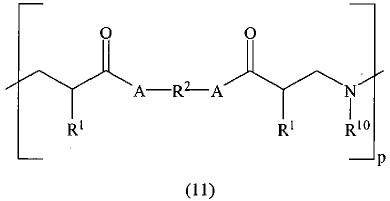 dans laquelle R¹⁰ est ^{t}BuO-C(O) - N(H) - (CR⁸₂) -NH-;
(b) la réaction du polymère selon la formule (11) avec un acide pour obtenir une formule selon la formule (11) dans laquelle R¹⁰ est H₂N- (CR⁸₂) -NH-; et
(c) la réaction du polymère obtenu dans l'étape (b) avec un composé répondant à la formule (12): 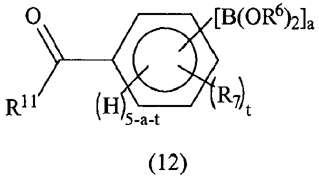 dans laquelle:
a est égal à 1 ou 2;
R⁶ est sélectionné, indépendamment les uns des autres, parmi:
(a) un atome d'hydrogène;
(b) C₁-C₁₂ alkyle, dans lequel le groupe alkyle est éventuellement substitué et/ou est éventuellement (partiellement) insaturé et/ou est éventuellement interrompu par un ou plusieurs hétéroatomes, dans lequel les hétéroatomes sont sélectionnés, indépendamment les uns des autres, parmi O, N et S;
(c) C₃-C₁₂ cycloalkyle, dans lequel le groupe cycloalkyle est éventuellement substitué et/ou est éventuellement (partiellement) insaturé et/ou est éventuellement interrompu par un ou plusieurs hétéroatomes, dans lequel les hétéroatomes sont sélectionnés, indépendamment les uns des autres, parmi O, N et S;
(d) C₆-C₂₀ aryle, dans lequel le groupe aryle est éventuellement substitué;
(e) C₆-C₂₀ hétéroaryle, dans lequel le groupe hétéroaryle comprend 1 à 3 hétéroatomes sélectionnés, indépendamment les uns des autres, parmi O, N et S et/ou dans lequel le groupe hétéroaryle est éventuellement substitué;
(f) C₇-C₂₀ alkylaryle dans lequel le groupe alkylaryle est éventuellement substitué et/ou dans lequel une partie alkyle du groupe alkylaryle est éventuellement (partiellement) insaturé et/ou est éventuellement interrompu par un ou plusieurs hétéroatomes, dans lequel les hétéroatomes sont sélectionnés, indépendamment les uns des autres, parmi O, N et S; et
(g) C₇-C₂₀ alkylhétéroaryle, dans lequel le groupe alkylhétéro-aryle comprend de 1 à 3 hétéroatomes sélectionnés, indépendamment les uns des autres, parmi O, N et S et/ou dans lequel le groupe alkylhétéroaryle est éventuellement substitué, et/ou dans lequel une partie alkyle du groupe alkylhétéroaryle est éventuellement (partiellement) insaturé et/ou est éventuellement interrompu par un ou plusieurs hétéroatomes, dans lequel les hétéroatomes sont sélectionnés, indépendamment les uns des autres, parmi O, N et S;
R⁷ est sélectionné, indépendamment les uns des autres, parmi:
(a) un atome d'halogène;
(b) C₁-C₁₂ alkyle, dans lequel le groupe alkyle est éventuellement substitué et/ou est éventuellement (partiellement) insaturé et/ou est éventuellement interrompu par un ou plusieurs hétéroatomes, dans lequel les hétéroatomes sont sélectionnés, indépendamment les uns des autres, parmi O, N et S;
(c) C₃-C₁₂ cycloalkyle, dans lequel le groupe cycloalkyle est éventuellement substitué et/ou est éventuellement (partiellement) insaturé et/ou est éventuellement interrompu par un ou plusieurs hétéroatomes, dans lequel les hétéroatomes sont sélectionnés, indépendamment les uns des autres, parmi O, N et S;
(d) C₆-C₂₀ aryle, dans lequel le groupe aryle est éventuellement substitué;
(e) C₆-C₂₀ hétéroaryle, dans lequel le groupe hétéroaryle comprend 1 à 3 hétéroatomes sélectionnés, indépendamment les uns des autres, parmi O, N et S et/ou dans lequel le groupe hétéroaryle est éventuellement substitué;
(f) C₇-C₂₀ alkylaryle dans lequel le groupe alkylaryle est éventuellement substitué et/ou dans lequel une partie alkyle du groupe alkylaryle est éventuellement (partiellement) insaturé et/ou est éventuellement interrompu par un ou plusieurs hétéroatomes, dans lequel les hétéroatomes sont sélectionnés, indépendamment les uns des autres, parmi O, N et S; et
(g) C₇-C₂₀ alkylhétéroaryle, dans lequel le groupe alkylhétéroaryle comprend de 1 à 3 hétéroatomes sélectionnés, indépendamment les uns des autres, parmi O, N et S et/ou dans lequel le groupe alkylhétéroaryle est éventuellement substitué, et/ou dans lequel une partie alkyle du groupe alkylhétéroaryle est éventuellement (partiellement) insaturé et/ou est éventuellement interrompu par un ou plusieurs hétéroatomes, dans lequel les hétéroatomes sont sélectionnés, indépendamment les uns des autres, parmi O, N et S;
R¹¹ est H ou un groupe partant;
t = 0-4;
et dans laquelle R¹, R² et A sont tels que définis dans la revendication 1.

10. Procédé selon la revendication 9, dans lequel certains groupes amino pendants sont soumis à une acylation.

11. Un polymère contenant du bore pouvant être obtenue par le procédé selon la revendication 8 ou pouvant être obtenu par le procédé selon la revendication 9 ou la revendication 10.

12. Un système de délivrance de médicament comprenant le polymère contenant du bore selon l'une quelconque des revendications 1 à 7 ou la revendication 11.

13. Un agrégat ou une nanoparticule comprenant le polymère contenant du bore selon l'une quelconque des revendications 1 à 7 ou la revendication 11.

14. L'agrégat ou la nanoparticule selon la revendication 13, dans lequel la nanoparticule comprend un composant biologiquement actif sélectionné dans le groupe des médicaments, des polymères anioniques, des molécules d'ADN ou leurs dérivés, des molécules d'ARN ou leurs dérivés, des peptides et leurs dérivés, et des protéines et leurs dérivés.

15. La nanoparticule selon la revendication 13 ou la revendication 14, dans laquelle R² est sélectionné, indépendamment les uns des autres, dans le groupe consistant en:
(a) C₁-C₂₀ alkylène, dans lequel le groupe alkylène est éventuellement substitué et est interrompu par un ou plusieurs groupes -S-S-; et
(b) C₃-C₂₀ cycloalkylène, dans lequel le groupe cycloalkylène est éventuellement substitué et est interrompu par un ou plusieurs groupes -S-S- extérieur au noyau.

16. Un hydrogel comprenant le polymère contenant du bore selon l'une quelconque des revendications 1 à 7 ou la revendication 11.

17. L'hydrogel selon la revendication 16, comprenant en outre un alcool polyvinylique.

18. L'hydrogel selon la revendication 16 ou la revendication 17, comprenant en outre un hydrate de carbone.

19. L'hydrogel selon l'une quelconque des revendications 16 à 18, comprenant en outre un agent de réticulation.

20. L'hydrogel selon l'une quelconque des revendications 16 à 19, dans lequel R² est sélectionné, indépendamment les uns des autres, dans le groupe consistant en:
(a) C₁-C₂₀ alkylène, dans lequel le groupe alkylène est éventuellement substitué et est interrompu par un ou plusieurs groupes -S-S-; et
(b) C₃-C₂₀ cycloalkylène, dans lequel le groupe cycloalkylène est éventuellement substitué et est interrompu par un ou plusieurs groupes -S-S- extérieur au noyau.
